(19) Europäisches Patentamt
European Patent Office
Office européen des brevets

(11) EP 1 803 729 A1

(12) **EUROPEAN PATENT APPLICATION**
published in accordance with Art. 158(3) EPC

(43) Date of publication:
**04.07.2007 Bulletin 2007/27**

(21) Application number: **05788199.7**

(22) Date of filing: **28.09.2005**

(51) Int Cl.:
***C07H 19/04*** *(2006.01)* ***A61K 31/7056*** *(2006.01)*
***A61K 31/706*** *(2006.01)* ***A61K 45/00*** *(2006.01)*
***A61P 3/04*** *(2006.01)* ***A61P 3/06*** *(2006.01)*
***A61P 3/10*** *(2006.01)* ***A61P 9/04*** *(2006.01)*
***A61P 9/10*** *(2006.01)* ***A61P 9/12*** *(2006.01)*
***A61P 19/06*** *(2006.01)* ***A61P 43/00*** *(2006.01)*

(86) International application number:
**PCT/JP2005/017807**

(87) International publication number:
**WO 2006/035796 (06.04.2006 Gazette 2006/14)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.09.2004 JP 2004283106**
**22.04.2005 JP 2005125257**

(71) Applicant: **Kissei Pharmaceutical Co., Ltd.**
**Matsumoto-shi**
**Nagano 399-8710 (JP)**

(72) Inventors:
- **TERANISHI, Hirotaka**
  **Kissei Pharmaceutical Co., Ltd**
  **Minamiazumi-gun,**
  **Nagano 3998304 (JP)**
- **KIKUCHI, Norihiko**
  **Kissei Pharmaceutical Co., Ltd.**
  **Minamiazumi-gun,**
  **Nagano 3998304 (JP)**
- **OHNO, Kohsuke**
  **c/o Central Research Laboratories**
  **Oaza Kashiwabara,**
  **Hotaka-machi,**
  **Minamiazumi-gun,**
  **Nagano 3998304 (JP)**

(74) Representative: **Hayes, Adrian Chetwynd et al**
**Boult Wade Tennant,**
**Verulam Gardens**
**70 Gray's Inn Road**
**London WC1X 8BT (GB)**

# (54) 1-( -D-GLYCOPYRANOSYL)-3-SUBSTITUTED NITROGENOUS HETEROCYCLIC COMPOUND, MEDICINAL COMPOSITION CONTAINING THE SAME, AND MEDICINAL USE THEREOF

(57) A compound having SGLT1 and/or SGLT2 inhibitory activity which is usable as an agent for the prevention or treatment of diabetes, postprandial hyperglycemia, impaired glucose tolerance, diabetic complications, obesity, etc. It is a 1-(β-D-glycopyranosyl)-3-substituted nitrogen-containing heterocyclic compound represented by the general formula (I), a prodrug, or a pharmaceutically acceptable salt thereof, or a hydrate or solvate thereof; an SGLT inhibitor containing the same; a pharmaceutical composition containing the same and a medicinal use thereof. In the formula, ring A represents optionally substituted aryl or heteroaryl; $Q^1$ to $Q^5$ independently represent a carbon atom having a hydrogen atom or substituent bonded thereto or a nitrogen atom; E represents a single bond, alkylene, -O-, -S- or -NH-; and R.represents methyl, ethyl, fluoromethyl or hydroxymethyl.

EP 1 803 729 A1

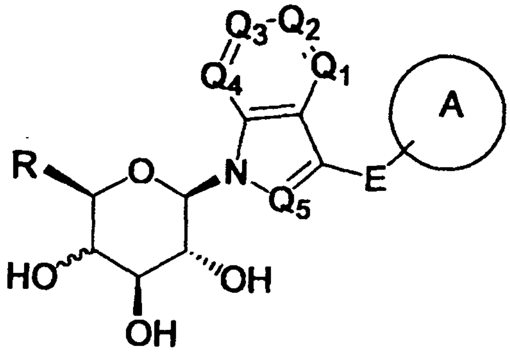

(I)

(G)

## Description

### Technical Field

The present invention relates to a 1-(β-D-glycopyranosyl)-3-substituted nitrogen-containing heterocyclic compound.

More particularly, the present invention relates to a 1-(β-D-glycopyranosyl)-3-substituted nitrogen-containing heterocyclic compound which can be used as an agent for the prevention or treatment of a disease associated with hyperglycemia such as diabetes, impaired glucose tolerance, diabetic complications or obesity,' a prodrug thereof, a pharmaceutically acceptable salt thereof, a hydrate or a solvate thereof, and a pharmaceutical composition comprising the same.

### Background Art

It is known that a sodium-dependent glucose transporter, hereinafter referred to as "SGLT", which is a co-transporter of monosaccharide and sodium has some subtypes. Namely, a sodium-dependent glucose transporter 1, hereinafter referred to as "SGLT1", exists in the small intestine and the S3 region of the kidney' s proximal tubule, and a sodium-dependent glucose transporter 2, hereinafter referred to as "SGLT2", exists in the S1 segment of the kidney's proximal tubule.

Among them, SGLT1 which exists in the small intestine participates in glucose and galactose absorption from the digestive tract (Non-patent references 1 and 2). In diabetic patients, carbohydrate digestion and absorption increase. Actually, it is confirmed that SGLT1 and its mRNA highly increase in the small intestine (see Non-patent reference 3). Therefore, inhibiting SGLT1 can control increase of blood sugar level by suppression of glucose and galactose absorption in the small intestine (see Patent reference 1).

On the other hand, SGLT2 participates in reabsorption of glucose filtrated through the glomerulus (see Non-patent reference 4). Therefore, inhibiting SGLT2 can normalize blood sugar level by suppression of glucose reabsorption (see Patent reference 5).

As the SGLT1 inhibitors, pyrazole derivatives (see Patent references 1 and 2), benzylphenol derivatives (see Patent reference 3) and the like are known. And as the SGLT2 inhibitors, glucopyranosyloxypyrazole derivatives (see Patent reference 4), glucopyranosyloxybenzylbenzene derivatives (see Patent reference 5) and the like are known.

Both of the above mentioned SGLT1 inhibitors and SGLT2 inhibitors have a glucopyranosyl group bound to an aryl group or a heteroaryl group through an oxygen atom. Until now, it is not known that any compound whose glycopyranosyl group such as glucopyranosyl group and galactopyranosyl group binds to a nitrogen atom in a ring of a nitrogen-containing heterocyclic compound has an SGLT1 and/or SGLT2 inhibitory activity.

By the way, as the compound whose glucopyranosyl group binds to a nitrogen atom in a ring of a nitrogen-containing heterocyclic compound, 1-(β-D-glucopyranosyl)-3-(2-thiazolyl)indole and 1-(β-D-glucopyranosyl)-6-methoxy-3-(2-thiazolyl)indole are known (see Non-patent reference 5). However, it is not known that these compounds have an SGLT1 and/or SGLT2 inhibitory activity and further have an activity to suppress the increase of blood sugar level and/or to normalize blood sugar level.

[Non-patent reference 1] Yoshikatsu Kanai, Kidney and Dialysis, 1998.12, Vol.45, extra edition, pp.232-237;
[Non-patent reference 2] E. Turk and 4 persons, Nature, 1991.3, Vol.350, pp.354-356;
[Non-patent reference 3] J. Dyer and 4 persons, American Journal of Physiology, 2002.2, Vol.282, No.2, pp.G241-G248;
[Non-patent reference 4] Yoshikatsu Kanai and 4 persons, J. Clin. Invest., 1994.1, Vol.93, pp.397-404;
[Non-patent reference 5] M. Pedras and 2 persons, Bioorganic & Medicinal Chemistry, 2002, Vol.10, pp.3307-3312;
[Patent reference 1] International Publication No. WO04/ 014932 pamphlet;
[Patent reference 2] International Publication No. WO01/ 018491 pamphlet;
[Patent reference 3] Japanese patent publication No. JP2004-196788;
[Patent reference 4] International publication No. WO01/ 16147 pamphlet;
[Patent reference 5] International publication No. WO01/ 68660 pamphlet.

### Disclosure of the Invention

### Objects to be Solved by the Invention

The present invention aims to provide a compound which has an SGLT1 and/or SGLT2 inhibitory activity.

The present inventors have studied earnestly on compounds having an inhibitory activity against SGLT1 and/or SGLT2. As a result, it was found that certain 1- (β-D-glycopyranosyl) -3-substituted nitrogen-containing heterocyclic compound represented by the following general formula (I) has an excellent inhibitory activity against SGLT1 and/or

SGLT2, thereby forming the basis of the present invention.

That is, the gist of the present invention resides in a 1-(β-D-glycopyranosyl)-3-substituted nitrogen-containing heterocyclic compound represented by a general formula (I) or a prodrug thereof, a pharmaceutically acceptable salt thereof, or a hydrate or solvate thereof; a pharmaceutical composition comprising the same and a medical use thereof.

[Chem.1]

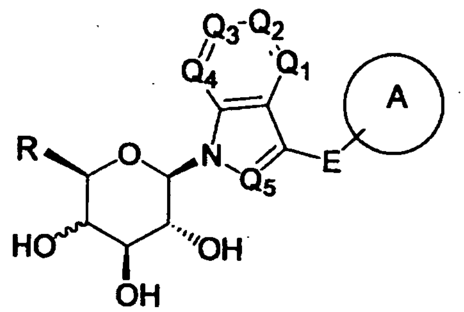

(I)

wherein ring A represents an optionally substituted aryl group or heteroaryl group; $Q^1$ to $Q^5$ independently represent a carbon atom which a hydrogen atom or a substituent combines with, or a nitrogen atom; E represents a single bond, an alkylene group, -O-, -S- or -NH-; and R represents a methyl group, an ethyl group, a fluoromethyl group or a hydroxymethyl group.

**Effects of the Invention**

Since a 1-(β-D-glycopyranosyl)-3-substituted nitrogen-containing heterocyclic compound (I) of the present invention or a prodrug thereof, a pharmaceutically acceptable salt thereof, or a hydrate or solvate thereof has an excellent inhibitory activity against SGLT1 and/or SGLT2, it can control the increase of blood sugar level and normalize blood sugar level.

**Best Mode to Put the Invention to Practice**

Meanings of terms used in this description are as follows.

The term "nitrogen-containing heterocyclic compound" means a heterocyclic compound having any nitrogen atoms as a hetero atom.

The term "halogen atom" means a fluorine atom, a chlorine atom, a bromine atom or an iodine atom.

The term "alkyl" means optionally branched alkyl having 1 to 6 carbon atoms.

The term "alkenyl" means optionally branched alkenyl having 2 to 6 carbon atoms.

The term "alkynyl" means optionally branched alkynyl having 2 to 6 carbon atoms.

The term "alkylene" means.optionally branched alkylene having 1 to 6 carbon atoms.

The term "alkenylene" means optionally branched alkenylene having 2 to 6 carbon atoms.

The term "alkoxy" means optionally branched alkoxy having 1 to 6 carbon atoms.

The term "(di)alkylamino" means monoalkylamino or dialkylamino whose two alkyls may be different.

The term "aryl" means phenyl or naphthyl.

The term "heteroaryl" means monocyclic or fused cyclic heteroaryl having 1 or 2 or more hetero atoms selected from a group consisting of an oxygen atom, a nitrogen atom and a sulfur atom.

The term "(hetero)aryl" means aryl or heteroaryl.

The term "cycloalkyl" means cycloalkyl having 3 to 7 carbon atoms.

The term "heterocycloaikyl" means 3 to 7-membered heterocycloalkyl having 1 or 2 or more hetero atoms selected from a group consisting of an oxygen atom, a nitrogen atom and a sulfur atom.

The term "(hetero)cycloalkyl" means cycloalkyl or heterocycloalkyl.

The term "acyl" means optionally branched aliphatic carboxyl acyl having 2 to 7 carbon atoms, (hetero)-cycloalkylcarboxyl acyl or (hetero)arylcarboxyl acyl.

As the glycopyranosyl group in the general formula (I), a glucopyranosyl group or a galactopyranosyl group wherein a hydroxy group at 6-position may be substituted by a fluorine atom is preferable, especially a glucopyranosyl

group wherein a hydroxy group at 6-position may be substituted by a fluorine atom is preferable.
As the aryl group of ring A, a phenyl group is preferable, and when the aryl group has 2 or more substituents, these substituents may be different.
As the heteroaryl group, a 5-memberd monocyclic heteroaryl group such as a furyl group, a thienyl group, a pyrrolyl group, an oxazolyl group, a thiazolyl group, an imidazolyl group and the like, a 6-memberd monocyclic heteroaryl such as a pyridyl group, a pyrimidinyl group, a pyridazinyl group, a pyrazinyl group and the like; and a fused cyclic heteroaryl group such as an indolyl group, an isoindolyl group, a quinolyl group, an isoquinolyl group, a benzofuranyl group, a benzothienyl group and the like can be illustrated.

As a substituent which the (hetero) aryl group may have, for example, a halogen atom, a hydroxy group or a cyano group; an alkyl group, an alkenyl group, an alkynyl group, an alkoxy group, an alkylamino group, an alkylthio group, an alkylsulfinyl group and an alkylsulfonyl group, each of which may have any substituents $\alpha$ (to be described below, the same hereinafter); a (hetero)aryl group and a (hetero)cycloalkyl group, each of which may have any substituents $\alpha$ and optionally binding with a (hetero)aryl group via an alkylene group, -O-, -NH- or -S-; a -U-V-W-N($R^A$)-Y-Z group, a -U-V-COO-$R^B$ group and the like can be illustrated.

In the -U-V-W-N($R^A$)-Y-Z group or -U-V-COO-$R^B$ group, U means a single bond, -O- or -S-. As U, a single bond or -O- is preferable.
V means a single bond, or an alkylene or alkenylene group, which may have a hydroxy group.
W means a single bond, -CO-, -$SO_2$- or -C(=NH)-. As W, a single bond or -CO- is preferable.
$R^A$ means a hydrogen atom, or an alkyl group, a (hetero) aryl group or a (hetero) cycloalkyl group, each of which may have any substituents $\alpha$. As $R^A$, a hydrogen atom or an alkyl group optionally having any substituents $\alpha$ is preferable.
Y means a single bond or an alkylene group.
Z means a hydrogen atom; a formyl group; or an alkyl group, a (hetero) aryl group or a (hetero) cycloalkyl group, each of which may have any substituents $\alpha$; an acyl group optionally having any substituents $\alpha$; an alkoxy group or an arylalkoxycarbonyl group, each of which may have any substituents $\alpha$; -CON($R^1$) ($R^2$), -CSN ($R^1$)($R^2$), -$SO_2$N($R^1$)($R^2$) or -C (=N$R^1$)N ($R^2$)($R^3$); one to three amino acid residues, wherein the terminal carboxylic acid may be an alkoxycarbonyl group optionally having a hydroxy group, an alkoxy group, an amino group or a (di)alkylamino group; or an amide with an alicyclic amine or an alkylamine, each of which may have an alkyl group, a (hetero)cycloalkyl group, an alkoxycarbonyl group or an acyl group, each of which may have a hydroxy group, an alkoxy group, an amino group or a (di)alkylamino group, or a carboxamide group; or an alphatic, (hetero)cycloalkyl or (hetero)aryl carboxylic acid residue having an alicyclic amine optionally having an alkyl group, a (hetero)cycloalkyl group, an alkoxycarbonyl group or an acyl group, each of which may have a hydroxy group, an alkoxy group, an amino group or a (di)alkylamino group.
$R^1$, $R^2$ and $R^3$ independently mean a hydrogen atom, a nitro group, a cyano group, a sulfamoyl group, an acyl group, an alkoxycarbonyl group, an aryl group or an alkylsulfonyl group; or an alkyl group optionally having any substituents $\alpha$. As $R^1$, $R^2$ and $R^3$, a hydrogen atom or an alkyl group optionally having any substituents $\alpha$ is independently preferable.
$R^A$ binding and a part of a group forming Z may bind together to form an alicyclic amine optionally having any substituents $\alpha$.

$R^B$ means a hydrogen atom; an alkoxyalkyl group having a carboxy group or an alkoxycarbonyl group; an alkyl group, a (hetero) aryl group or a (hetero) cycloalkyl group, each of which may have any substituents $\alpha$; one to three amino acid residues, wherein the terminal carboxylic acid may be an alkoxycarbonyl group optionally having a hydroxy group, an alkoxy group, an amino group or a (di)alkylamino group; or an amide with an alicyclic amine or an alkylamine, each of which may have an alkyl group, a (hetero)cycloalkyl group, an alkoxycarbonyl group or an acyl group, each of which may have a hydroxy group, an alkoxy group, an amino group or a (di) alkylamino group, or a carboxamide group; or an alphatic, (hetero).cycloalkyl or (hetero)aryl carboxylic acid residue having an alicyclic amine optionally having an alkyl group, a (hetero)cycloalkyl group, an alkoxycarbonyl group or an acyl group, each of which may have a hydroxy group, an alkoxy group, an amino group or a (di)alkylamino group.

As the substituent that a (hetero)aryl group may have, a halogen atom, a hydroxy group; an alkyl group, an alkenyl group, an alkoxyl group, an alkylamino group, or an alkylthio group each of which may have further substituents $\alpha$; a- U-V-W-N($R^A$)-Y-Z group or a -U-V-COO-$R^B$ group is preferable. Among these substituents, a fluorine atom, a hydroxy group, a methyl group, an ethyl group, a hydroxymethyl group, a 2-hydroxyethyl group, a 3-hydroxypropyl group, a methoxy group, an ethoxy group, a 1-propyloxy group, a 2-propyloxy group, a 2-hydroxyethoxy group, a 3-hydroxypropyloxy group; a -U-V-W-N($R^A$) -Y-Z group or a -U-V-COO-$R^B$ group is more preferable. A fluorine atom, a hydroxy group, a methyl group, an ethyl group, a 2-hydroxyethyl group, a methoxy group, an ethoxy group, a 2-propyloxy group; a -U-V-W-N($R^A$)-Y-Z group or a -U-V-COO-$R^B$ group is the most preferable.

As the alicyclic amine, pyrrolidine, piperidine, piperazine, morpholine and the like can be illustrated.
As the amino acid, a natural amino acid (L-, D- or DL-form may be employed) or a synthetic amino acid may be employed.
As the synthetic amino acid, a homoamino acid such as 2-methylalanine and a noramino acid such as norvaline can be illustrated.
When U is -O- or -S-, V and W are not a single bond at the same time.

In case that any of $Q^1$ to $Q^5$ is a carbon atom, as the substituent bound thereto, for example, a halogen atom,

a hydroxy group, an amino group, a carboxyl group, a cyano group, a (di) alkylamino group or a cycloalkyloxy group; and an alkyl group, an alkoxy group, a cycloalkyl group and an alkoxycarbonyl group, each of which may have any substituents α are illustrated. Among them, a halogen atom, a hydroxy group; or an alkyl group or an alkoxy group each of which may have any substituents α is preferable, a fluorine atom, a chlorine atom, a hydroxy group, a methyl group, an ethyl group, a 2-propyl group, a trifluoromethyl group or a methoxy group is more preferable, and a fluorine atom, a chlorine atom or a methyl group is the most preferable.
The number of nitrogen atoms in $Q^1$ to $Q^5$ is preferably 0 to 2 in total, and more preferably 0, that is, all of $Q^1$ to $Q^5$ are more preferably a carbon atom bound to a hydrogen atom or a substituent.
E is preferably an alkylene group or -S-, especially a methylene group or -S-.
R is preferably a hydroxymethyl group.

The substituent α means a group selected from a group consisting of a halogen atom, a hydroxy group, an acyloxy group, an amino group, an acylamino group, a cyano group, a carboxyl group, a carbamoyl group, an alkoxy group, a (di) alkylamino group, an alkoxycarbonyl group, a hydroxyalkoxycarbonyl group, a (hetero)aryl group and a (hetero) cycloalkyl group, preferably selected from a group consisting of a halogen atom, a hydroxy group, an amino group, a cyano group, a carboxyl group, a carbamoyl group, an alkoxy group, a (di) alkylamino group, analkoxycarbonyl group, a hydroxyalkoxycarbonyl group, a (hetero) aryl group and a (hetero) cycloalkyl group in case that any groups have the plural substituents, these substituents may be the same or different.

An example of the processes for preparing a 1-(β-D-glycopyranosyl)-3-substituted nitrogen-containing heterocyclic compound represented by the general formula (I) of the present invention is shown below, and a β-D-glucopyranosyl group is used as the β-D-glycopyranosyl group to explain.

[Chem.2]

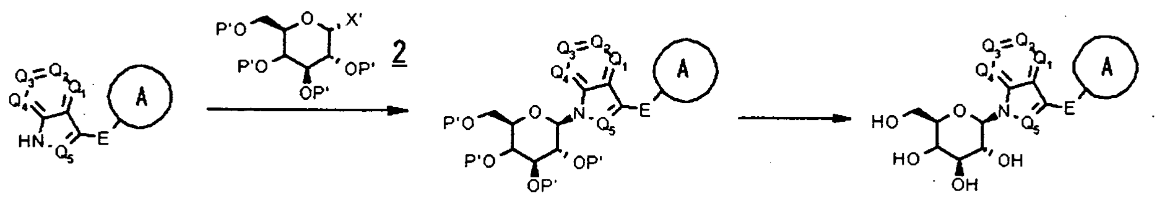

2

1 3 (I)

wherein ring A, $Q^1$ to $Q^5$ and E have the same meanings as defined above, P' represents a hydroxy-protective group, and X' represents a chlorine atom or a bromine atom.

In the following description, an α-D-glucopyranosyl chloride is used as an α-D-glycopyranosyl halide. A 1-(β-D-glucopyranosyl)-3-substituted nitrogen-containing heterocyclic compound (I) can be prepared by allowing a 3-substituted nitrogen-containing heterocyclic compound (1) to react with an α-D-glucopyranosyl halide (2) in which hydroxy groups are protected, and removing the hydroxy-protective groups in the given 1-(β-D-glucopyranosyl)-3-substituted nitrogen-containing heterocyclic compound (3).

The 3-substituted nitrogen-containing heterocyclic compound (1) and the α-D-glucopyranosyl chloride (2) in which hydroxy groups are protected can be commercially available or can be prepared by known processes.

As the hydroxy-protective group, any protective group commonly used in the field of sugar chemistry may be employed. For example, a carboxylic acyl group such as an acetyl group, a benzoyl group or a pivaloyl group, an optionally substituted benzyl group which may have any substituents such as a benzyl group or a p-methoxybenzyl group or the like can be illustrated.

The reaction may be performed by mixing them in an adequate solvent in the presence of silver compound such as silver carbonate or silver oxide, and optionally adding a phase transfer catalyst such as benzyl tri(n-butyl) ammonium chloride, benzyl tri(*n*-butyl)ammonium bromide or benzyl tri(*n*-butyl)ammonium hydrosulfate for from 1 hour to 3 days at from 0°C to reflux temperature of the solvent.

Alkali metal carbonate salt such as sodium carbonate, potassium carbonate, cesium carbonate or the like, an alkali metal hydroxide such as sodium hydroxide, potassium hydroxide or the like, or an alkali metal alkoxide such as potassium t-butoxide or the like can be used instead of the silver compound as a base.

As the reaction solvent, for example, water; ethers such as tetrahydrofuran, 1,2-dimethoxyethane, *t*-buthylmethyl-ether and the like; halogenated hydrocarbons such as methylene chloride and the like; aromatic hydrocarbons such as toluene, benzotrifluoride and the like; aprotic polar solvents such as acetonitrile, *N,N*-dimethylformamide and the like are illustrated.

The hydroxy-protective group can be removed by a common method in the sugar chemistry. For example, a

protective group which is a carboxylic acyl group may be removed by adding an alkali metal hydroxide such as sodium hydroxide or potassium hydroxide, or an alkali metal alkoxide such as sodium methoxide or sodium ethoxide as a base in an adequate solvent and then mixing for from 1 hour to 24 hours at from 0°C to reflux temperature of the reaction solvent.

As the reaction solvent, for example, water; alcohols such as methanol, ethanol, 2-propanol and the like; water-soluble ethers such as tetrahydrofuran, dioxane and the like are illustrated.

When the protective group is a benzyl group optionally having any substituents, it can be removed by hydogenolysis which may be performed in an adequate solvent by adding a noble metal catalyst such as palladium and the like and mixing under a hydrogen atmosphere at from atmospheric pressure to medium pressure at from 0°C to reflux temperature of the solvent for from 30 minutes to 24 hours.

As the reaction solvent, for example, alcohols such as methanol, ethanol and the like; ethers such as tetrahydrofuran and the like; carboxylic esters such as ethyl acetate and the like; carboxylic acid such as acetic acid and the like can be illustrated.

The benzyl group optionally having any substituents can also be removed by adding an acid such as boron trichloride, boron tribromide, boron trifluoride diethyl ether complex or the like, and optionally adding a thiol compound such as ethanethiol, and mixing them in an adequate solvent for from 30 minutes to 24 hours at from 0°C to reflux temperature of the solvent.

As the reaction solvent, for example, halogenated hydrocarbons such as methylene chloride, 1, 2-dichloroethane and the like; acetonitrile and the like are illustrated.

After the above mentioned deprotection reaction, the reaction mixture may be treated in the usual way and then a 1-(β-D-glucopyranosyl)-3-substituted nitrogen-containing heterocyclic compound (I) can be obtained optionally by a conventional purification method.

When a 3-substituted nitrogen-containing heterocyclic compound (1) is not available easily, a 1-(β-D-glucopyranosyl)-3- substituted nitrogen-containing heterocyclic compound (I) can be prepared by a variety of methods from which a skilled person can select. A typical example of the procedures is explained by following Manufacturing method 1 to Manufacturing method 5.

[Manufacturing method 1]

The manufacturing method of 1-(β-D-glucopyranosyl)-3-substituted nitrogen-containing heterocyclic compound (I) wherein E is a methylene group is as follows.

[Chem.3]

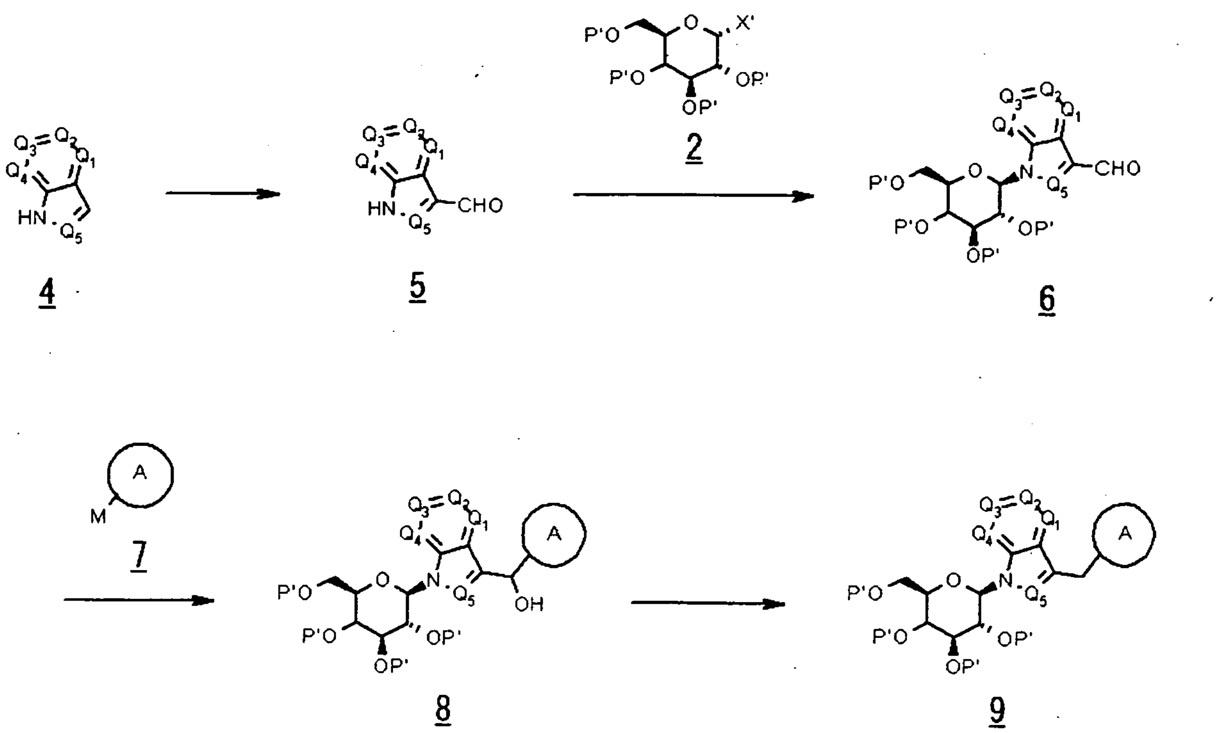

4 5 2 6 7 8 9

wherein ring A, $Q^1$ to $Q^5$, $E^1$, P' and X' have the same meanings as defined above, and M represents Li, MgCl, MgBr, MgI, ZnCl, ZnBr or ZnI.

A 1-(β-D-glucopyranosyl)-3-formyl nitrogen-containing heterocyclic compound (6) can be prepared by allowing a 3-formyl nitrogen-containing heterocyclic compound (5) prepared by subjecting a nitrogen-containing heterocyclic compound (4) to Vilsmeier reaction to react with an α-D-glucopyranosyl chloride (2). Furthermore, a 1-(β-D-glucopyranosyl)-3-formyl nitrogen-containing heterocyclic compound (6) can be also prepared by reacting a nitrogen-containing heterocyclic compound (4) and an α-D-glucopyranosyl chloride (2) and then subjecting the same to Vilsmeier reaction.

Next, the 1-(β-D-glucopyranosyl)-3-formyl nitrogen-containing heterocyclic compound (6) is allowed to react with an organic metal compound (7) such as a Grignard reagent or the like to obtain an adduct alcohol compound (8), and the hydroxy group of the compound (8) is removed by hydrogenolysis or the like to obtain a 1-(β-D-glucopyranosyl)-3-(hetero)arylmethyl nitrogen-containing heterocyclic compound (9). Furthermore, a 1-(β-D-glucopyranosyl)-3-substituted nitrogen-containing heterocyclic compound (I) wherein E is a methylene group can be prepared by deprotection of the compound (9).

[Manufacturing method 2]

A 1-(β-D-glucopyranosyl)-3-substituted nitrogen-containing heterocyclic compound (I) wherein E is a methylene group can be also prepared by the following method.

[Chem. 4]

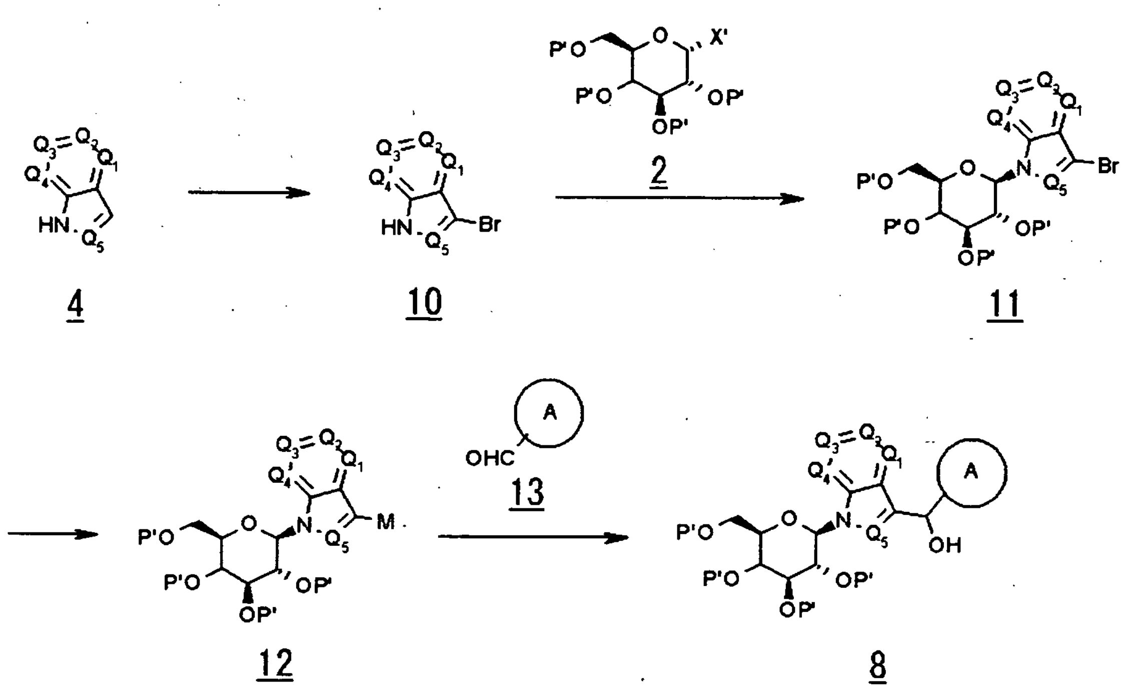

4

10

2

11

12

13

8

wherein ring A, $Q^1$ to $Q^5$, E, P', X' and M have the same meanings as defined above.

A 1-(β-D-glucopyranosyl)-3-bromo nitrogen-containing heterocyclic compound (11) is prepared by bromination of a nitrogen-containing heterocyclic compound (4) at 3 position and allowing given 3-bromo nitrogen-containing heterocyclic compound (10) to react with an α-D-glucopyranosyl chloride (2). The organic metal compound (12) prepared from the compound (11) is allowed to react with a (hetero)arylcarbaldehyde (13) to obtain an adduct alcohol compound (8) in Manufacturing method 1 and a 1-(β-D-glucopyranosyl)-3-substituted nitrogen-containing heterocyclic compound (I) wherein E is a methylene group can be also prepared from the same by Manufacturing method 1.

[Manufacturing method 3]

A 1-(β-D-glucopyranosyl)-3-substituted nitrogen-containing heterocyclic compound (I) wherein E is an ethylene group can be prepared as follows.

[Chem.5]

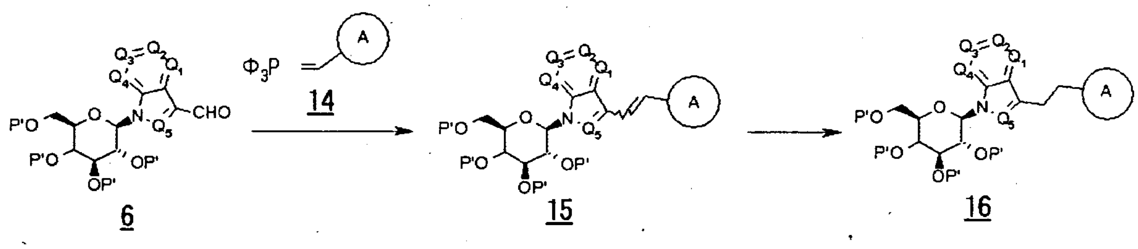

6 14 15 16

wherein ring A, $Q^1$ to $Q^5$ and P' have the same meanings as defined above.

A 1-(β-D-glucopyranosyl)-3-formyl nitrogen-containing heterocyclic compound (6) is allowed to react with a Wittig reagent (14) prepared from a (hetero)arylmethyl halide or the like to obtain an olefin compound (15). After reducing the olefin part of the compound (15), resulted 1-(β-D-glucopyranosyl)-3-[2-(hetero)arylethyl] nitrogen-containing heterocyclic compound (16) is deprotected to obtain a 1-(β-D-glucopyranosyl)-3-substituted nitrogen-containing heterocyclic compound,(I) wherein E is an ethylene group. Further, a 1-(β-D-glucopyranosyl)-3- substituted nitrogen-containing heterocyclic compound (I) wherein E is an ethylene group can be also prepared by subjecting the olefin compound (15) to deprotection and reducing the olefin part of the same.

[Manufacturing method 4]

A 1-(β-D-glucopyranosyl)-3-substituted nitrogen-containing heterocyclic compound (I) wherein E is -O- or -NH- can be prepared by the following method.

[Chem.6]

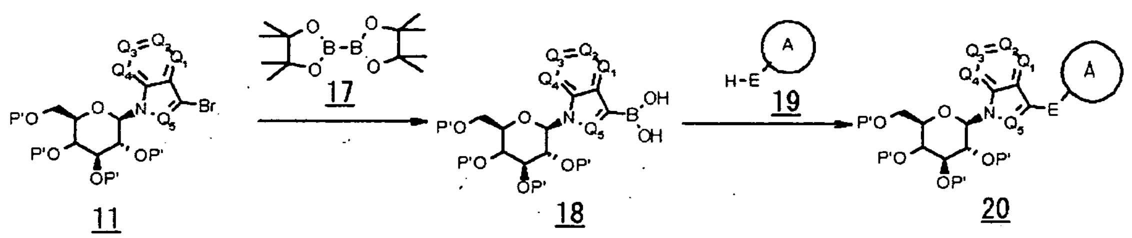

11 17 18 19 20

wherein ring A, $Q^1$ to $Q^5$ and P' have the same meanings as defined above and E represents -O- or -NH-.

A 1-(β-D-glucopyranosyl)-3-bromo nitrogen-containing heterocyclic compound (11) is allowed to react with a diboron (17) to obtain a boric ester. The boric ester is hydrolyzed to a 1-(β-D-glucopyranosyl) nitrogen-containing heterocyclic 3-boronic acid compound(18). The compound (18) is allowed to react with a hydroxy(hetero)aryl or an amino (hetero)aryl compound (19) to obtain a 1- (β-D-glucopyranosyl)-3-substituted nitrogen-containing heterocyclic compound (20) wherein E is -O- or -NH-. The compound (20) is deprotected to prepare a 1-(β-D-glucopyranosyl)-3-substituted nitrogen-containing heterocyclic compound (I) wherein E is -O- or -NH-.

[Manufacturing method 5]

A 1-(β-D-glucopyranosyl)-3-substituted nitrogen-containing heterocyclic compound (I) wherein E is -Scan be prepared by the following method.

[Chem.7]

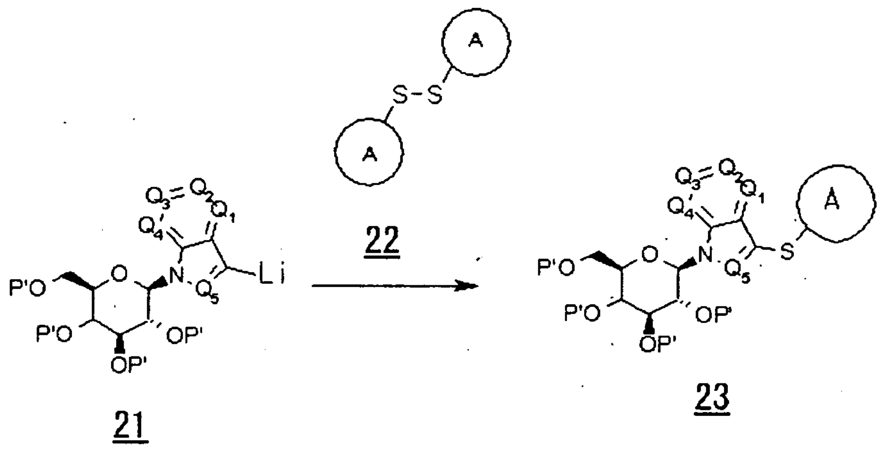

21 22 23

wherein ring A, $Q^1$ to $Q^5$ and P' have the same meanings as defined above.

A lithium compound (21) prepared from a 1-(β-D-glucopyranosyl)-3-bromo nitrogen-containing heterocyclic compound (11) is allowed to react with a di (hetero) aryl disulfide (22) to obtain a 1-(β-D-glucopyranosyl)-3-(hetero) arylthio nitrogen-containing heterocyclic compound (23). The compound (23) is deprotected to prepare a 1-(β-D-glucopyranosyl)-3-substituted nitrogen-containing heterocyclic compound (I) wherein E is -S-.

[Manufacturing method 6]

A 1-(β-D-glucopyranosyl)-3-substituted nitrogen-containing heterocyclic compound (I) wherein E is -S- can be also prepared by the following method.

[Chem.8]

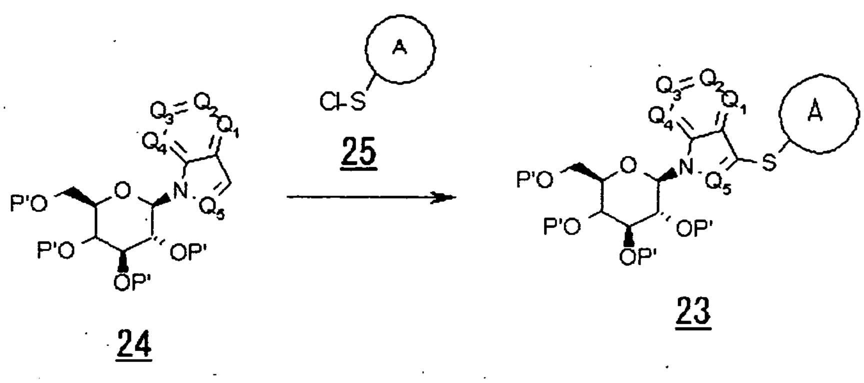

24 25 23

wherein ring A, $Q^1$ to $Q^5$ and P' have the same meanings as defined above.

A 1-(β-D-glucopyranosyl) nitrogen-containing heterocyclic compound (24) is allowed to react with a (hetero) arylsulfenyl chloride (25) prepared from a di(hetero)aryl disulfide (22) or a (hetero)arylmercaptan to obtain a 1-(β-D-glucopyranosyl)-3- (hetero)arylthio nitrogen-containing heterocyclic compound (23). By deprotecting the compound (23), a 1-(β-D-glucopyranosyl)-3-substituted nitrogen-containing heterocyclic compound (I) wherein E is -S- can be prepared.

Further, in the above-mentioned Manufacturing methods 1 to 5, a nitrogen-containing heterocyclic compound (4) wherein $Q^1$ to $Q^5$ is independently a carbon atom bound to a hydrogen atom or a substituent, that is, an indole compound, may be prepared by known methods such as Fisher' s indole synthesis or appropriate chemical modification to a commercially available indole compound.

A nitrogen-containing heterocyclic compound (4) wherein any of $Q^1$ to $Q^5$ is a nitrogen atom may be prepared by using a commercially available compound such as 4-azaindole (APIN Co.), 5-azaindole (APIN Co.), 6-azaindole (ASTATECH Co.) or 7-azaindole (ALDRICH Co.), or a compound prepared by known methods, for example, 4,6-diazaindole (Ektova,L.V.et. al., Khikiko-Farmatsevticheskii Zhurnal, 22(7), 860-3,1988) or their combination as the occasion. As the above azaindole compound can be acylated at 3-position according to Zhongxing Zhang's method (J. Org.Chem. 2002,67,622-6227), a nitrogen-containing heterocyclic compound having a desirable substituent can be prepared by conventionally used method. Further, 1H-indazole-3-carbaldehyde is available from CHEMPACIFIC Co..

In addition, a glucopyranose derivative corresponding to the α-D-glucopyranosyl chloride (2) such as 6-deoxy-6-fluoro-D-glucopyranose (CMS-CHEMICAL Co.), 6-deoxy-6-fluoro-D-galactopyranose (MATRIX Co.) and 6-deoxy-D-glucose (SIGMA Co.) are commercially available.

A substituent of the compound having ring A can be introduced to an easily available compound having ring A by optionally combining conventional halogenation, amination, nitration, sulfonation, diazotization, thiolation, esterification, amidation, oxidation, reduction, dehydration, hydrolization, coupling and the like (for example, see WO04/014932 and WO04/018491 pamphlets).

When a compound used or generated in the above mentioned manufacturing methods has a functional group which changes in the reaction condition or inhibits the reaction progression, the group may be protected by an appropriate protective group used by a skilled person in the art and remove the protecting group in an appropriate step.

A 1-(β-D-glycopyranosyl)-3-substituted nitrogen-containing heterocyclic compound represented by above general formula (I) of the present invention may be allowed to react with a reagent forming a prodrug, for example, a halogenated alkyl such as ethyl chloride, benzyl chloride or the like; a halogenated acyl such as acetyl chloride, benzoyl chloride or the like; a halogenated formyl ester such as ethyl chloroformate, benzyl chloroformate or the like to obtain a prodrug wherein a carboxyl group, a hydroxy group and/or an amino group is converted by conventional method.

A 1-(β-D-glycopyranosyl)-3-substituted nitrogen-containing heterocyclic compound represented by the general formula (I) or a prodrug thereof can be converted into a pharmaceutically acceptable salt by conventional method. As the salt, for example, a salt with an inorganic acid such as hydrochloric acid, nitric acid and the like; a salt with an organic acid such as acetic acid, methanesulfonic acid and the like and a sodium salt and a potassium salt ; and a salt with an organic base such as *N,N'*-dibenzyletylenediamine, 2-aminoethanol and the like can be illustrated.

Occasionally a 1-(β-D-glycopyranosyl)-3-substituted nitrogen-containing heterocyclic compound represented by the general formula (I) or a prodrug thereof is obtained as a hydrate or a solvate thereof after purification or salt formation process. For the pharmaceutical composition of the present invention, any of the 1-(β-D-glycopyranosyl)-3-substituted nitrogen-containing heterocyclic compound, any of a prodrug thereof, or a pharmaceutically acceptable salt thereof, or a hydrate or solvate thereof can be employed.

Furthermore, a 1-(β-D-glycopyranosyl)-3-substituted nitrogen-containing heterocyclic compound represented by the general formula (I) or a prodrug thereof sometimes has tautomers, geometrical isomers and/or optical isomers. For the pharmaceutical composition of the present invention, any of the isomers and a mixture thereof can be employed.

A Pharmaceutical composition may be prepared by mixing a 1-(β-D-glycopyranosyl)-3-substituted nitrogen-containing heterocyclic compound (I) of the present invention, or a prodrug thereof or a pharmaceutically acceptable salt thereof, or a hydrate or solvate thereof and a conventional pharmaceutical carrier.

The pharmaceutical carrier may be used optionally in combination according to a dosage form as described below. As the pharmaceutical carrier, additives such as lactose or the like; lubricants such as magnesium stearate or the like; disintegrators such as carboxymethyl cellulose or the like; binders such as hydroxypropylmethylcellulose or the like; surfactants such as macrogol or the like; foamings such as sodium bicarbonate or the like; dissolving aids such as cyclodextrin or the like; acidities such as citric acid or the like; stabilizers such as sodium edeate or the like; pH controls such as phosphoric acid salt or the like can be illustrated.

As the dosage form of the pharmaceutical composition of the present invention, oral administrations such as powders, granules, fine granules, dry syrups, tablets, capsules or the like; parenteral administrations such as injections, poultices, suppositories or the like are illustrated.

As the 1-(β-D-glycopyranosyl)-3-substituted nitrogen-containing heterocyclic compound represented by the general formula (I) shows a potent inhibitory activity against human SGLT1 and/or SGLT2 in human SGLT1 and SGLT2 inhibitory activity confirmatory tests, it can inhibit the postprandial increase of the blood sugar lever increase by inhibiting the absorption of glucose or galactose, or normalize the blood glucose level by inhibiting the reabsorption of glucose. Accordingly, the pharmaceutical composition of the present invention can be used as an inhibitor of postprandial hyperglycemia, or for manufacturing an agent for the prevention or treatment of a disease selected from a group consisting of diabetes, impaired glucose tolerance, diabetic complications (for example, retinopathy, neuropathy, nephropathy, ulcer, macroangiopathy), obesity, hyperinsulinemia, galactosemia, hyperlipidemia, hypercholesterolemia, hypertriglyceridemia, lipid metabolism disorder, atherosclerosis, hypertension, congestive heart failure, edema, hyperuricemia and gout, or the inhibition of impaired glucose tolerance advancing into diabetes.

For manufacturing the above agent for the prevention or treatment, the dosage of the compound represented by the general formula (I) of the present invention, or a pharmaceutically acceptable salt thereof, or a hydrate or solvate thereof is appropriately within the range of from 0.1 to 1,000 mg per day per adult human in case of oral administration and approximately within the range of from 0.01 to 100 mg par day per adult human in the case of parenteral administration

in formulation.

Furthermore, a drug of the present invention can be used in combination with other drug (s). Examples of such other drugs include an insulin sensitivity enhancer, an amylase inhibitor, an α-glucosidase inhibitor, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, a glycogen synthase kinase-3 inhibitor, an 11β-hydroxysteroiddehydrogenaze inhibitor, glucagon-like peptide-1, a glucagon-like peptide-1 analogue, a glucagon-like peptide-lagonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid dipeptidase inhibitor, insulin-like growth factor-I, platelet-derived growth factor (PDGF), a platelet-derived growth factor (PDGF) analogue (e.g., PDGF-AA, PDGF-BB, PDGF-AB), epidermal growth factor (EGF), nerve growth factor, a carnitine derivative, uridine, 5-hydroxy-1-methylhidantoin, EGB-761, bimoclomol, sulodexide, Y-128, an antidiarrhoics, a cathartics, a hydroxymethylglutaryl coenzyme A reductase inhibitor, a fibrate, a $\beta_3$-adrenoceptor agonist, an acyl-coenzyme A: cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyltransferase inhibitor, a squalene synthase inhibitor, a squalene epoxidase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an $\alpha_2$-adrenoceptor agonist, an antiplatelets agent, a uric acid synthesis inhibitor, a uricosuric agent, and a urinary alkalinizer.

As an insulin sensitivity enhancer, peroxisome proliferator-activated receptor-γ agonists such as troglitazone, pioglitazone hydrochloride, rosiglitazone maleate, sodium darglitazone, GI-262570, isaglitazone, LG-100641, NC-2100, T-174, DRF-2189, CLX-0921, CS-011, GW-1929, ciglitazone, sodium englitazone and NIP-221, peroxisome proliferator-activated receptor-α agonists such as GW-9578 and BM-170744, peroxisome proliferator-activated receptor-α/γ agonists such as GW-409544, KRP-297, NN-622, CLX-0940, LR-90, SB-219994, DRF-4158 and DRF-MDX8, retinoid X receptor agonists such as ALRT-268, AGN-4204, MX-6054, AGN-194204, LG-100754 and bexarotene, and other insulin sensitivity enhancers such as reglixane, ONO-5816, MBX-102, CRE-1625, FK-614, CLX-0901, CRE-1633, NN-2344, BM-13125, BM-501050, HQL-975, CLX-0900, MBX-668, MBX-675, S-15261, GW-544, AZ-242, LY-510929, AR-H049020 and GW-501516 are illustrated. Insulin sensitivity enhancers are used preferably for the prevention or treatment of diabetes, impaired glucose tolerance, diabetic complications, obesity, hyperinsulinemia, hyperlipidemia, hypercholesterolemia, hypertriglyceridemia, lipid metabolism disorder or atherosclerosis, and more preferably for the prevention or treatment of diabetes, impaired glucose tolerance or hyperinsulinemia because of improving the disturbance of insulin signal transduction in peripheral tissues and enhancing glucose uptake into the tissues from the blood, leading to lowering of blood glucose level.

As an amylase inhibitor, for example, RSH-2083 and the like are illustrated.
As an α-glucosidase inhibitor, for example, acarbose, voglibose, miglitol, CKD-711, emiglitate, MDL-25,637, camiglibose andMDL-73, 945, AZM-127 and the like are illustrated.
Amylase inhibitor or α-glucosidase inhibitor is used preferably for the prevention or treatment of diabetes, impaired glucose tolerance, diabetic complications, obesity or hyperinsulinemia. They are used more preferably for the prevention or treatment of impaired glucose tolerance because of inhibiting the gastrointestinal enzymatic digestion of carbohydrates contained in foods, and inhibiting, delaying the absorption of glucose or the like into the body.

As a biguanide, for example, phenformin, buformin hydrochloride, metformin hydrochloride or the like are illustrated. Biguanides are used preferably for the prevention or treatment of diabetes, impaired glucose tolerance, diabetic complications or hyperinsulinemia, and more preferably for the prevention or treatment of diabetes, impaired glucose tolerance or hyperinsulinemia because of lowering blood glucose level by inhibitory effects on hepatic gluconeogenesis, accelerating effects on anaerobic glycolysis in tissues or improving effects on insulin resistance in peripheral tissues.

Asaninsulinsecretion enhancer,for example,tolbutamide, chlorpropamide, tolazamide, acetohexamide, glyclopyramide, glyburide (glibenclamide), gliclazide, 1-butyl-3-metanilyl-urea, carbutamide, glibornuride, glipizide, gliquidone, glisoxapide, glybuthiazol, glybuzole, glyhexamide, sodium glymidine, glypinamide, phenbutamide, tolcyclamide, glimepiride, nateglinide, mitiglinide calcium hydrate, repaglinide or the like are illustrated. In addition, the insulin secretion enhancers include glucokinase activators such as RO-28-1675. Insulin secretion enhancers are used preferably for the prevention or treatment of diabetes, impaired glucose tolerance or diabetic complications, and more preferably for the prevention or treatment of diabetes or impaired glucose tolerance because of lowering blood glucose level by acting on

pancreatic β-cells and enhancing the insulin secretion.

As insulin or an insulin analogue, for example, human insulin, animal-derived insulin, human or animal-derived insulin analogues or the like are illustrated. These preparations are used preferably for the prevention or treatment of diabetes, impaired glucose tolerance or diabetic complications, and more preferably for the prevention or treatment of diabetes or impaired glucose tolerance.

As a glucagon receptor antagonist, for example, BAY-27-9955, NNC-92-1687 or the like are illustrated; as insulin receptor kinase stimulants, TER-17411, L-783281, KRX-613 or the like are illustrated; as tripeptidyl peptidase II inhibitors, UCL-1397 or the like are illustrated; as dipeptidyl peptidase IV inhibitors, for example, NVP-DPP728A, TSL-225, P-32/98 or the like are illustrated; as protein tyrosine phosphatase 1B inhibitors, for example, PTP-112, OC-86839, PNU-177496 or the like are illustrated; as glycogen phosphorylase inhibitors, for example, NN-4201, CP-368296 or the like are illustrated; as fructose-bisphosphatase inhibitors, for example, R-132917 or the like are illustrated; as pyruvate dehydrogenase inhibitors, for example, AZD-7545 or the like are illustrated; as hepatic gluconeogenesis inhibitors, for example, FR-225659 or the like are illustrated; as glucagon-like peptide-1 analogues, for example, exendin-4, CJC-1131 or the like are illustrated; as glucagon-like peptide 1 agonists; AZM-134, LY-315902 or the like are illustrated; and as amylin, amylin analogues or amylin agonists, for example, pramlintide acetate or the like are illustrated. These drugs, glucose-6-phosphatase inhibitors, D-chiroinositol, glycogen synthase kinase-3 inhibitors and glucagon-like peptide-1 are used preferably for the prevention or treatment of diabetes, impaired glucose tolerance, diabetic complications or hyperinsulinemia, and more preferably for the prevention or treatment of diabetes or impaired glucose tolerance.

As an aldose reductase inhibitor, for example, ascorbyl gamolenate, tolrestat, epalrestat, ADN-138, BAL-ARI8, ZD-5522, ADN-311, GP-1447, IDD-598, fidarestat, sorbinil, ponalrestat, risarestat, zenarestat, minalrestat, methosorbinil, AL-1567, imirestat, M-16209, TAT, AD-5467, zopolrestat, AS-3201, NZ-314, SG-210, JTT-811, lindolrestat or the like are illustrated. Aldose reductase inhibitors are preferably used for the prevention or treatment of diabetic complications because of inhibiting aldose reductase and lowering excessive intracellular accumulation of sorbitol in accelerated polyol pathway which are in continuous hyperglycemic condition in the tissues in diabetic complications.

As an advanced glycation endproduct formation inhibitors, for example, pyridoxamine, OPB-9195, ALT-946, ALT-711, pimagedine hydrochloride or the like are illustrated. Advanced glycation endproducts formation inhibitors are preferably used for the prevention or treatment of diabetic complications because of inhibiting formation of advanced glycation endproducts which are accelerated in continuous hyperglycemic condition in diabetes and declining of cellular damage.

As a protein kinase C inhibitor, for example, LY-333531, midostaurin or the like are illustrated. Protein kinase C inhibitors are preferably used for the prevention or treatment of diabetic complications because of inhibiting of protein kinase C activity which is accelerated in continuous hyperglycemic condition in diabetes.

As a γ-aminobutyric acid receptor antagonist, for example, topiramate or the like are illustrated; as sodium channel antagonists, for example, mexiletine hydrochloride, oxcarbazepine or the like are illustrated; as transcrit factor NF-κB inhibitors, for example, dexlipotam or the like are illustrated; as lipid peroxidase inhibitors, for example, tirilazad mesylate or the like are illustrated; as *N*-acetylated-α-linked-acid-dipeptidase inhibitors, for example, GPI-5693 or the like are illustrated; and as carnitine derivatives, for example, carnitine, levacecarnine hydrochloride, levocarnitine chloride, levocarnitine, ST-261 or the like are illustrated. These drugs, insulin-like growth factor-I, platelet-derived growth factor, platelet derived growth factor analogues, epidermal growth factor, nerve growth factor, uridine, 5-hydroxy-1-methylhidantoin, EGB-761, bimoclomol, sulodexide and Y-128 are preferably used for the prevention or treatment of diabetic complications.

As an antidiarrhoics or cathartic, for example, polycarbophil calcium, albumin tannate, bismuth subnitrate or the like are illustrated. These drugs are preferably used for the prevention or treatment of diarrhea, constipation or the like accompanying diabetes or the like.

As a hydroxymethylglutaryl coenzyme A reductase inhibitor, for example, sodium cerivastatin, sodium pravastatin, lovastatin, simvastatin, sodium fluvastatin, atorvastatin calciumhydrate, SC-45355, SQ-33600, CP-83101, BB-476, L-669262, S-2468, DMP-565, U-20685, BAY-x-2678, BAY-10-2987, calcium pitavastatin, calcium rosuvastatin, colestolone, dalvastatin, acitemate, mevastatin, crilvastatin, BMS-180431, BMY-21950, glenvastatin, carvastatin, BMY-22089, bervastatin or the like are illustrated. Hydroxymethylglutaryl coenzyme A reductase inhibitors are used preferably for the prevention or treatment of hyperlipidemia, hypercholesterolemia, hypertriglyceridemia, lipid metabolism disorder or atherosclerosis, and more preferably for the prevention or treatment of hyperlipidemia, hypercholesterolemia or atherosclerosis because of lowering blood cholesterol level by inhibiting hydroxymethylglutaryl coenzyme A reductase.

As a fibrate, for example, bezafibrate, beclobrate, binifibrate, ciprofibrate, clinofibrate, clofibrate, aluminum clofibrate, clofibric acid, etofibrate, fenofibrate, gemfibrozil, nicofibrate, pirifibrate, ronifibrate, simfibrate, theofibrate, AHL-157 or the like are illustrated. Fibric acid derivatives are used preferably for the prevention or treatment of hyperinsulinemia, hyperlipidemia, hypercholesterolemia, hypertriglyceridemia, lipid metabolism disorder or atherosclerosis, and more preferably for the prevention or treatment of hyperlipidemia, hypertriglyceridemia or atherosclerosis because of activating hepatic lipoprotein lipase and enhancing fatty acid oxidation, leading to lowering of blood triglyceride level.

As a $\beta_3$-adrenoceptor agonist, for example, BRL-28410, SR-58611A, ICI-198157, ZD-2079, BMS-194449, BRL-37344, CP-331679, CP-114271, L-750355, BMS-187413, SR-59062A, BMS-210285, LY-377604, SWR-0342SA, AZ-40140, SB-226552, D-7114, BRL-35135, FR-149175, BRL-26830A, CL-316243, AJ-9677, GW-427353, N-5984, GW-2696, YM178 or the like are illustrated. $\beta_3$-Adrenoceptor agonists are used preferably for the prevention or treatment of obesity, hyperinsulinemia, hyperlipidemia, hypercholesterolemia, hypertriglyceridemia or lipid metabolism disorder, and more preferably for the prevention or treatment of obesity or hyperinsulinemia because of stimulating $\beta_3$-adrenoceptor in adipose tissue and enhancing the fatty acid oxidation, leading to induction of energy expenditure.

As an acyl-coenzyme A cholesterol acyltransferase inhibitor, for example, NTE-122, MCC-147, PD-132301-2, DUP-129, U-73482, U-76807, RP-70676, P-06139, CP-113818, RP-73163, FR-129169, FY-038, EAB-309, KY-455, LS-3115, FR-145237, T-2591, J-104127, R-755, FCE-28654, YIC-C8-434, avasimibe, CI-976, RP-64477, F-1394, eldacimibe, CS-505, CL-283546, YM-17E, lecimibide, 447C88, YM-750, E-5324, KW-3033, HL-004, eflucimibe or the like are illustrated. Acyl-coenzyme A cholesterol acyltransferase inhibitors are used preferably for the prevention or treatment of hyperlipidemia, hypercholesterolemia, hypertriglyceridemia or lipid metabolism disorder, and more preferably for the prevention or treatment of hyperlipidemia or hypercholesterolemia because of lowering blood cholesterol level by inhibiting acyl-coenzyme A cholesterol acyltransferase.

As a thyroid hormone receptor agonist, for example, sodium liothyronine, sodium levothyroxine, KB-2611 or the like are illustrated; as cholesterol absorption inhibitor, for example, ezetimibe, SCH-48461 or the like are illustrated; as lipase inhibitor, for example, orlistat, ATL-962, AZM-131, RED-103004 or the like are illustrated; as carnitine palmitoyltransferase inhibitor, for example, etomoxir or the like are illustrated; as squalene synthase inhibitor, for example, SDZ-268-198, BMS-188494, A-87049, RPR-101821, ZD-9720, RPR-107393, ER-27856, TAK-475 or the like are illustrated; as nicotinic acid derivative, for example, nicotinic acid, nicotinamide, nicomol, niceritrol, acipimox, nicorandil or the like are illustrated; as bile acid sequestrant, for example, colestyramine, colestilan, colesevelam hydrochloride, GT-102-279 or the like are illustrated; as sodium/bile acid cotransporter inhibitor, for example, 264W94, S-8921, SD-5613 or the like are illustrated; and as cholesterol ester transfer protein inhibitor, for example, PNU-107368E, SC-795, JTT-705, CP-529414 or the like are illustrated. These drugs, probcol, microsomal trigylceride transfer protein inhibitor, lipoxygenase inhibitor and low-density lipoprotein receptor enhancer are preferably used for the prevention or treatment of hyperlipidemia, hypercholesterolemia, hypertriglyceridemia or lipid metabolism disorder.

As an appetite suppressant, for example, monoamine reuptake inhibitor, serotonin reuptake inhibitor, serotonin releasing stimulant, serotonin agonist (especially $5HT_{2C}$-agonist), noradrenaline reuptake inhibitor, noradrenaline releasing stimulant, $\alpha_1$-adrenoceptor agonist, $\beta_2$-adrenocept or agonist, dopamine agonist, cannabinoid receptor antagonist, $\gamma$-aminobutyric acid receptor antagonist, $H_3$-histamine antagonist, L-histidine, leptin, leptin analogue, leptin receptor agonist, melanocortin receptor agonist (especially, MC3-R agonists, MC4-R agonist), $\alpha$-melanocyte stimulating hormone, cocaine-and amphetamine-regulated transcript, mahogany protein, enterostatin agonist, calcitonin, calcitonin-gene-related peptide, bombesin, cholecystokinin agonist (especially CCK-A agonist), corticotropin-releasing hormone, corticotropin-releasing hormone analogue, corticotropin-releasing hormone agonist, urocortin, somatostatin, somatostatin analogues, somatostatin receptor agonist, pituitary adenylate cyclase-activating peptide, brain-derived neurotrophic factor, ciliary neurotrophic factor, thyrotropin-releasing hormone, neurotensin, sauvagine, neuropeptide Y antagonists, opioid peptide antagonist, galanin antagonist, melanin-concentrating hormone antagonist, agouti-related protein inhibitor and orexin receptor antagonist are illustrated. Concretely, as monoamine reuptake inhibitor, mazindol or the like are illustrated; as serotonin reuptake inhibitor, dexfenfluramine hydrochloride, fenfluramine, sibutramine hydrochloride, fluvoxamine maleate, sertraline hydrochloride or the like are illustrated; as serotonin agonist, inotriptan, (+)-norfenfluramine or the like are illustrated; as noradrenaline reuptake inhibitor, bupropion, GW-320659 or the like are illustrated; as noradrenaline releasing stimulant, rolipram, YM-992 or the like are illustrated; as $\beta_2$-adrenoceptor agonist, amphetamine, dextroamphetamine, phentermine, benzphetamine, methamphetamine, phendimetrazine, phenmetrazine, diethylpropion, phenylpropanolamine, clobenzorex or the like are illustrated; as dopamine agonist, ER-230, doprexin, bromocriptine mesylate or the like are illustrated; as cannabinoid receptor antagonist, rimonabant or the like are illustrated; as $\gamma$-aminobutyric acid receptor antagonist, topiramate or the like are illustrated; as $H_3$-histamine antagonist, GT-2394 or the like are illustrated; as leptin, leptin analogues or leptin receptor agonist, LY-355101 or the like are illustrated; as cholecystokinin agonist (especially CCK-A agonist), SR-146131, SSR-125180, BP-3.200, A-71623, FPL-15849, GI-248573, GW-7178, GI-181771, GW-7854, A-71378 or the like are illustrated; and as neuropeptide Y antagonist, SR-120819-A, PD-160170, NGD-95-1, BIBP-3226, 1229-U-91, CGP-71683, BIBO-3304, CP-671906-01, J-115814 or the like are illustrated. Appetite suppressant are used preferably for the prevention or treatment of diabetes, impaired glucose tolerance, diabetic complications, obesity, hyperlipidemia, hypercholesterolemia, hypertriglyceridemia, lipid metabolism disorder, atherosclerosis, hypertension, congestive heart failure, edema, hyperuricemia or gout, and more preferably for the prevention or treatment of obesity because of stimulating or inhibiting the activities of intracerebral monoamines or bioactive peptides in central appetite regulatory system and suppressing the appetite, leading to reduction of energy intake.

As an angiotensin-converting enzyme inhibitor, for example, captopril, enalapri maleate, alacepril, delapril

hydrochloride, ramipril, lisinopril, imidapril hydrochloride, benazepril hydrochloride, ceronapril monohydrate, cilazapril, sodium fosinopril, perindopril erbumine, calcium moveltipril, quinapril hydrochloride, spirapril hydrochloride, temocapril hydrochloride, trandolapril, calcium zofenopril, moexipril hydrochloride, rentiapril or the like are illustrated. Angiotensin-converting enzyme inhibitor is preferably used for the prevention or treatment of diabetic complications or hypertension.

As a neutral endopeptidase inhibitor, for example, omapatrilat, MDL-100240, fasidotril, sampatrilat, GW-660511X, mixanpril, SA-7060, E-4030, SLV-306, ecadotril or the like are illustrated. Neutral endopeptidase inhibitor is preferably used for the prevention or treatment of diabetic complications or hypertension.

As an angiotensin II receptor antagonist, for example, candesartan cilexetil, candesartan cilexetil/hydrochlorothiazide, potassium losartan, eprosartan mesylate, valsartan, telmisartan, irbesartan, EXP-3174, L-158809, EXP-3312, olmesartan, tasosartan, KT-3-671, GA-0113, RU-64276, EMD-90423, BR-9701 or the like are illustrated. Angiotensin II receptor antagonist is preferably used for the prevention or treatment of diabetic complications or hypertension.

As an endothelin-converting enzyme inhibitor, for example, CGS-31447, CGS-35066, SM-19712 or the like are illustrated; as endothelin receptor antagonists, for example, L-749805, TBC-3214, BMS-182874, BQ-610, TA-0201, SB-215355, PD-180988, sodium sitaxsentan, BMS-193884, darusentan, TBC-3711, bosentan, sodium tezosentan, J-104132, YM-598, S-0139, SB-234551, RPR-118031A, ATZ-1993, RO-61-1790, ABT-546, enlasentan, BMS-207940 or the like are illustrated. These drugs are preferably used for the prevention or treatment of diabetic complications or hypertension, and more preferably for the prevention or treatment of hypertension.

As a diuretic agent, for example, chlorthalidone, metolazone, cyclopenthiazide, trichloromethiazide, hydrochlorothiazide, hydroflumethiazide, benzylhydrochlorothiazide, penflutizide, methyclothiazide, indapamide, tripamide, mefruside, azosemide, etacrynic acid, torasemide, piretanide, furosemide, bumetanide, meticrane, potassium canrenoate, spironolactone, triamterene, aminophylline, cicletanine hydrochloride, LLU-$\alpha$, PNU-80873A, isosorbide, D-mannitol, D-sorbitol, fructose, glycerin, acetazolamide, methazolamide, FR-179544, OPC-31260, lixivaptan, conivaptan hydrochloride or the like are illustrated. Diuretic drug is preferably used for the prevention or treatment of diabetic complications, hypertension, congestive heart failure or edema, and more preferably for the prevention or treatment of hypertension, congestive heart failure or edema because of reducing blood pressure or improving edema by increasing urinary excretion.

As a calcium antagonist, for example, aranidipine, efonidipine hydrochloride, nicardipine hydrochloride, barnidipine hydrochloride, benidipine hydrochloride, manidipine hydrochloride, cilnidipine, nisoldipine, nitrendipine, nifedipine, nilvadipine, felodipine, amlodipine besilate, pranidipine, lercanidipine hydrochloride, isradipine, elgodipine, azelnidipine, lacidipine, vatanidipine hydrochloride, lemildipine, diltiazem hydrochloride, clentiazem maleate, verapamil hydrochloride, S-verapamil, fasudil hydrochloride, bepridil hydrochloride, gallopamil hydrochloride or the like are illustrated; as vasodilating antihypertensive agents, for example, indapamide, todralazine hydrochloride, hydralazine hydrochloride, cadralazine, budralazine or the like are illustrated; as sympathetic blocking agents, for example, amosulalol hydrochloride, terazosin hydrochloride, bunazosin hydrochloride, prazosin hydrochloride, doxazosin mesylate, propranolol hydrochloride, atenolol, metoprolol tartrate, carvedilol, nipradilol, celiprolol hydrochloride, nebivolol, betaxolol hydrochloride, pindolol, tertatolol hydrochloride, bevantolol hydrochloride, timolol maleate, carteolol hydrochloride, bisoprolol hemifumarate, bopindolol malonate, nipradilol, penbutolol sulfate, acebutolol hydrochloride, tilisolol hydrochloride, nadolol, urapidil, indoramin or the like are illustrated; as centrally acting antihypertensive agent, for example, reserpine or the like are illustrated; and as $\alpha_2$-adrenoceptor agonist, for example, clonidine hydrochloride, methyldopa, CHF-1035, guanabenz acetate, guanfacine hydrochloride, moxonidine, lofexidine, talipexole hydrochloride or the like are illustrated. These drugs are preferably used for the prevention or treatment of hypertension.

As an antiplatelets agent, for example, ticlopidine hydrochloride, dipyridamole, cilostazol, ethyl icosapentate, sarpogrelate hydrochloride, dilazep dihydrochloride, trapidil, beraprost sodium, aspirin or the like are illustrated. Antiplatelets agent is preferably used for the prevention or treatment of atherosclerosis or congestive heart failure.

As a uric acid synthesis inhibitor, for example, allopurinol, oxypurinol or the like are illustrated; as uricosuric agents, benzbromarone, probenecid or the like are illustrated; and as urinary alkalinizers, sodium hydrogen carbonate, potassium citrate, sodium citrate or the like are illustrated. These drugs are preferably used for the prevention or treatment of hyperuricemia or gout.

As the other drug combined with the 1-($\beta$-D-glucopyranosyl)-3-substituted nitrogen-containing heterocyclic compound of the present invention in the use for the prevention or treatment of diabetes, for example, the drug selected from at least one member of the group consisting of an insulin sensitivity enhancer, an amylase inhibitor, an $\alpha$-glucosidase inhibitors, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, a glycogen synthase kinase-3 inhibitor, an 11$\beta$-hydroxy-steroiddehydrogenase inhibitor, glucagon-like peptide-1, a glucagon-like peptide-1 analogue, a glucagon-like peptide-1 agonist, amylin, an amylin analogue, an amylin agonist and an appetite suppressant is preferable; the drug selected from at least one member of the group consisting of an insulin

sensitivity enhancer, an amylase inhibitor, an α-glucosidase inhibitors, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, a glycogen synthase kinase-3 inhibitor, an 11β-hydroxysteroid-dehydrogenase inhibitor, glucagon-like peptide-1, a glucagon-like peptide-1 analogue, a glucagon-like peptide-1 agonist, amylin, an amylin analogue and an amylin agonist is more preferable; and the drug selected from at least one member of the group consisting of an insulin sensitivity enhancer, an amylase inhibitor, an α-glucosidase inhibitors, a biguanide, an insulin secretion enhancer and an insulin or insulin analogue is most preferable.

Similarly, in the use for the prevention or treatment of diabetic complications, for example, the drug selected from at least one member of the group consisting of an insulin sensitivity enhancer, an amylase inhibitor, an α-glucosidase inhibitor, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, glycogen synthase kinase-3 inhibitors, an 11β-hydroxysteroiddehydrogenase inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet derived growth factor analogue, epidermal growth factor, nerve growth factor, a carnitine derivative, uridine, 5-hydroxy-1-methylhidantoin, EGB-761, bimoclomol, sulodexide, Y-128, an antidiarrhoics, a cathartics, an angiotensin-converting enzyme inhibitor, a neutral endopeptidase inhibitor, an angiotensin II receptor antagonist, an endothelin-converting enzyme inhibitor, an endothelin receptor antagonist and a diuretic agent is preferable; and the drug selected from at least one member of the group consisting of an aldose reductase inhibitor, an angiotensin-converting enzyme inhibitor, a neutral endopeptidase inhibitor and an angiotensin II receptor antagonist is more preferable.

Furthermore, in the use for the prevention or treatment of obesity, the drug selected from at least one member of the group consisting of an insulin sensitivity enhancer, an amylase inhibitor, an α-glucosidase inhibitor, a glucose absorption inhibitor, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, a glycogen synthase kinase-3 inhibitor, an 11β-hydroxysteroid-dehydrogenase inhibitor, glucagon-like peptide-1, a glucagon-like peptide-1 analogue, a glucagon-like peptide-1 agonist, amylin, an amylin analogue, an amylin agonist, a $\beta_3$-adrenoceptor agonist and an appetite suppressant is preferable; and, the drug selected from at least one member of the group consisting of an amylase inhibitor, an α-glucosidase inhibitor, a $\beta_3$-adrenoceptor agonist and an appetite suppressant is more preferable..

## Examples

The present invention is further illustrated inmore detail by way of the following Preparation Examples, Examples and Test Examples. However, the present invention is not limited thereto.

Preparation Example 1

1-(2,3,4,6-Tetra-*O*-benzyl-β-D-glucopyranosyl)-3-formyl-1H-indole

[Chem. 9]

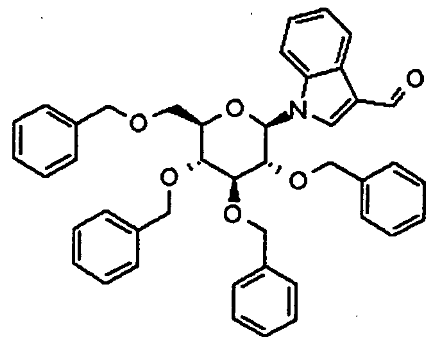

To a solution of 3-formyl-1H-indole (1 g) in *N,N*-dimethylformamide (20 mL) was added 55% sodium hydride (0.2 g) under ice-cooling, and the resulting mixture was stirred for 20 minutes. To the reaction mixture was added 2,3,4,6,-tetra-*O*-benzyl-α-D-glucopyranosylchloride (Cicchillo,R.M.;Norris,P,Carbohydrate Research, 328(2000) 431-434)(4.24 g)at the same temperature, and the mixture was stirred at room temperature overnight. The reaction mixture was poured into water, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: n-hexane/ethyl acetate = 5/1-3/1) to give the title compound (1.21 g).
$^{1}$H-NMR ($CDCl_3$) δ ppm:
3.65 (1H, d, J=10.5Hz), 3.65-3.8 (2H, m), 3.8-4.0 (4H, m), 4.28 (1H, d, J=10.5Hz), 4.52 (1H, d, J=12.0Hz), 4.58 (1H, d, J=12.5Hz), 4.67 (1H, d, J=10.7Hz), 4.90 (1H, d, J=10.6Hz), 6.6-6.7 (2H, m), 7.0-7.1 (2H, m), 7.1-7.2 (1H, m), 7.2-7.25 (2H, m), 7.25-7.40 (15H,m), 7.59(1H, d, J=8.3Hz), 7.77(1H, s), 8.33(1H, d, J=7.3Hz), 9.98 (1H, s).

Preparation Examples 2 to 4

1-(2,3,4,6-Tetra-*O*-benzyl-β-D-glucopyranosyl)-3-formyl-4-methyl-1H-indole, 1-(2,3,4,6-tetra-*O*-benzyl-β-D-glucopyranosyl)-3-formyl-6-methyl-1H-indole, and 1-(2,3,4,6-tetra-*O*-benzyl-β-D-glucopyranosyl)-3-formyl-7-methyl-1H-indole were prepared in a similar manner to that described in Preparation Example 1 except for using 3-formyl-4-methyl-1H-indole, 3-formyl-6-methyl-1H-indole, and 3-formyl-7-methyl-1H-indole instead of 3-formyl-1H-indole.

Preparation Example 5

1-(2,3,4,6-Tetra-*O*-benzyl-β-D-galactopyranosyl)-3-formy1-4-methyl-1H-indole

The title compound was prepared in a similar manner to that described in Preparation Example 1 using 3-formyl-4-methyl-1H-indoleinstead of 3-formyl-1H-indole, and using 2,3,4,6-tetra-*O*-benzyl-α-D-galactopyranosylchloride instead of 2,3,4,6-tetra-*O*-benzyl-α-D-glucopyranosyl-chloride.

Preparation Example 6

1-(2,3,4,6-Tetra-*O*-benzyl-β-D-glucopyranosyl)-3-(4-bromobenzyl)-4-methyl-1H-indole

To a solution of p-dibromobenzene (0.799 g) in tetrahydrofuran (12 mL) was added a hexane solution of n-butyllitium (2.71 mol/L, 1.05 mL) at -78°C, and the resulting mixture was stirred for 30 minutes at the same temperature. To the reaction solution was added 1- (2, 3, 4, 6-tetra-*O*-benzyl-β-D-glucopyranosyl)-3-formyl-4-methyl-1H-indole (0.77 g) in tetrahydrofuran (6 mL) at the same temperature, and the mixture was stirred for 1 hour under ice-cooling. The reaction solution was poured into a saturated aqueous ammonium hydrochloride solution, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: n-hexane/ethyl acetate = 5/1-2/1) to give the corresponding adduct compound, 1-(2,3,4,6-tetra-*O*-benzyl-β-D-glucopyranosyl)-3-[hydroxy-(4-bromophenyl)methyl]-4-methyl-1H-indole (0.813 g). To a

solution of this adduct compound(0.813 g) in dichloromethane (10 mL) were added dropwise triethylsilane (0.561 g)and boron trifluoride diethyl ether complex (0.343 g) successively, and the resulting mixture was stirred for 15 minutes at room temperature. The reaction solution was poured into a saturated aqueous potassium carbonate solution, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: *n*-hexane/ethyl acetate = 4/1) to give the title compound (0.738 g).
$^1$H-NMR ($CDCl_3$) δ ppm:
2.53 (3H, s), 3.52 (1H, d, J=9.9Hz), 3.6-4.0 (6H, m), 4.14 (1H, d, J=9.8Hz), 4.20 (1H, d, J=16.5Hz), 4.24 (1H, d, J=16.5Hz), 4.51 (1H, d, J=12.5Hz), 4.59 (1H, d, J=12.5Hz), 4.66(1H, d, J=10.7Hz), 4.8-4.95 (3H, m), 5.28 (1H, d, J=8.7Hz), 6.7-6.8 (2H, m), 6.8-6.9 (2H, m), 7.0-7.1 (3H,m), 7.15-7.25 (5H, m), 7.25-7.35 (15H, m), 7.41 (1H, d, J=8.3Hz).

Preparation Example 7

1-(2,3,4,6-Tetra-*O*-benzyl-β-D-glucopyranosyl)-3-({4-[(1E)-3-carboxyprop-1-enyl]phenyl}methyl)-4-methyl-1H-indole

A suspension of 1-(2,3,4,6-tetra-*O*-benzyl-β-D-glucopyranosyl)-3-(4-bromobenzyl)-4-methyl-1H-indole (0.3 g), 3-butenoic acid (62 mg), palladium (II) acetate (8 mg), tris(2-methylphenyl)phosphine (22 mg) and triethylamine (181 mg) in acetonitrile (5 mL) was heated under reflux for 6 hours. The reaction mixture was diluted with ethyl acetate and the insoluble material was removed by filtration on celite. The filtrate was poured into 1M hydrochloric acid, and the resulting mixture was extracted with ethyl acetate. The organic layer was washed with water and brine successively, and dried over anhydrous magnesium sulfate. The solvent was removed under reduced pressure, and the residue was purified by column chromatography on silica gel (eluent: *n*-hexane/ethyl acetate = 3/1-2/1) to give the title compound (0.237 g).
$^1$H-NMR ($CDCl_3$) δ ppm:
2.55 (3H, s), 3.28 (2H, m), 3.54 (1H, d, J=9.8Hz), 3.6-4.0 (6H, m), 4.12 (1H, d, J=10.0Hz), 4.24 (1H, d, J=16.3Hz), 4.28 (1H, d, J=16.3Hz), 4.51 (1H, d, J=12.0Hz), 4.59 (1H, d, J=11.8Hz), 4.65 (1H, d, J=11.0Hz), 4.8-4.95 (3H, m), 5.27 (1H, d, J=9.0Hz), 6.15-6.30 (1H, m), 6. 47 (1H, d, J=15.5Hz), 6.7-6.8 (2H, m), 6.8-6.9 (2H, m), 7.0-7.25 (10H, m), 7.25-7.35 (13H,m), 7.41 (1H, d, J=8.5Hz).

Preparation Example 8

(*S*)-2-(2-Amino-2-methyl-propionylamino)-3-hydroxypropionic acid ethyl ester

[Chem.10]

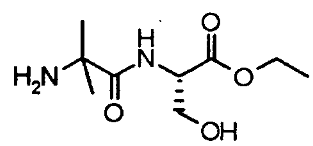

To a solution of 2-benzyloxycarbonylamino-2-methyl-propionic acid (500 mg), L-serine ethyl ester hydrochloride (715 mg), 1-hydroxybenzotriazole (855 mg) and triethylamine (1.07 g) in *N,N*-dimethylformamide (3 mL) was added 1-ethyl-3- (3-dimethylaminopropyl) carbodiimide hydrochloride (1.21 g), and the resulting mixture was stirred at room temperature for 3 days. The reaction liquid was poured into water, and the resulting mixture was extracted with ethyl acetate. The organic layer was washed with a saturated aqueous sodium hydrogen carbonate solution, water and brine successively, and dried over anhydrous magnesium sulfate. The solvent was removed under reduced pressure, and the residue was purified by column chromatography on silica gel (eluent: dichloromethane /methanol = 80/1) to give (S)-2-(2-benzyloxycarbonylamino-2-methyl-propionylamino)-3-hydroxy-propionic acid ethyl ester (0.49 g). To a solution of (S)-2-(2-benzyloxycarbonylamino-2-methyl-propionylamino)-3-hydroxy-propionic acid ethyl ester (0.49 g) in methanol (5 mL) was added 10% palladium-carbon powder (100 mg), and the mixture was stirred under a hydrogen atmosphere for 2 hours. The insoluble material was removed by filtration, and the solvent of the filtrate was removed under reduced pressure to give the title compound (0.28 g).
$^1$H-NMR ($CDCl_3$) δ ppm:
1.30 (3H, t, J=7.1 Hz), 1.39 (6H, d, J=5.0 Hz), 3.9-4.0 (2H, m), 4.25 (2H, q, J=7.1 Hz), 4.5-4.6 (1H, m), 8.0-8.05 (1H, brs).

Preparation Example 9

3-(4-Methoxybenzoyl)-7-azaindole

[Chem.11]

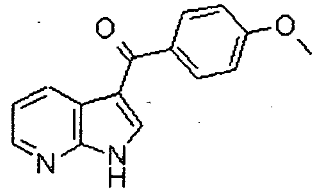

To a solution of 7-azaindole (2 g) in dichloromethane (150 mL) was added aluminum chloride (9.03 g) at room temperature, and the resultingmixture was stirred for 1 hour. To the reaction mixture was added p-anisoyl chloride (8.66 g) at the same temperature, and the resulting mixture was stirred overnight. To the reaction mixture was added methanol (50 mL), and the resultingmixturewas stirred for 1 hour. The solvent was removed under reduced pressure. To the residue was added ethyl acetate (100 mL), and the resulting mixture was stirred at room temperature. Precipitated crystals were collected by filtration, and the collected crystals were dissolved with ethyl acetate. The organic layer was washed with water and brine successively, and dried over anhydrous magnesium sulfate. The solvent was removed under reduced pressure to give the title compound (1.28 g).
$^1$H-NMR ($CDCl_3$) $\delta$ ppm:
3.9 (3H, s), 7.0-7.1 (2H, m), 7.31 (1H, dd, J=7.8 Hz, 4.7 Hz), 7.75-7.9 (2H, m), 7.99 (1H,m), 8.34 (1H, dd, J=4.7 Hz, 1.6 Hz), 8.6 (1H, dd, J=7.8 Hz, 1.6 Hz).

Preparation Example 10

1-(2,3,4,6-Tetra-*O*-acetyl-β-D-glucopyranosyl)-6-methyl-1H-indole

[Chem.12]

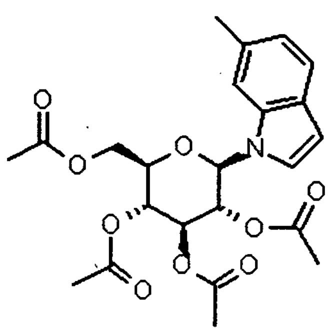

To a solution of 6-methyl-1H-indole (2 g) in acetic acid (50 mL) was added sodium cyanoborohydride (2.4 g) under ice-cooling, and the resulting mixture was stirred at room temperature for 1 hour. The reaction mixture was poured into a saturated aqueous sodium hydrogen carbonate solution with ice-cooling, and the resulting mixture was extracted twice with ethyl acetate. The organic layer was washed with brine, and dried over anhydrous sodium sulfate. The solvent was removed under reduced pressure, and the residue was purified by column chromatography on silica gel (eluent: n-hexane/ethyl acetate = 5/1-1/1) to give 6-methyl-2,3-dihydro-1H-indole (1.53 g).
A mixture of 6-methyl-2,3-dihydro-1H-indole (1.5 g), D-glucose (2.1 g) and water (0.2 mL) was stirred at 100˚C for 2 hour, and then the reaction mixtue was cooled to room temperature. To the residue was added acetic anhydride (9.2 g), N, N-dimethylaminopyridine (1.4 g) and pyridine (20 mL) , and the resulting mixture was stirred at room temperature for 2 hour. The reaction mixture was poured into ice-water. The resulting mixture was acidified with 1M hydrochloric

acid, and extracted with ethyl acetate. The organic layer was washed with 1M hydrochloric acid, water, a saturated aqueous sodium hydrogen carbonate solution and brine successively, and dried over anhydrous sodium sulfate. The solvent was removed under reduced pressure, and the residue was purified by column chromatography on silica gel (eluent: n-hexane/ethyl acetate = 3/1-1/3) to give 1-(2,3,4,6-tetra-*O*-acetyl-D-glucopyranosyl)-6-methyl-2,3-dihydro-1H-indole (3.55 g).

To a solution of 1-(2,3,4,6-tetra-*O*-acetyl-D-glucopyranosyl)-6-methyl-2,3-dihydro-1H-indole (2.9 g) in 1,4-dioxane (100 mL) was added, 2,3-dichloro-5,6-dicyano-1,4-benzoquinone (1.7 g) at room temperature, and the resulting mixture was stirred for 1 hour. The reaction mixture was poured into ice-water. To the resulting mixture was added a saturated aqueous sodium hydrogen carbonate solution, and extracted with ethyl acetate. The organic layer was washed with a saturated aqueous sodium hydrogen carbonate solution, water and brine successively, and dried over anhydrous sodium sulfate. The solvent was removed under reduced pressure, and the residue was purified by column chromatography on silica gel (eluent: n-hexane/ethyl acetate = 2/1-1/2) to give the title compound (2.42 g).

$^1$H-NMR ($CDCl_3$) δ ppm:

1.69 (3H, s), 2.04 (3H, s), 2.08 (3H, s), 2.09 (3H, s), 2.49 (3H, s), 3.95-4.05 (1H, m), 4.16 (1H, dd, J=12.4Hz, 2.4Hz), 4.3 (1H, dd, J=12.5Hz, 4.8Hz), 5.25-5.35 (1H, m), 5.4-5.5 (1H, m), 5.5-5.65 (2H, m) , 6.45-6.55(1H, m) , 6.95-7.05(1H, m) , 7.15 (1H, d, J=3.4Hz), 7.2 (1H, br s), 7.47 (1H, d, J=8.2Hz).

Preparation Example 11

<u>2,3,4,6-Tetra-*O*-benzyl-α-D-galactopyranosylchloride</u>

[Chem.13]

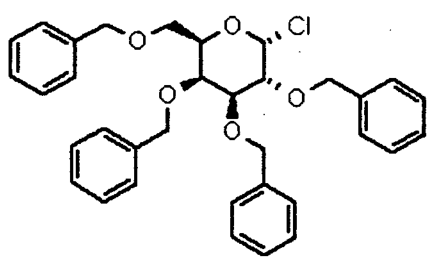

To a solution of 2,3,4,6-tetra-*O*-benzyl-D-galacto-pyranose (3 g) in N, N-dimethylformamide (12 mL) was added thionyl chloride (0.86 g) under ice-cooling, and the resulting mixture was stirred for 12 hours. To the reaction mixture was added additional thionyl chloride (0. 46 g) , and the resulting mixture was stirred overnight. The resulting mixture was basified with 1 M aqueous sodium hydroxide solution, and extracted with diethyl ether. The organic layer was washed with water twice and brine successively, and dried over anhydrous magnesium sulfate. The solvent was removed under reduced pressure, and the residue was purified by column chromatography on silica gel (eluent: n-hexane/ethyl acetate = 5/1) to give the title compound (1.83 g).

$^1$H-NMR ($CDCl_3$) δ ppm:

3.5-3.6 (2H, m) , 3.95-4.05 (2H, m) , 4.15-4.25 (2H, m) , 4.40 (1H, d, J=11.8 Hz), 4.48 (1H, d, J=11.6 Hz), 4.56 (1H, d, J=11.0 Hz), 4.7-4.8 (3H, m), 4.85 (1H, d, J=11.6 Hz), 4.94 (1H, d, J=11.0 Hz), 6.14 (1H, d, J=3.7 Hz), 7.2-7.4 (20H, m).

Preparation Examples 12 to 21

1-(2,3,4,6-Tetra-*O*-benzyl-β-D-glucopyranosyl)-4-chloro-3-formyl-1H-indole, 1-(2,3,4,6-tetra-*O*-benzyl-β-D-glucopyranosyl)-6-chloro-3-formyl-1H-indole, 1-(2,3,4,6-tetra-*O*-benzyl-β-D-glucopyranosyl)-7-chloro-3-formyl-1H-indole, 1-(2,3,4,6-tetra-*O*-benzyl-β-D-glucopyranosyl)-4-fluoro-3-formyl-1H-indole, 1-(2,3,4,6-tetra-*O*-benzyl-β-D-glucopyranosyl)-5-fluoro-3-formyl-1H-indole, 1-(2,3,4,6-tetra-*O*-benzyl-β-D-glucopyranosyl)-6-fluoro-3-formyl-1H-indole, 1-(2,3,4,6-tetra-*O*-benzyl-β-D-glucopyranosyl)-7-fluoro-3-formyl-1H-indole, 1-(2,3,4,6-tetra-*O*-benzyl-β-D-glucopyranosyl)-3-formyl-5-methoxy-1H-indole, 1-(2,3,4,6-tetra-*O*-benzyl-β-D-glucopyranosyl)-3-formyl-6-trifluoromethyl-1H-indole and 1-(2,3,4,6-tetra-*O*-benzyl-β-D-glucopyranosyl)-7-benzyloxy-3-formyl-1H-indole were prepared in a similar manner to that described in Preparation Example 1 using 4-chloro-3-formyl-1H-indole, 6-chloro-3-formyl-1H-indole, 7-chloro-3-formyl-1H-indole, 4-fluoro-3-formyl-1H-indole, 5-fluoro-3-formyl-1H-indole, 6-fluoro-3-formyl-1H-indole, 7-fluoro-3-formyl-1H-indole, 3-formyl-5-methoxy-1H-indole, 3-formyl-6-trifluoromethyl-1H-indole and' 7-benzyloxy-3-formyl-1H-indole instead of 3-formyl-1H-indole.

Preparation Example 22

1-(2,3,4,6-Tetra-*O*-acetyl-β-D-glucopyranosyl)-1H-indole

The title compound was prepared in a similar manner to that described in Preparation Example 10 using indoline instead of 6-methyl-2,3-dihydro-1H-indole.

Preparation Example 23

1-(2,3,4,6-Tetra-*O*-benzyl-β-D-glucopyranosyl)-3-({4-[(1E)-2-(methoxycarbonyl)vinyl]phenyl}methyl)-4-methyl-1H-indole

[Chem.14]

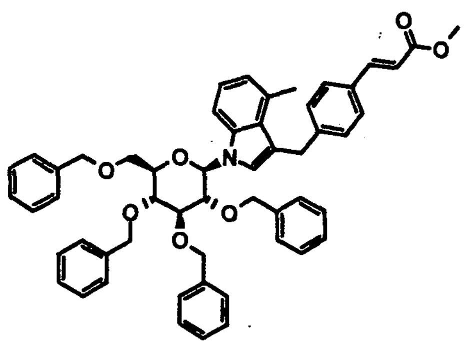

A suspension of 1-(2,3,4,6-tetra-*O*-benzyl-β-D-glucopyranosyl)-3-(4-bromobenzyl)-4-methyl-1H-indole (0.107 g), methyl acrylate (26 mg), palladium (II) acetate (3 mg), tris(2-methylphenyl)phosphine (8 mg) and triethylamine (66 mg) in acetonitrile (1.5 mL) was heated under reflux for 6 hours. The reaction mixture was diluted with ethyl acetate and the insoluble material was removed by filtration on celite. The filtrate was poured into 1M hydrochloric acid, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: *n*-hexane/ethyl acetate = 8/1-5/1) to give the title compound (82 mg).
$^1$H-NMR ($CDCl_3$) δ ppm:
2.54 (3H, s), 3.54 (1H, d, J=10.6 Hz), 3.60-4.05 (9H, m), 4.14 (1H, d, J=10.5 Hz), 4.25-4.40 (2H, m), 4.51 (1H, d, J=11.9 Hz), 4.59 (1H, d, J=11. 9 Hz), 4.66 (1H, d, J=10.7Hz), 4.80-4.95 (3H, m), 5.29 (1H, d, J=9.0 Hz), 6.36 (1H, d, J=16.1 Hz), 6.77 (2H, d, J=7.4 Hz), 6.80-6.90 (2H, m), 7.00-7.25 (8H, m), 7.25-7.35 (13H, m), 7.37 (2H, d, J=8.0 Hz), 7.42 (1H, d, J=8. 5 Hz), 7.64 (1H, d, J=16.1 Hz).

Preparation Example 24

1-(2,3,4,6-Tetra-*O*-benzyl-β-D-glucopyranosyl)-3-([4-{(1E)-2-carboxyvinyl}phenyl]methyl)-4-methyl-1H-indole

[Chem.15]

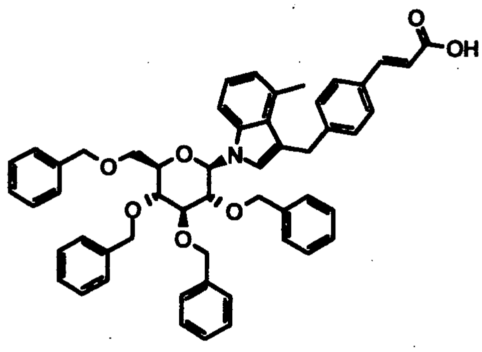

To a solution of 1-(2,3,4,6-tetra-*O*-benzyl-β-D-glucopyranosyl)-3-({4-[(1E)-2-(methoxycarbonyl)vinyl]phenyl}-methyl)-4-methyl-1H-indole (60 mg) in tetrahydrofuran (2 mL)/methanol (2 mL) was added a 5M aqueous sodium hydroxide solution (2 mL), and the resulting mixture was stirred at 50°C for 3 hours. The resulting mixture was acidified with 2M hydrochloric acid, and extracted with ethyl acetate. The organic layer was washed with water and brine successively, and dried over anhydrous magnesium sulfate. The solvent was removed under reduced pressure, and the residue was purified by column chromatography on silica gel (eluent: n-hexane/ethyl acetate = 5/1) to give the title compound (51 mg).
$^1$H-NMR ($CDCl_3$) δ ppm:
2.54 (3H, s), 3.53 (1H, d, J=10.2 Hz), 3.60-4.05 (6H, m), 4.15 (1H, d, J=11. 5 Hz) , 4.25-4.40 (2H, m) , 4.51 (1H, d, J=12.1 Hz) , 4.60 (1H, d, J=12.1 Hz), 4.65 (1H, d, J=10.7 Hz), 4.80-4.95 (3H, m), 5.29 (1H, d, J=8.7 Hz), 6.37 (1H, d, J=16.2 Hz), 6.77 (2H, d, J=7.1 Hz), 6.80-6.90 (2H, m), 7.00-7.25 (8H, m), 7.25-7.35 (13H, m), 7.37 (2H, d, J=8.2 Hz), 7.43 (1H, d, J=8.3 Hz), 7.72 (1H, d, J=16.2 Hz).

Preparation Example 25

1-(2,3,4,6-Tetra-*O*-benzyl-β-D-glucopyranosyl)-3-{(4-carboxyphenyl)methyl}-4-methyl-1H-indole

[Chem.16]

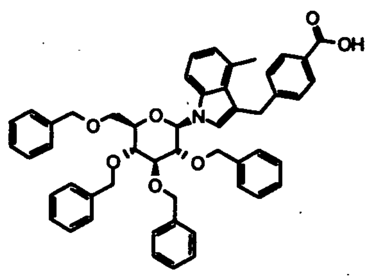

To a solution of 1-(2,3,4,6-tetra-*O*-benzyl-β-D-glucopyranosyl)-3-formyl-4-methyl-1H-indole (510 mg) and 4-bromobenzoic acid *t*-butyl ester (175 mL) in tetrahydrofuran (7.5 mL) was added *t*-butyllitium (1.44 M, *n*-pentane solution, 0.95 mL) at -78°C, and the resulting mixture was stirred for 15 minutes at the same temperature. To the reaction mixture was added water under ice-cooling, the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: n-hexane/ethyl acetate = 5/1-3/1) to give 1- (2, 3,4,6-tetra-*O*-benzyl-β-D-glucopyranosyl)-3-[hydroxy-(4-*t*-butoxycarbonyl)methyl]-4-methyl-1H-indole (310 mg).
To a solution of 1-(2,3,4,6-tetra-*O*-benzyl-β-D-glucopyranosyl)-3-[hydroxy-(4-*t*-butoxycarbonyl)methyl]-4-methyl-1H-indole (310 mg) in dichloromethane (4 mL) were added dropwise triethylsilane (0.21 g)and boron trifluoride diethyl ether complex (0.26 g) successively under ice-cooling in a methanol bath, and the resulting mixture was stirred for 30 minutes.

To the reaction mixture was added a saturated aqueous potassium carbonate solution, and the resulting mixture was stirred for 10 minutes. The mixture was poured into water, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: *n*-hexane/ethyl acetate = 7/1) to give 1-(2,3,4,6-tetra-*O*-benzyl-β-D-glucopyranosyl)-3-{(4-*t*-butoxycarbonylphenyl)methyl}-4-methyl-1H-indole (125 mg).
To a solution of 1-(2,3,4,6-tetra-*O*-benzyl-β-D-glucopyranosyl)-3-{(4-*t*-butoxycarbonylphenyl)methyl}-4-methyl-1H-indole (124 mg) in dichloromethane (2 mL) was added trifluoroacetic acid (1 mL) at room temperature, and the resultingmixture was stirred at the same temperature for 2 hours. The solvent of the reaction mixture was removed under reduced pressure, and the residue was purified by column chromatography on silica gel (eluent: n-hexane/ethyl acetate = 2/1) to give the title compound (91 mg).
$^1$H-NMR ($CDCl_3$) δ ppm:
2.52 (3H, s), 3.53 (1H, d, J=10.6 Hz), 3.60-4.05 (6H, m), 4.15 (1H, d, J=10.2 Hz), 4.33 (1H, d, J=16.8 Hz), 4.37 (1H, d, J=16.8 Hz), 4.51 (1H, d, J=12.4 Hz), 4.60 (1H, d, J=12.4 Hz), 4.65 (1H, d, J=10.8 Hz), 4.80-4.95 (3H, m), 5.30 (1H, d, J=8.7 Hz), 6.76 (2H, d, J=6.8Hz), 6.80-6.90 (2H, m) , 7.00-7.25 (6H,m), 7.25-7.40 (15H, m), 7.43 (1H, d, J=8.4 Hz), 7.94 (2H, d, J=8.3 Hz).

Preparation Example 26

4-Chloro-3-{2-(4-methoxyphenyl)-prop-2-yl}-1H-indole

[Chem.17]

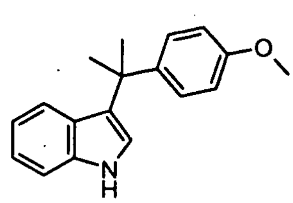

To a solution of 4-methoxyacetophenone (1.5 g) in tetrahydrofuran (30 mL) was added methyllitium (0.98 M, diethyl ether solution, 11.2 mL) under a argon atmosphere under dry ice-cooling in an acetone bath, and the resulting mixture was stirred for 2 hours at room temperature. To the reaction mixture was added an aqueous ammonium chloride solution to quench the reaction, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: *n*-hexane/ethyl acetate = 10/1-3/1) to give 2-(4-methoxyphenyl)-propan-2-ol (1.56 g).
To a solution of 4-chloro-indole (0.3 g) and 2-(4-methoxyphenyl)-propan-2-ol (0.33 g) in dichloromethane (15 mL) was added trifluoroacetic acid (0.23 mL) at room temperature, and the resulting mixture was stirred at room temperature for 1 hour. To the reaction mixture was added a saturated aqueous sodium hydrogen carbonate solution, and the resulting mixture was stirred for 10 minutes. The mixture was extracted with dichloromethane. The organic layer was washed with water and brine successively, and dried over anhydrous sodiumsulfate. The solvent was removed under reduced pressure, and the residue was purified by column chromatography on silica gel (eluent: *n*-hexane/ethyl acetate=10/1-2/1) to give the title compound (0.19 g).
$^1$H-NMR ($CDCl_3$) δ ppm:
1.21 (3H, t, J=7.6), 2.6 (2H, q, J=7.5Hz), 6.9-7 (2H, m), 7-7.15 (4H, m), 7.15-7.25 (1H, m), 7.3-7.45 (2H, m), 7.7-7.8 (1H, br s).

Preparation Example 27

3-(4-Ethylphenyl)oxy-1H-indole

[Chem.18]

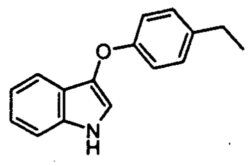

To a solution of 3-acetoxyindole (2.5 g) in *N*,*N*-dimethylformamide (20mL) was added 55% sodium hydride (0.38 g) under ice-cooling, and the resulting mixture was stirred for 20 minutes. To the reaction mixture was added 4-fluoroacetophenone (2.96 g), and the mixture was stirred at 120°C for 1 hour. The reaction mixture was poured into water, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: *n*-hexane/ethyl acetate = 8/1-1/1) to give 3-(4-acetylphenyl)oxy-1H-indole (0.82 g).
To a solution of 3-(4-acetylphenyl)oxy-1H-indole (0.25 g) in diethyleneglycol (4.5 mL) ware added hydrazine mono hydrate (1. 18 mL) and potassium hydroxide (0.68 g) at room temperature, and the resulting mixture was stirred at 210°C for 3 hours. After cooled to room temperature, the reaction mixture was poured into water, and the resulting mixture was extracted with dichloromethane. The organic layer was washed with water and brine successively, and dried over anhydrous sodium sulfate. The solvent was removed under reduced pressure, and the residue was purified by column chromatography on aminopropylated silica gel (eluent: n-hexane/ethyl acetate = 8/1-1/1) to give the title compound (52 mg).
$^1$H-NMR ($CDCl_3$) $\delta$ ppm:
1.8 (6H,s), 6.7-6.8 (2H, m) , 6.9-7 (1H,m), 7-7.1(1H,m), 7.1-7.2 (2H, m), 7.2-7.3 (2H, m), 8.1-8.2 (1H, br s).

Example 1

<u>3-Benzyl-1-(β-D-glucopyranosyl)-1H-indole</u>

[Chem.19]

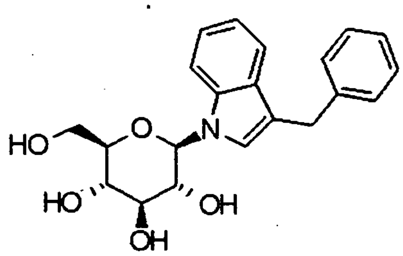

To 2,6-lutidine(1mL) were added 3-benzyl-1H-indole (0.35 g), acetobromo-α-D-glucose (1.4 g)and silver oxide (0.79 g), and the resulting mixture was stirred for 3 hours at room temperature. To the reaction mixture was added water, and the resulting mixture was extracted with ethyl acetate. The organic layer was washed with brine, and dried over anhydrous magnesium sulfate. The solvent was removed under reduced pressure. To the residue dissolved in methanol (3 mL) was added sodium methoxide (28% methanol solution, 0.1 mL), and the resulting mixture was stirred for 1 hour at room temperature. To the reaction mixture was added brine, and the resulting mixture was extracted with ethyl acetate. The organic layer was dried over anhydrous magnesium sulfate. The solvent was removed under reduced pressure, and the residue was purified by column chromatography on silica gel (eluent: ethyl acetate) to give the title compound (52 mg).
$^1$H-NMR ($CD_3OD$) $\delta$ ppm:
3.4-3.5 (1H, m), 3.5-3.65 (2H, m), 3.68 (1H, dd, J=11. 9Hz, 5.7Hz), 3.8-3.95 (2H, m), 4.06 (2H, s), 5.42 (1H, d, J=9.3Hz), 6.95-7.05 (1H, m), 7.05-7.3 (7H, m), 7.39 (1H, d, J=7.9Hz), 7.49 (1H, d, J=8.6Hz).

Example 2

-1-(β-D-Glucopyranosyl)-3-(4-methoxybenzyl)1H-indole

To a solution of 4-bromoanisole (73 mg) in tetrahydrofuran (1 mL) were added magnesium (11 mg) and a catalytic amount of iodine, and the resulting mixture was heated with stirring to prepare Grignard reagent (4-methoxyphenylmagnesium bromide). To a solution of 1-(2,3,4,6-tetra-*O*-benzyl-β-D-glucopyranosyl)-3-formyl-1H-indole (0.2g) in tetrahydrofuran (2mL) was added the above Grignard reagent under ice-cooling, and the resulting mixture was stirred for 3 hours at room temperature. To the reaction mixture was added a saturated aqueous ammonium chloride solution to quench the reaction, and the resulting mixture was extracted with ethyl acetate. The organic layer was washed with 1M hydrochloric acid, water and brine successively, and dried over anhydrous sodium sulfate. The solvent was removed under reduced pressure, and the residue was purified by column chromatography on silica gel (eluent: *n*-hexane/ethyl acetate = 3/1) to give the corresponding adduct compound, 1-(2,3,4,6-tetra-*O*-benzyl-β-D-glucopyranosyl)-3-[hydroxy-(4-methoxyphenyl)methyl]-1H-indole (155 mg). To a solution of the obtained adduct compound in tetrahydrofuran/methanol (= 1/1, 3 mL) was added 10% palladium-carbon powder (0.1 g) , and the mixture was stirred under a hydrogen atmosphere overnight. The insoluble material was removed by filtration, and the solvent of the filtrate was removed under reducedpressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (25 mg).
$^1$H-NMR ($CD_3OD$) δ ppm:
3.4-3.5 (1H, m), 3.5-3.65 (2H, m), 3.68 (1H, dd, J=12.5Hz, 5.7Hz) , 3.74 (3H, s) , 3.8-3.95 (2H, m) , 4.0 (2H, s) , 5.41 (1H, d, J=9.1Hz), 6.75-6.85.(2H, m), 6.95-7.05 (1H, m), 7.1-7.25 (4H, m), 7.38 (1H, d, J=7.9Hz), 7.48 (1H, d, J=8.2Hz).

Examples 3 to 74

The compounds described in Table 1 were prepared in a similar manner to that described in Example 2 using the corresponding bromobenzene compounds instead of 4-bromoanisole. In the preparation of example compounds, a benzyl group was used as a protecting group of hydroxy group except for the hydroxy group in a glucose moiety.

Examples 75 to 76

The compounds described in Table 1 were prepared in a similar manner to that described in Example 2 except for using the corresponding bromobenzenes instead of 4-bromoanisole, and using 1-(2,3,4,6-tetra-*O*-benzyl-β-D- galactopyranosyl)-3-formyl-1H-indole instead of 1-(2,3,4,6-tetra-*O*-benzyl-β-D-glucopyranosyl)-3-formyl-1H-indole.
[Table 1]

Table 1-1

| Example No. | Chemical structure | $^1$H-NMR ($CD_3OD$) δ ppm: |
|---|---|---|
| Ex.3 | 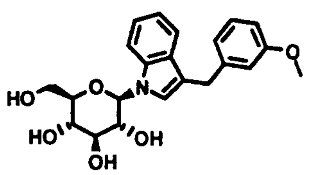 | 3.4-3.5 (1H, m), 3.5-3.6 (2H, m), 3.65-3.75 (4H, m), 3.8-3.95 (2H, m), 4.04 (2H, s), 5.42 (1H, d, J=9Hz), 6.65-6.75 (1H, m), 6.8-6.9 (2H, m), 6.95-7.05 (1H, m), 7.1-7.2 (3H, m), 7.4 (1H, d, J=7.9Hz), 7.49 (1H, d, J=8.6Hz) |
| Ex.4 | 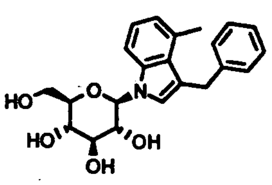 | 2.41 (3H, s), 3.4-3.5 (1H, m), 3.5-3.6 (2H, m), 3.69 (1H, dd, J=12.3Hz, 5.7Hz), 3.8-3.95 (2H, m), 4.25 (2H, s), 5.42 (1H, d, J=9.2Hz), 6.65-6.75 (1H, m), 6.95-7.05 (1H, m), 7.08 (1H, s), 7.1-7.25 (5H, m), 7.33 (1H, d, J=8.1 Hz) |
| Ex.5 | 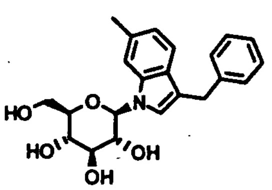 | 2.42 (3H, s), 3.4-3.5 (1H, m), 3.5-3.6 (2H, m), 3.68 (1H, dd, J=12.2Hz, 5.9Hz), 3.8-3.9 (2H, m), 4.03 (2H, s), 5.38 (1H, d, J=8.9Hz), 6.8-6.9 (1H, m), 7.08 (1H, s), 7.1-7.15 (1H, m), 7.15-7.35 (6H, m) |

(continued)

| Example No. | Chemical structure | $^1$H-NMR ($CD_3OD$) δ ppm: |
|---|---|---|
| Ex.6 | 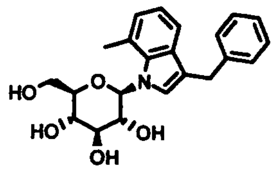 | 2.71 (3H, s), 3.35-3.4 (1H, m), 3.45-3.6 (2H, m), 3.65 (1H, dd, J=12Hz. 6.1 Hz), 3.8-3.95 (2H, m), 4.04 (2H, s), 5.79 (1H, d, J=8.9Hz), 6.8-6.95 (2H, m), 7.05-7.15 (1H, m), 7.15-7.3 (6H, m) |
| Ex.7 | 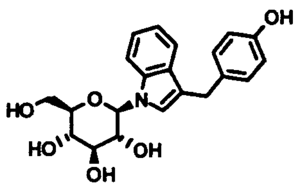 | 3.4-3.5 (1H, m), 3.5-3.65 (2H, m), 3.68 (1H, dd, J=11.7Hz, 5.6Hz), 3.8-3.95 (2H, m), 3.96 (2H, s), 5.41 (1H, d, J=8.7Hz), 6.6-6.75 (2H, m), 6.95-7.05 (1H, m), 7.05-7.2 (4H, m), 7.39 (1H, d, J=7.5Hz), 7.48 (1H, d, J=8.3Hz) |
| Ex.8 | 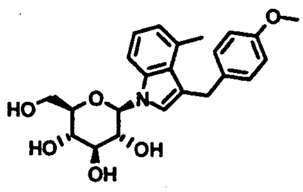 | 2.43 (3H, s), 3.4-3.5 (1H, m), 3.5-3.6 (2H, m), 3.6-3.8 (4H, m), 3.8-3.95 (2H, m), 4.18 (2H, s), 5.4 (1H, d, J=9.3Hz), 6.65-6.75 (1H, m), 6.75-85 (2H, m), 6.95-7.15 (4H, m), 7.32 (1H, d, J=8Hz) |

Table 1-2

| Example No. | Chemical structure | $^1$H-NMR ($CD_3OD$) δ ppm: |
|---|---|---|
| Ex.9 | 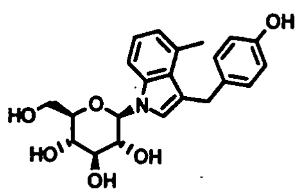 | 2.44 (3H, s), 3.4-3.5 (1H, m), 3.5-3.6 (2H, m), 3.69 (1H, dd, J=12.5Hz, 5.7Hz), 3.8-3.95 (2H, m), 4.15 (2H, s), 5.39 (1H, d, J=9.2Hz), 6.6-6.7 (2H, m), 6.73 (1H, d, J=7.1 Hz), 6.9-7.05 (4H, m), 7.32 (1 H, d, J=8.1 Hz) |
| Ex.10 | 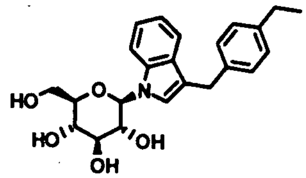 | 1.19 (3H, t, J=7.5Hz), 2.58 (2H, q, J=7.6Hz), 3.4-3.5 (1H, m), 3.5-3.65 (2H, m), 3.68 (1H, dd, J=12.0Hz, 5.8Hz), 3.8-3.95 (2H, m), 4.02 (2H, s), 5.41 (1H, d, J=9.2Hz), 6.9-7.25 (7H, m), 7.39 (1H, d, J=7.9Hz), 7.48 (1H, d, J=8.4Hz) |
| Ex.11 | 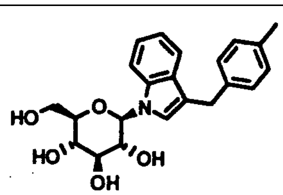 | 2.27 (3H, s), 3.4-3.65 (3H, m), 3.68 (1H, dd, J=12.3Hz, 5.8Hz), 3.8-3.95 (2H, m), 4.01 (2H, s), 5.41 (1H, d, J=9.0Hz), 6.9-7.1 (3H, m), 7.1-7.2 (4H, m), 7.38 (1H, d, J=8.0Hz), 7.48 (1H, d, J=8.2Hz) |
| Ex.12 | 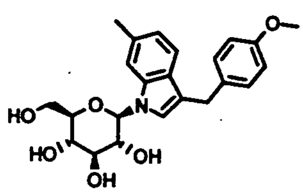 | 2.42 (3H, s), 3.4-3.5 (1H, m), 3.5-3.6 (2H, m), 3.68 (1H, dd, J=12.0Hz, 5.8Hz), 3.73 (3H, s), 3.8-3.9 (2H, m), 3.96 (2H, s), 5.38 (1H, d, J=9.1 Hz), 6.75-6.9 (3H, m), 7.05 (1H, s), 7.15-7.2 (2H, m). 7.2-7.3 (2H, m) |

(continued)

| Example No. | Chemical structure | $^1$H-NMR ($CD_3OD$) δ ppm: |
| --- | --- | --- |
| Ex.13 | 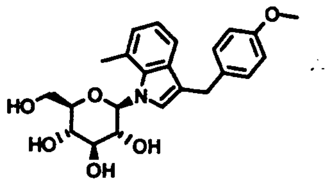 | 2.71 (3H, s), 3.35-3.45 (1H, m), 3.45-3.6 (2H, m), 3.65 (1H, dd, J=12.5Hz, 6.0Hz), 3.73 (3H, s), 3.8-3.95 (2H, m), 3.97 (2H, s), 5.79 (1H, d, J=8.8Hz), 6.75-6.85 (2H, m), 6.85-6.95 (2H, m), 7.1-7.3 (4H, m) |
| Ex.14 | 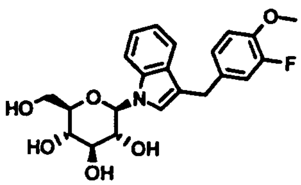 | 3.4-3.5 (1H, m), 3.5-3.65 (2H, m), 3.69 (1H, dd, J=12.3Hz. 5.9Hz), 3.75-3.95 (5H, m), 4.01 (2H, s), 5.43 (1H, d, J=9.2Hz), 6.9-7.05 (4H, m), 7.1-7.25 (2H, m), 7.36 (1H, d, J=7.9Hz), 7.49 (1H, d, J=8.5Hz) |
| Ex. 15 | 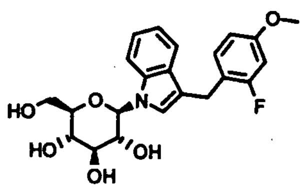 | 3.4-3.5 (1H, m), 3.5-3.65 (2H, m), 3.68 (1H, dd, J=12.0Hz. 5.6Hz), 3.74 (3H, s), 3.8-3.95 (2H, m), 4.0 (2H, s), 5.41 (1H, d, J=9.4Hz), 6.55-6.7 (2H, m), 6.95-7.05 (1H, m), 7.05-7.2 (3H, m), 7.43 (1H, d, J=7.6Hz), 7.49 (1 H, d, J=8.4Hz) |

Table 1-3

| Example No. | Chemical structure | $^1$H-NMR ($CD_30D$) δ ppm: |
| --- | --- | --- |
| Ex.16 | 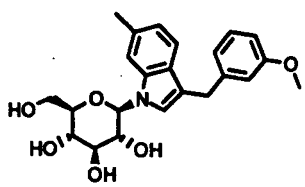 | 2.42 (3H, s), 3.4-3.5 (1H, m), 3.5-3.6 (2H, m), 3.68 (1H, dd, J=12.2Hz. 5.8Hz), 3.71 (3H, s), 3.8-3.95 (2H, m), 4.0 (2H, s), 5.38 (1H, d, J=8.9Hz), 6.65-6.75 (1H, m), 6.8-6.9 (3H, m), 7.09 (1H, s), 7.1-7.15 (1H, m), 7.2-7.3 (2H, m) |
| Ex.17 | 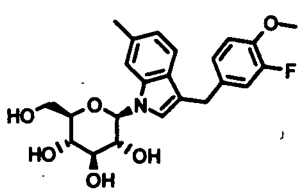 | 2.42 (3H, s), 3.4-3.5 (1H, m), 3.5-3.65 (2H, m), 3.69 (1H, dd, J=11.9Hz, 5.7Hz), 3.75-3.95 (5H, m), 3.97 (2H, s), 5.39 (1H, d, J=9.1 Hz), 6.8-6.9 (1 H, m), 6.9-7.05 (3H, m), 7.1 (1H, s), 7.23 (1H, d, J=7.9Hz), 7.29 (1H, s) |
| Ex.18 | 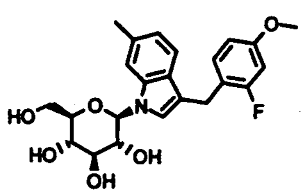 | 2.42 (3H, s), 3.4-3.5 (1H, m), 3.5-3.65 (2H, m), 3.68 (1H, dd, J=12.0Hz, 5.8Hz), 3.74 (3H, s), 3.8-3.9 (2H, m), 3.96 (2H, s), 5.38 (1H, d, J=9.3Hz), 6.59 (1H, dd, J=8.6Hz, 2.7Hz), 6.63 (1H, dd, J=11.9Hz, 2.4Hz), 6.8-6.9 (1H, m), 7.0-7.15 (2H, m), 7.25-7.35 (2H, m) |
| Ex.19 | 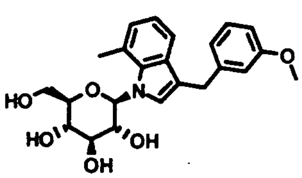 | 2.71 (3H, s), 3.35-3.45 (1H, m), 3.5-3.6 (2H, m), 3.65 (1H, dd, J=12.4Hz, 5.9Hz), 3.71 (3H, s), 3.8-3.95 (2H, m), 4.02 (2H, s), 5.79 (1H, d, J=9.0Hz), 6.65-6.95 (5H, m), 7.1-7.2 (1H, m), 7.2-7.3 (2H, m) |

(continued)

| Example No. | Chemical structure | $^1$H-NMR ($CD_30D$) δ ppm: |
|---|---|---|
| Ex.20 | 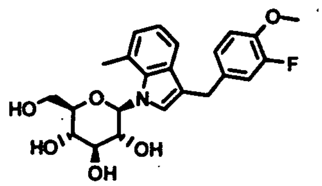 | 2.71 (3H, s), 3.35-3.5 (1H, m). 3.5-3.6 (2H, m), 3.66 (1H. dd, J=12.0Hz. 6.0Hz), 3.75-3.95 (5H, m), 3.98 (2H, s), 5.8 (1H, d, J=8.7Hz), 6.85-7.05 (5H, m), 7.15-7.3 (2H, m) |
| Ex.21 | 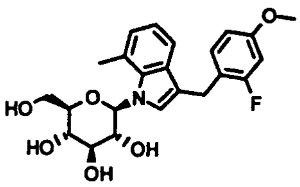 | 2.71 (3H, s), 3.35-3.45 (1H, m), 3.45-3.6 (2H, m), 3.65 (1H, dd, J=12.0Hz. 6.0Hz), 3.74 (3H, s), 3.8-3.95 (2H, m), 3.98 (2H, s), 5.79 (1H, d, J=8.8Hz), 6.55-6.7 (2H, m), 6.85-7.0 (2H, m), 7.0-7.15 (1H, m), 7.15-7.35 (2H, m) |

Table 1-4

| Example No. | Chemical structure | $^1$H-NMR ($CD_3OD$) δ ppm: |
|---|---|---|
| Ex.22 | 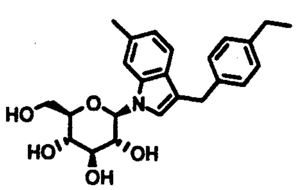 | 1.19 (3H, t, J=7.5Hz), 2.42 (3H, s), 2.58 (2H, q, J=7.5Hz), 3.4-3.5 (1 H, m), 3.5-3.6 (2H, m), 3.68 (1 H, dd, J=12.2Hz, 5.8Hz), 3.8-3.95 (2H, m), 3.99 (2H, s), 5.38 (1H, d, J=9.2Hz), 6.84 (1 H, d, J=8.0Hz), 7.0-7.1 (3H, m), 7.1-7.2 (2H, m), 7.2-7.35 (2H, m) |
| Ex.23 | 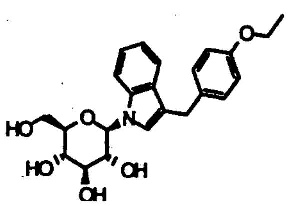 | 1.34 (3H, t, J=6.8Hz), 3.4-3.5 (1H, m), 3.5-3.65 (2H, m), 3.68 (1H, dd, J=12Hz, 5.8Hz), 3.8-4.05 (6H, m), 5.4 (1H, d, J=9.1Hz), 6.75-6.85 (2H, m), 6.95-7.05 (1H, m), 7.1-7.2 (4H, m), 7.38 (1H, d, J=7.9Hz), 7.48 (1H, d, J=8.6Hz). |
| Ex.24 | 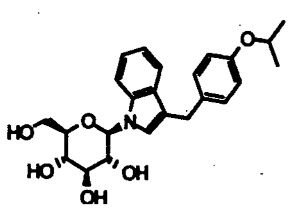 | 1.26 (6H, d, J=6.1 Hz), 3.4-3.5 (1 H, m), 3.5-3.65 (2H, m), 3.68 (1H, dd, J=12.4Hz, 5.7Hz), 3.8-3.95 (2H, m), 3.99 (2H, s), 4.45-4.55 (1H, m), 5.41 (1H, d, J=9.2Hz), 6.75-6.8 (2H, m), 6.95-7.05 (1 H, m), 7.1-7.2 (4H, m), 7.39 (1 H, d, J=8Hz), 7.48 (1H, d, J=8.4Hz) |
| Ex.25 | 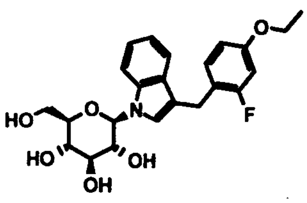 | 1.35 (3H, t, J=6.9Hz), 3.4-3.5 (1H, m), 3.5-3.6 (2H, m), 3.68 (1H, dd, J=12Hz, 5.8Hz), 3.8-4.05 (6H, m), 5.41 (1 H, d, J=8.8Hz), 6.55-6.6 (1 H, m), 6.6-6.7 (1 H, m), 6.95-7.05 (1H, m), 7.05-7.2 (3H, m), 7.43 (1H, d, J=7.8Hz), 7.49 (1H, d, J=8Hz) |
| Ex.26 | 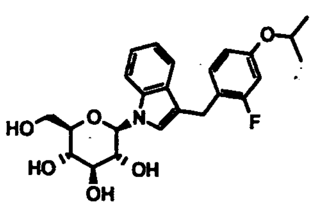 | 1.27 (6H, d, J=5.9Hz), 3.4-3.5 (1H, m), 3.5-3.6 (2H, m),3.68 (1H, dd, J=12.3Hz, 5.7Hz), 3.8-3.95 (2H, m), 3.99 (2H, s), 4.45-4.6 (1H, m), 5.41 (1 H, d, J=8.8Hz), 6.55-6.6 (1 H, m), 6.6-6.65 (1 H, m), 6.95-7.05 (1 H, m), 7.05-7.2 (3H, m), 7.44 (1 H, d, J=7.9Hz), 7.49 (1H, d, J=8.3Hz) |

(continued)

| Example No. | Chemical structure | $^1$H-NMR ($CD_3OD$) δ ppm: |
|---|---|---|
| Ex.27 | 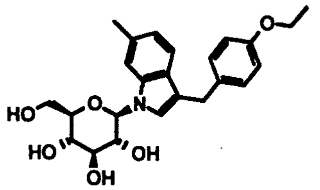 | 1.34 (3H, t, J=6.9Hz), 2.42 (3H, s), 3.4-3.5 (1H, m), 3.5-3.6 (2H, m), 3.68 (1 H, dd, J=12Hz, 5.8Hz), 3.8-3.9 (2H, m), 3.9-4 (4H, m), 5.38 (1 H, d, J=9.1Hz), 6.7-6.8 (2H, m), 6.8-6.9 (1H, m), 7.05 (1H, s), 7.1-7.2 (2H, m), 7.2-7.3 (2H, m) |
| Ex.28 | 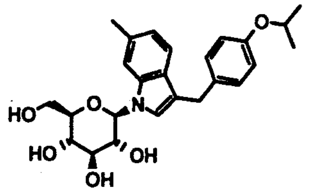 | 1.27 (6H, d, J=5.9Hz), 2.42 (3H, s), 3.4-3.5 (1H, m), 3.5-3.6 (2H, m), 3.68 (1H, dd, J=12.4Hz, 5.8Hz), 3.8-3.9 (2H, m), 3.96 (2H, s), 4.45-4.55 (1H, m), 5.38 (1H, d, J=9.3Hz), 6.7-6.8 (2H, m), 6.8-6.9 (1H, m), 7.06 (1H, s), 7.1-7.2 (2H, m); 7.2-7.3 (2H, m) |

Table 1-5

| Example No. | Chemical structure | $^1$H-NMR ($CD_3OD$) δ ppm: |
|---|---|---|
| Ex.29 | 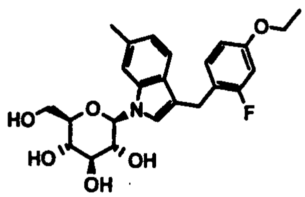 | 1.35 (3H, t, J=6.8Hz), 2.42 (3H, s), 3.4-3.5 (1 H, m), 3.5-3.6 (2H, m), 3.68 (1H, dd, J=12.3Hz, 5.7Hz), 3.8-3.9 (2H, m), 3.9-4.05 (4H, m), 5.38 (1 H, d, J=9.2Hz), 6.55-6.6 (1H, m), 6.6-6.65 (1H, m), 6.8-6.9 (1H, m), 7.05-7.15 (2H, m), 7.25-7.35 (2H, m) |
| Ex.30 | 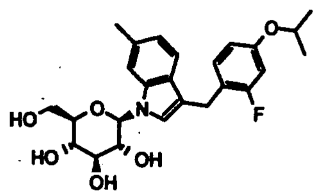 | 1.27 (6H, d, J=6.1Hz), 2.43 (3H, s), 3.4-3.5 (1H, m), 3.5-3.6 (2H, m), 3.68 (1 H, dd, J=12.4Hz, 5.7Hz), 3.8-3.9 (2H, m), 3.96 (2H, s), 4.45-4.55 (1 H, m), 5.38 (1 H, d, J=9.1Hz), 6.55-6.6 (1 H, m), 6.6-6.65 (1H, m), 6.85-6.9 (1H, m), 7.05-7.15 (2H, m), 7.25-7.35 (2H, m) |
| Ex.31 | 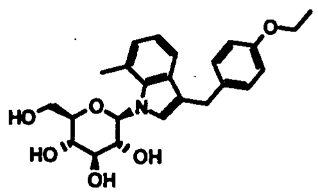 | 1.34 (3H, t, J=6.9Hz), 2.7 (3H, s), 3.35-3.45 (1H, m), 3.45-3.6 (2H, m), 3.65 (1H, dd, J=12.2Hz, 5.9Hz), 3.8-4 (6H, m), 5.79 (1H, d, J=8.7Hz), 6.7-6.8 (2H, m), 6.8-6.95 (2H, m), 7.1-7.3 (4H, m) |
| Ex.32 | 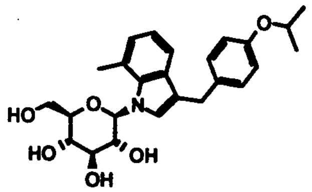 | 1.26 (6H, d, J=5.4Hz), 2.71 (3H, s), 3.35-3.75 (4H, m), 3.8-4.1 (4H, m), 4.45-4.55 (1 H, m), 5.79 (1H, d, J=7Hz), 6.7-7 (4H, m), 7.05-7.35 (4H, m) |

(continued)

| Example No. | Chemical structure | $^1$H-NMR ($CD_3OD$) δ ppm: |
|---|---|---|
| Ex.33 | 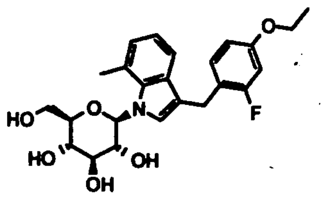 | 1.34 (3H, t, J=6.7Hz), 2.71 (3H, s), 3.35-3.45 (1H, m), 3.45-3.6 (2H, m), 3.65 (1H, dd, J=11.7Hz, 5.9Hz), 3.8-4.05(6H, m), 5.79 (1 H, d, J=8Hz), 6.5-6.6 (1 H, m), 6.6-6.7 (1 H, m), 6.85-6.95 (2H, m), 7-7.1 (1H, m), 7.15-7.35 (2H, m) |
| Ex.34 | 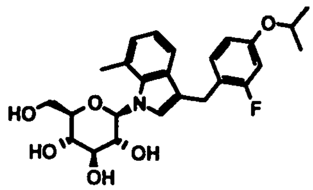 | 1.27 (6H, d, J=6Hz), 2.71 (3H, s), 3.35-3.45 (1H, m), 3.45-3.6 (2H, m), 3.65 (1H, dd, J=11.8Hz, 6.1Hz), 3.8-4 (4H, m), 4.45-4.55 (1 H, m), 5.79 (1H, d, J=8Hz), 6.5-6.65 (2H, m), 6.8-6.95 (2H, m), 7-7.1 (1H, m), 7.15-7.3 (2H, m) |
| Ex.35 | 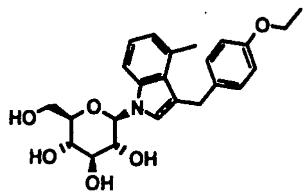 | 1.34 (3H, t, J=6.8Hz), 2.43 (3H, s), 3.4-3.5 (1H, m), 3.5-3.6 (2H, m), 3.69 (1H, dd, J=11.8Hz, 5.7Hz), 3.8-3.9 (2H, m), 3.97 (2H, q, J=6.9Hz), 4.18 (2H, s), 5.39 (1 H, d, J=9.1Hz), 6.7-6.75 (1H, m), 6.75-6.85 (2H, m), 6.95-7.1 (4H, m), 7.32 (1H, d, J=8Hz) |

Table 1-6

| Example No. | Chemical structure | $^1$H-NMR ($CD_3OD$) δ ppm: |
|---|---|---|
| Ex.36 | 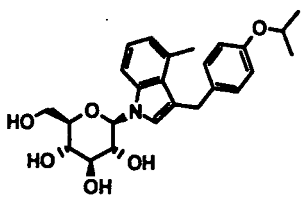 | 1.27 (6H, d, J=6.1 Hz), 2.43 (3H, s), 3.4-3.5 (1 H, m), 3.5-3.6 (2H, m), 3.69 (1H, dd, J=12.1Hz, 5.7Hz), 3.8-3.95 (2H, m), 4.18 (2H, s), 4.45-4.55 (1H, m), 5.4 (1H, d, J=9.1Hz), 6.7-6.75 (1H, m), 6.75-6.8 (2H, m), 6.95-7.1 (4H, m), 7.32 (1H, d, J=8.4Hz) |
| Ex.37 | 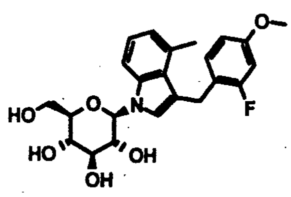 | 2.42 (3H, s), 3.4-3.5 (1 H, m), 3.5-3.6 (2H, m), 3.69 (1H, dd, J=12Hz, 5.8Hz), 3.75 (3H, s), 3.8-3.9 (2H, m), 4.15 (1 H, d, J=17Hz), 4.19 (1 H, d, J=17Hz), 5.4 (1H, d, J=9.1 Hz), 6.55-6.6 (1 H, m), 6.65-6.7 (1 H, m), 6.7-6.8 (1 H, m), 6.85-6.95 (1 H, m), 6.95-7.05 (1H, m), 7.06 (1 H, s), 7.34 (1H, d, J=8.5Hz) |
| Ex.38 | 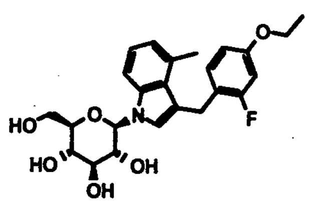 | 1.35 (3H, t, J=7Hz), 2.42 (3H, s), 3.4-3.5 (1H, m), 3.5-3.6 (2H, m), 3.69 (1 H, dd, J=12.1Hz, 5.7Hz), 3.8-3.9 (2H, m), 3.98 (2H, q, J=7Hz), 4.1-4.2 (2H, m), 5.4 (1H, d, J=9.1 Hz), 6.5-6.6 (1 H, m), 6.6-6.7 (1H, m), 6.7-6.8 (1 H, m), 6.8-6.95 (1H, m), 6.95-7.05 (1H, m), 7.06 (1H, s), 7.34 (1H, d, J=8.3Hz) |
| Ex.39 | 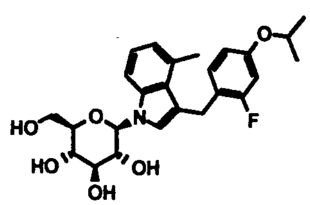 | 1.28 (6H, d, J=6.3Hz), 2.43 (3H, s), 3.4-3.5 (1H, m), 3.5-3.6 (2H, m), 3.69 (1 H, dd, J=11.9Hz, 5.7Hz), 3.8-3.9 (2H, m), 4.15 (1H, d, J=17.2Hz), 4.18 (1H, d, J=17.3Hz), 4.45-4.6 (1 H, m), 5.4 (1H, d, J=9.2Hz), 6.5-6.6 (1H, m), 6.6-6.7 (1H, m), 6.7-6.8 (1 H, m), 6.8-6.9 (1 H, m), 6.95-7.05 (1H, m), 7.07 (1H, s), 7.34 (1 H, d, J=8.4Hz) |

(continued)

| Example No. | Chemical structure | $^1H$-NMR ($CD_3OD$) δ ppm: |
|---|---|---|
| Ex.40 | 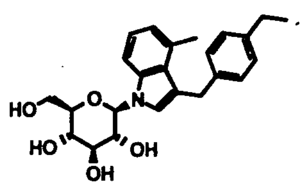 | 1.2 (3H, t, J=7.6Hz), 2.43 (3H, s), 2.59 (2H, q, J=7.6Hz), 3.4-3.5 (1 H, m), 3.5-3.6 (2H, m), 3.69 (1H, dd, J=12.3Hz, 5.9Hz), 3.8-3.95 (2H, m), 4.21 (2H, s), 5.4 (1H, d, J=9.2Hz), 6.7-6.75 (1H, m), 6.95-7.1 (6H, m), 7.33 (1H, d, J=8.6Hz) |
| Ex.41 | 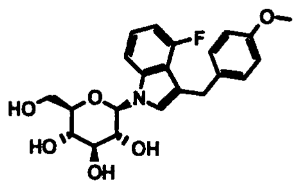 | 3.4-3.5 (1 H, m), 3.5-3.6 (2H, m), 3.68 (1H, dd, J=12.1 Hz, 5.7Hz), 3.74 (3H, s), 3.75-3.9 (2H, m), 4.08 (2H, s), 5.39 (1 H, d, J=9.1Hz), 6.6-6.75 (1 H, m), 6.75-6.85 (2H, m), 7-7.1 (2H, m), 7.1-7.2 (2H, m), 7.3 (1H, d, J=8.4Hz) |

Table 1-7

| Example No. | Chemical structure | $^1H$-NMR ($CD_3OD$) δ ppm: |
|---|---|---|
| Ex.42 | 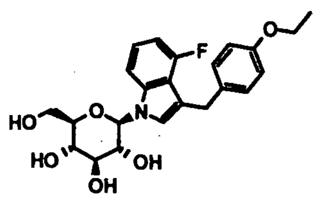 | 1.34 (3H, t, J=7.1Hz), 3.4-3.5 (1H, m), 3.5-3.6 (2H, m), 3.68 (1H, dd, J=12.4Hz, 5.7Hz), 3.75-3.9 (2H, m), 3.97 (2H, q, J=6.9Hz), 4.07 (2H, s), 5.39 (1 H, d, J=9.3Hz), 6.6-6.75 (1H, m), 6.75-6.85 (2H, m), 7-7.1 (2H, m), 7.1-7.2 (2H, m), 7.29 (1H, d, J=8.4Hz) |
| Ex.43 | 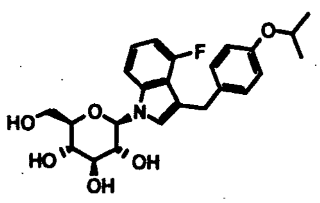 | 1.26 (6H, d, J=5.9Hz), 3.4-3.5 (1H, m), 3.5-3.6 (2H, m), 3.68 (1H, dd, J=12.1Hz, 5.9Hz), 3.75-3.9 (2H, m), 4.07 (2H, s), 4.45-4.55 (1H, m), 5.39 (1H, d, J=8.9Hz), 6.6-6.85 (3H, m), 7-7.1 (2H, m), 7.1-7.2 (2H, m), 7.3 (1H, d, J=8.2Hz) |
| Ex.44 | 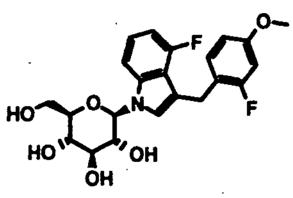 | 3.4-3.5 (1H, m), 3.5-3.6 (2H, m), 3.68 (1 H, dd, J=12Hz, 5.7Hz), 3.75 (3H, s), 3.75-3.9 (2H, m), 4.09 (2H, s), 5.39 (1H, d, J=8.8Hz), 6.55-6.75 (3H, m), 7-7.15 (3H, m), 7.25-7.35 (1H, m) |
| **Ex.45** | 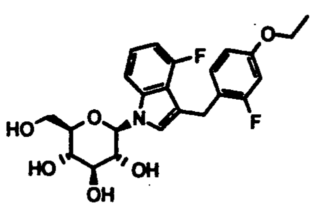 | 1.35 (3H, t, J=6.9Hz), 3.4-3.5 (1H, m), 3.5-3.6 (2H, m), 3.68 (1H, dd, J=12.1Hz, 5.9Hz), 3.75-3.9 (2H, m), 3.98 (2H, q, J=7Hz), 4.09 (2H, s), 5.39 (1H, d, J=9.1Hz), 6.55-6.75 (3H, m), 7-7.15 (3H, m), 7.31 (1 H, d, J=8.2Hz) |
| Ex.46 | 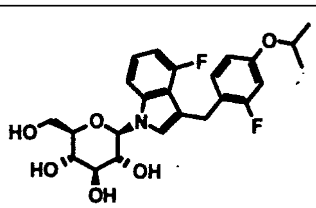 | 1.28 (6H, d, J=5.9Hz), 3.4-3.5 (1 H, m), 3.5-3.6 (2H, m), 3.68 (1H, dd, J=12.1Hz, 5.8Hz), 3.75-3.9 (2H, m), 4.09 (2H, s), 4.45-4.6 (1 H, m), 5.39 (1 H, d, J=9.1Hz), 6.5-6.75 (3H, m), 7-7.15 (3H, m), 7.31 (1 H, d, J=8.4Hz) |

(continued)

| Example No. | Chemical structure | $^1$H-NMR ($CD_3OD$) δ ppm: |
|---|---|---|
| Ex.47 | 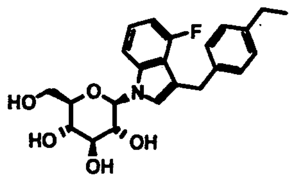 | 1.19 (3H, t, J=7.6Hz), 2.58 (2H, q, J=7.5Hz), 3.4-3.5 (1H, m), 3.5-3.6 (2H, m), 3.68 (1H, dd, J=12Hz, 5.7Hz), 3.75-3.9 (2H, m), 4.1 (2H, s), 5.39 (1H, d, J=9.1Hz), 6.6-6.75 (1H, m), 7-7.1 (4H, m), 7.1-7.2 (2H, m), 7.3 (1H, d, J=8.4Hz) |
| Ex.48 | 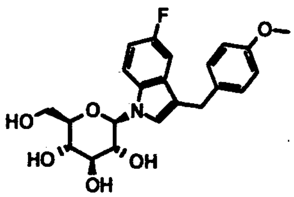 | 3.4-3.5 (1H, m), 3.5-3.6 (2H, m), 3.69 (1H, dd, J=12.2Hz, 5.6Hz), 3.74 (3H, s), 3.8-3.9 (2H, m), 3.96 (2H, s), 5.37 (1H, d, J=9.3Hz), 6.75-6.85 (2H, m), 6.85-6.95 (1H, m), 6.95-7.05 (1 H, m), 7.1-7.2 (2H, m), 7.21 (1H, s), 7.4-7.5 (1H, m) |

Table 1-8

| Example No. | Chemical structure | $^1$H-NMR ($CD_3OD$) δ ppm: |
|---|---|---|
| Ex.49 | 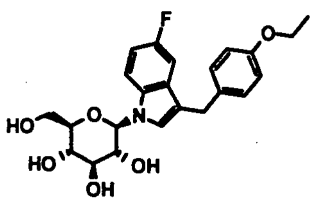 | 1.34 (3H, t, J=7Hz), 3.4-3.5 (1H, m), 3.5-3.6 (2H, m), 3.69 (1H, dd, J=12.3Hz, 5.7Hz), 3.8-3.9 (2H, m), 3.9-4.05 (4H, m), 5.37 (1 H, d, J=8.8Hz), 6.75-6.85 (2H, m), 6.85-6.95 (1H, m), 6.95-7.05 (1H, m), 7.1-7.2 (2H, m), 7.21 (1H, d, J=8.4Hz), 7.4-7.5 (1H, m) |
| Ex.50 | 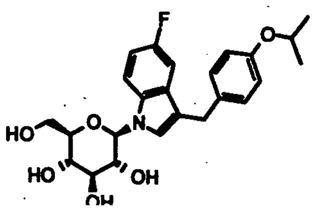 | 1.27 (6H, d, J=5.9Hz), 3.4-3.5 (1H, m), 3.5-3.6 (2H, m), 3.69 (1H, dd, J=12.5Hz, 5.7Hz), 3.8-3.9 (2H, m), 3.95 (2H, s), 4.45-4.6 (1H, m), 5.37 (1H, d, J=9Hz), 6.75-6.85 (2H, m), 6.85-6.95 (1H, m), 6.95-7.05 (1H, m), 7.1-7.2 (2H, m), 7.22 (1 H, d, J=8.2Hz), 7.4-7.5 (1 H, m) |
| Ex.51 | 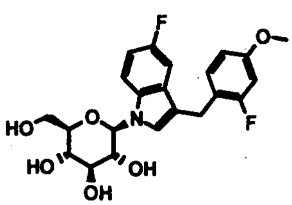 | 3.4-3.5 (1H, m), 3.5-3.6 (2H, m), 3.69 (1H, dd, J=12.3Hz, 5.7Hz), 3.75 (3H, s), 3.8-3.9 (2H, m), 3.96 (2H, s), 5.37 (1H, d, J=9.3Hz), 6.55-6.7 (2H, m), 6.85-6.95 (1H, m), 7.05-7.15 (2H, m), 7.23 (1H, s), 7.4-7.5 (1H, m) |
| Ex.52 | 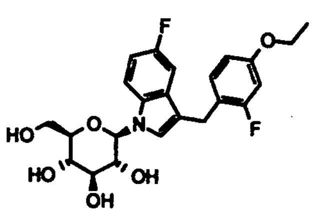 | 1.35 (3H, t, J=7Hz), 3.4-3.5 (1H, m), 3.5-3.6 (2H, m), 3.69 (1H, dd, J=11.9Hz, 5.7Hz), 3.8-3.9 (2H, m), 3.9-4.05 (4H, m), 5.37 (1H, d, J=8.9Hz), 6.55-6.7 (2H, m), 6.85-6.95 (1 H, m), 7.05-7.15 (2H, m), 7.23 (1 H, s), 7.4-7.5 (1 H, m) |
| Ex.53 | 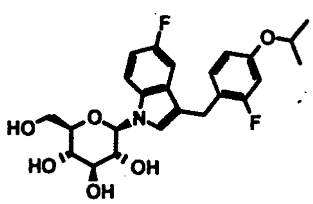 | 1.28 (6H, d, J=6.1 Hz), 3.4-3.5 (1 H, m), 3.5-3.6 (2H, m), 3.69 (1H, dd, J=11.9Hz, 5.8Hz), 3.8-3.9 (2H, m), 3.95 (2H, s), 4.45-4.6 (1 H, m), 5.59 (1 H, d, J=9.3Hz), 6.55-6.7 (2H, m), 6.85-6.95 (1H, m), 7.05-7.15 (2H, m), 7.23 (1H, s), 7.4-7.5 (1 H, m) |

(continued)

| Example No. | Chemical structure | $^1$H-NMR ($CD_3OD$) δ ppm: |
|---|---|---|
| Ex.54 | 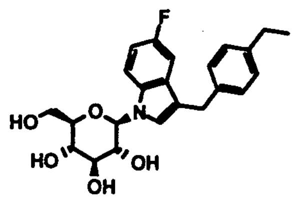 | 1.19 (3H, t, J=7.7Hz), 2.59 (2H, q, J=7.6Hz), 3.4-3.5 (1H, m), 3.5-3.6 (2H, m), 3.69 (1H, dd, J=12.5Hz, 5.7Hz), 3.8-3.9 (2H, m), 3.98 (2H, s), 5.37 (1H, d, J=9.1Hz), 6.85-6.95 (1H, m), 6.95-7.05 (1H, m), 7.05-7.15 (2H, m), 7.15-7.2 (2H, m), 7.22 (1H, s), 7.4-7.5 (1H, m) |

Table 1-9

| Example No. | Chemical structure | $^1$H-NMR ($CD_3OD$) δ ppm: |
|---|---|---|
| Ex.55 | 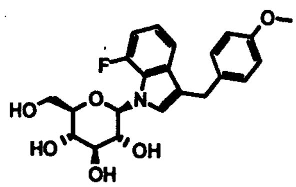 | 3.4-3.6 (3H, m), 3.68 (1H, dd, J=12Hz, 5.2Hz), 3.73 (3H, s), 3.75-3.9 (2H, m), 3.98 (2H, s), 5.67 (1H, d, J=8.9Hz), 6.75-7 (4H, m), 7.1-7.2 (3H, m), 7.22 (1 H, s) |
| Ex.56 | 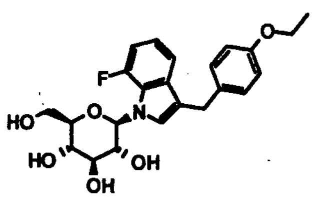 | 1.34 (3H, t, J=7Hz), 3.4-3.6 (3H, m), 3.68 (1 H, dd, J=12.2Hz, 5.3Hz), 3.75-3.9 (2H, m), 3.9-4.05 (4H, m), 5.67 (1H, d, J=8.7Hz), 6.75-7 (4H, m), 7.1-7.25 (4H, m) |
| Ex.57 | 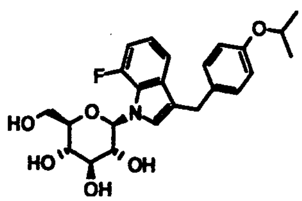 | 1.26 (6H, d, J=6Hz), 3.4-3.6 (3H, m), 3.68 (1 H, dd, J=12.3Hz, 5.1Hz), 3.75-3.9 (2H, m), 3.98 (2H, s), 4.45-4.6 (1H, m), 5.67 (1H, d, J=7.8Hz), 6.7-7 (4H, m), 7.1-7.3 (4H, m) |
| Ex.58 | 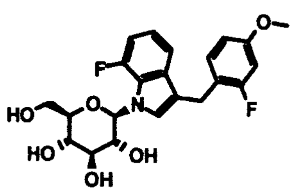 | 3.4-3.6 (3H, m), 3.67 (1H, dd, J=12.2Hz, 5.3Hz), 3.74 (3H, s), 3.75-3.9 (2H, m), 3.99 (2H, s), 5.67 (1H, d, J=8.4Hz), 6.55-6.7 (2H, m), 6.8-7 (2H, m), 7.05-7.15 (1H, m), 7.2-7.3 (2H, m) |
| Ex.59 | 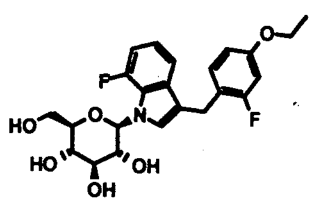 | 1.35 (3H, t, J=6.9Hz), 3.4-3.6 (3H, m), 3.67 (1H, dd, J=12.1 Hz, 5.3Hz), 3.75-3.9 (2H, m), 3.9-4.05 (4H, m), 5.67 (1H, d, J=8.8Hz), 6.55-6.7 (2H, m), 5.8-7 (2H, m), 7-7.15 (1H, m), 7.2-7.3 (2H, m) |
| Ex.60 | 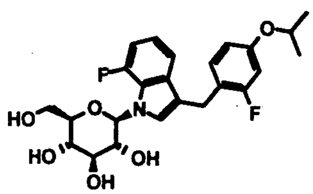 | 1.27 (6H, d, J=6Hz), 3.4-3.6 (3H, m), 3.67 (1H, dd, J=12.1 Hz, 5.4Hz), 3.75-3.9 (2H, m), 3.98 (2H, s), 4.45-4.6 (1H, m), 5.67 (1H, d, J=8.8Hz), 6.5-3.7 (2H, m), 6.8-7 (2H, m), 7-7.15 (1H, m), 7.2-7.3 (2H, m) |

Table 1-10

| Example No. | Chemical structure | $^1$H-NMR ($CD_3OD$) δ ppm: |
|---|---|---|
| Ex.61 | 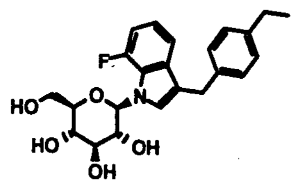 | 1.19 (3H, t, J=7.6Hz), 2.58 (2H, q, J=7.6Hz), 3.4-3.6 (3H, m), 3.68 (1 H, dd, J=12.1 Hz, 5.3Hz), 3.75-3.9 (2H, m), 4.01 (2H, s), 5.67 (1H, d, J=8.6Hz), 6.8-7 (2H, m), 7-7.1 (2H, m), 7.1-7.3 (4H, m) |
| Ex.62 | 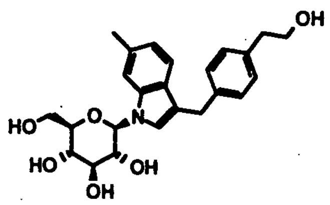 | 2.42 (3H, s), 2.76 (2H, t, J=7.1Hz), 3.4-3.5 (1 H, m), 3.5-3.6 (2H, m), 3.65-3.75 (3H, m), 3.8-3.95 (2H, m), 3.99 (2H, s), 5.38 (1H, d, J=9.1Hz), 6.8-6.85 (1H, m), 7.05-7.15 (3H, m), 7.15-7.3 (4H, m) |
| Ex.63 | 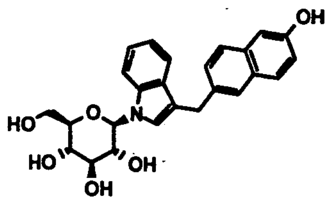 | 3.4-3.5 (1H, m), 3.5-3.65 (2H, m), 3.68 (1H, dd, J=12.2Hz, 5.7Hz), 3.8-3.95 (2H, m), 4.17 (2H, s), 5.43 (1H, d, J=9.1Hz), 6.95-7.1 (3H, m), 7.1-7.15 (1H, m), 7.19 (1H, s), 7.3-7.35 (1H, m), 7.4-7.45 (1H, m), 7.45-7.55 (2H, m), 7.55-7.65 (2H, m) |
| Ex.64 | 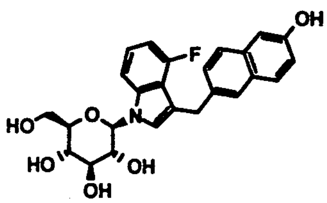 | 3.4-3.5 (1 H, m), 3.5-3.6 (2H, m), 3.68 (1 H, dd, J=12.2Hz, 5.8Hz), 3.75-3.9 (2H, m), 4.26 (2H, s), 5.39 (1H, d, J=9.3Hz), 6.65-6.75 (1H, m), 6.95-7.15 (4H, m), 7.25-7.4 (2H, m), 7.5-7.55 (1H, m), 7.55-7.65 (2H, m) |
| Ex.65 | 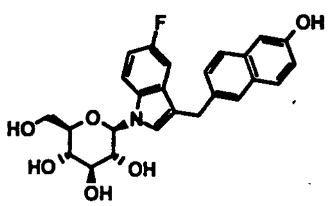 | 3.4-3.5 (1 H, m), 3.5-3.6 (2H, m), 3.69 (1 H, dd, J=12.2Hz, 5.7Hz), 3.8-3.9 (2H, m), 4.13 (2H, s), 5.39 (1H, d, J=9.1Hz), 6.85-6.95 (1H, m), 6.95-7.1 (3H, m), 7.26 (1 H, s), 7.3-7.35 (1 H, m), 7.4-7.5(1H, m), 7.5-7.65 (3H, m) |
| Ex.66 | 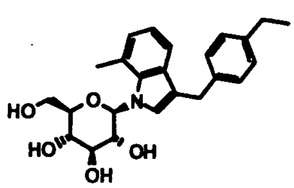 | 1.19 (3H, t, J=7.5Hz), 2.58 (2H, q, J=7.6Hz), 2.71 (3H, s), 3.35-3.45 (1H, m), 3.45-3.6 (2H, m), 3.65 (1H, dd, J=12Hz, 6Hz), 3.8-3.95 (2H, m), 4 (2H, s), 5.8 (1H, d, J=8.7Hz), 6.85-6.9 (2H, m), 7-7.1 (2H, m), 7.1-7.3 (4H, m) |

Table 1-11

| Example No. | Chemical structure | $^1$H-NMK ($CD_3OD$) δ ppm: |
|---|---|---|
| Ex.67 | 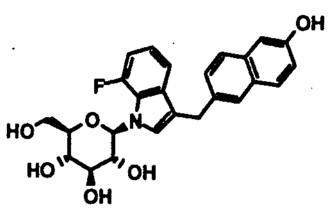 | 3.4-3.6 (3H, m), 3.68 (1H, dd, J=12Hz, 5.5Hz), 3.8-3.9 (2H, m), 4.16 (2H, s), 5.68 (1H, d, J=9.2Hz), 6.8-6.95 (2H, m), 6.95-7.1 (2H, m), 7.2-7.25 (1H, m), 7.26 (1 H, s), 7.3-7.35 (1H, m), 7.5-7.55 (1 H, m), 7.55-7.65 (2H, m) |

(continued)

| Example No. | Chemical structure | $^1$H-NMK ($CD_3OD$) δ ppm: |
| --- | --- | --- |
| Ex.68 | 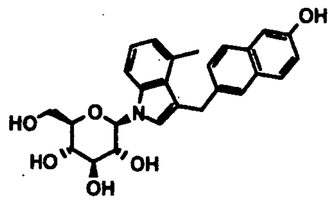 | 2.45 (3H, s), 3.4-3.5 (1H, m), 3.5-3.6 (2H, m), 3.69 (1H, dd, J=12Hz, 5.7Hz), 3.8-3.95 (2H, m), 4.36 (2H, s), 5.42 (1 H, d, J=9.2Hz), 6.7-6.75 (1 H, m), 6.95-7.15 (4H, m), 7.25-7.4 (2H, m), 7.44 (1H, s), 7.5-7.6 (2H, m) |
| Ex.69 | 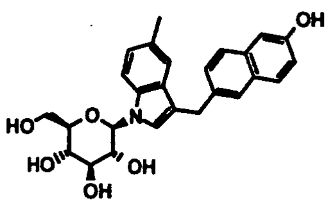 | 2.34 (3H, s), 3.4-3.5 (1H, m), 3.5-3.6 (2H, m), 3.68 (1H, dd, J=12.3Hz, 5.7Hz), 3.8-3.9 (2H, m), 4.14 (2H, s), 5.38 (1H, d, J=9Hz), 6.95-7.15 (3H, m), 7.12 (1H, s), 7.2-7.25 (1 H, m), 7.3-7.35 (1 H, m), 7.35-7.4 (1H, m), 7.5-7.55 (1H, m), 7.55-7.65 (2H, m) |
| Ex.70 | 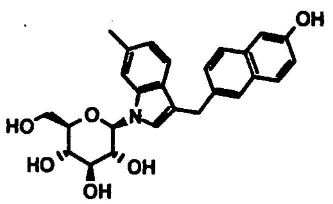 | 2.41 (3H, s), 3.4-3.5 (1H, m), 3.5-3.6 (2H, m), 3.68 (1H, dd, J=12.1 Hz, 5.7Hz), 3.8-3.95 (2H, m), 4.13 (2H, s), 5.4 (1H, d, J=9.1Hz), 6.8-6.9 (1H, m), 6.95-7.15 (3H, m), 7.25-7.35 (3H, m), 7.5-7.55 (1H, m). 7.55-7.6 (2H, m) |
| Ex.71 | 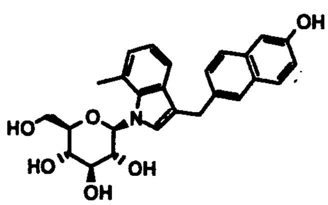 | 2.72 (3H, s), 3.35-3.45 (1H, m), 3.45-3.6 (2H, m), 3.65 (1H, dd, J=12.2Hz, 6Hz), 3.8-3.95 (2H, m), 4.15 (2H, s), 5.8 (1H, d, J=8.9Hz), 6.85-6.9 (2H, m), 6.95-7.05 (2H, m), 7.25-7.35 (3H, m), 7.45-7.6 (3H, m) |
| Ex.72 | 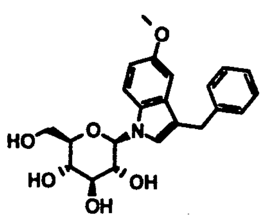 | 3.4-3.5 (1H, m), 3.5-3.6 (2H, m), 3.6-3.75 (4H, m), 3.8-3.95 (2H, m), 4.03 (2H, s), 5.34 (1H, d. J=8.9Hz), 6.75-6.9 (2H, m), 7.05-7.2 (2H, m), 7.2-7.3 (4H, m), 7.38 (1H, d, J=8.9Hz) |

Table 1-12

| Example No. | Chemical structure | $^1$H-NMR ($CD_3OD$) δ ppm: |
| --- | --- | --- |
| Ex.73 | 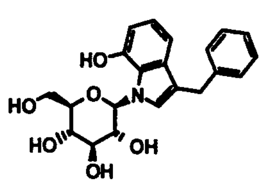 | 3.4-3.6 (3H, m), 3.68 (1H, dd, J=12.1Hz, 5.2Hz), 3.75-3.9 (2H, m), 4.02 (2H, s), 6.22 (1H, d, J=8.9Hz), 6.5-6.6 (1H, m), 6.75-6.85 (1H, m), 6.85-6.95 (1H, m), 7.05-7.35 (6H, m) |
| Ex.74 | 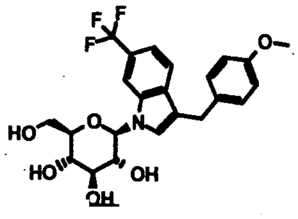 | 3.4-3.55 (1H, m), 3.55-3.65 (2H, m), 3.65-3.8 (4H, m), 3.8-3.95 (2H, m), 4.04 (2H, s), 5.51 (1H, d, J=9.2Hz), 6.75-6.85 (2H, m), 7.1-7.3 (3H, m), 7.37 (1H, s), 7.45-7.6 (1H, m), 7.84 (1H, s) |

(continued)

| Example No. | Chemical structure | $^1$H-NMR ($CD_3OD$) δ ppm: |
|---|---|---|
| Ex.75 | 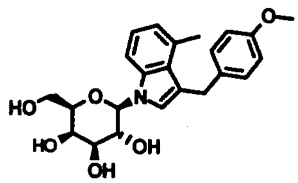 | 2.43 (3H, s), 3.65-3.85 (7H, m), 3.97 (1H, d, J=3.1Hz), 4.15-4.25 (3H, m), 5.33 (1H, d, J=8.8Hz), 6.72 (1H, d, J=7.2Hz), 6.82 (2H, d, J=8.8Hz), 6.95-7.05 (1H, m), 7.05-7.15 (3H, m), 7.39 (1 H, d, J=8.0Hz) |
| Ex.76 | 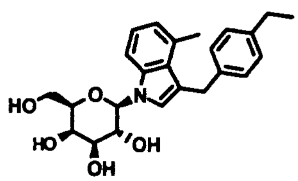 | 1.20 (3H, t, J=7.5Hz), 2.43 (3H, s), 2.59 (2H, q, J=7.5Hz), 3.65-3.85 (4H, m), 3.98 (1H, d, J=3.1 Hz), 4.15-4.25 (3H, m), 5.33 (1 H, d, J=8.7Hz), 6.72 (1 H, d, J=7.4Hz), 6.95-7.05 (1H, m), 7.05-7.15 (5H, m), 7.39 (1 H, d, J=8.1 Hz) |

Example 77

6-Chloro-1-(β-D-glucopyranosyl)-3-(4-methoxybenzyl)-1H-indole

[Chem.20]

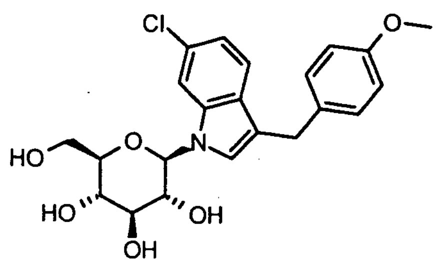

To a solution of 4-bromoanisole (0.12g) in tetrahydrofuran (0.5 mL) were added magnesium (18 mg) and a catalytic amount of iodine, and the resulting mixture was heated with stirring to prepare Grignard reagent (4-methoxyphenylmagnesium bromide). To a solution of 1-(2,3,4,6-tetra-*O*-benzyl-β-D-gluco-pyranosyl)-6-chloro-3-formyl-1H-indole (0.35 g) in tetrahydrofuran (2 mL) was added the above Grignard reagent under ice-cooling, and the resulting mixture was stirred at room temperature overnight. To the reaction mixture was added a saturated aqueous ammonium chloride solution to quench the reaction, and the resulting mixture was extracted with ethyl acetate. The organic layer was washed with 1M hydrochloric acid, water and brine successively, and dried over anhydrous sodium sulfate. The solvent was removed under reduced pressure, and the residue was purified by column chromatography on silica gel (eluent: *n*-hexane/ethyl acetate = 100/0-50/50) to give the corresponding adduct compound, 1-(2,3,4,6-tetra-*O*-benzyl-β-D-glucopyranosyl)-6-chloro-3-[hydroxy-(4-methoxyphenyl)-methyl]-1H-indole (0.34g).
To a solution of the obtained adduct compound (0.33 g) in dichloromethane (5 mL) were added dropwise triethylsilane (0.24 g) and boron trifluoride diethyl ether complex (0.29 g) successively under ice-cooling in a methanol bath, and the resulting mixture was stirred for 1 hour. To the reaction mixture was added a saturated aqueous potassium carbonate solution, and the resulting mixture was stirred for 10 minutes. The mixture was extracted with dichloromethane. The organic layer was washed with water and brine successively, and dried over anhydrous sodium sulfate. The solvent was removed under reduced pressure, and the residue was purified by column chromatography on silica gel (eluent: n-hexane/ethyl acetate = 10/1-2/1) to give the corresponding reduced compound, 1-(2,3,4,6-tetra-*O*-benzyl-β-D-glucopyranosyl)-6-chloro-3- (4-methoxy-benzyl)-1H-indole (0.25 g).
To a solution of the obtained reduced compound (60 mg) in tetrahydrofuran/methanol (= 1/1, 3 mL) was added 10% palladium-carbon powder (15 mg), and the mixture was stirred under a hydrogen atmosphere for 1 hour at room temperature. The insoluble material was removed by filtration, and the solvent of the filtrate was removed under reduced

pressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (17 mg).

$^1$H-NMR ($CD_3OD$) δ ppm:

3.45-3.5 (1H, m), 3.5-3.6 (2H, m), 3.69 (1H, dd, J=12.1 Hz, 5.7 Hz), 3.7 (3H,s), 3.8-3.9 (2H, m), 3.98 (2H, s), 5.37 (1H, d, J=9 Hz), 6.75-6.85 (2H, m), 6.9-7 (1H, m), 7.1-7.2 (3H, m), 7.25-7.35 (1H, m), 7.45-7.55 (1H, m).

Examples 78 to 87

The compounds described in Table 2 were prepared in a similar manner to that described in Example 77 using the corresponding 1-(2,3,4,6-tetra-*O*-benzyl-β-D-gluco-pyranosyl)-formyl-1H-indole compounds, and using the corresponding bromobenzene compounds instead of 4-bromoanisole.

[Table 2]

Table 2-1

| Example No. | Chemical structure | $^1$H-NMR ($CD_3OD$) δ ppm: |
|---|---|---|
| Ex.78 | 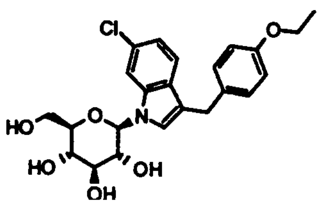 | 1.34 (3H, t, J=7.1 Hz), 3.4-3.5 (1H, m), 3.5-3.65 (2H, m), 3.69 (1H, dd, J=12.1Hz, 5.9Hz), 3.75-3.9 (2H, m), 3.9-4.05 (4H, m), 5.38 (1H, d, J=9.1 Hz), 6.75-6.85 (2H, m), 6.97 (1H, dd, J=8.5Hz, 1.7Hz), 7.1-7.2 (3H, m). 7.32 (1 H, d, J=8.6Hz), 7.53 (1H, d, J=1.6Hz) |
| Ex.79 | 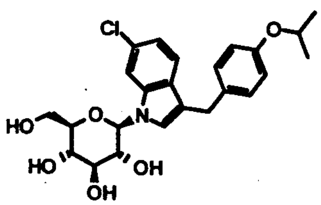 | 1.26 (6H, d, J=6.2Hz), 3.4-3.5 (1H, m), 3.5-3.65 (2H, m), 3.69 (1H, dd, J=12.2Hz, 5.8Hz), 3.8-3.9 (2H, m), 3.95 (2H, s), 4.45-4.6 (1H, m), 5.38 (1H, d, J=9.1 Hz), 6.75-6.85 (2H, m), 6.97 (1 H, dd, J=8.6Hz, 1.8Hz), 7.1-7.2 (3H, m), 7.33 (1H, d, J=8.3Hz), 7.53 (1H, d, J=6.2Hz) |
| Ex.80 | 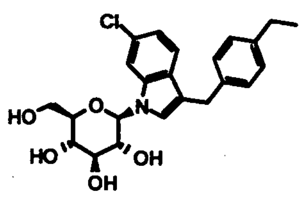 | 1.19 (3H, t, J=7.6Hz), 2.58 (2H, q, J=7.6Hz), 3.4-3.5 (1H, m), 3.5-3.6 (2H, m), 3.69 (1H, dd, J=12.2Hz, 5.7Hz), 3.75-3.95 (2H, m), 4.0 (2H, s), 5.38 (1 H, d, J=9.3Hz), 6.9-7 (1H, m), 7-7.1 (2H, m), 7.1-7.25 (3H, m), 7.3-7.4 (1 H, m), 7.5-7.55 (1 H, m) |
| Ex.81 | 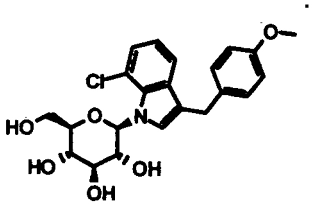 | 3.4-3.5 (1 H, m), 3.5-3.6 (2H, m), 3.69 (1 H, dd, J=12.5Hz, 5Hz), 3.74 (3H, s), 3.8-3.9 (2H, m), 3.99 (2H, s), 6.38 (1H, d, J=9.5Hz), 6.75-6.85 (2H, m), 6.9-7 (1H, m), 7.1-7.2 (3H, m), 7.25-7.4 (2H, m) |
| Ex.82 | 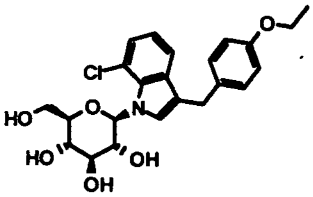 | 1.34 (3H, t, J=7Hz), 3.4-3.5 (1H, m), 3.5-3.6 (2H, m), 3.69 (1H, dd, J=12.2Hz, 5.1 Hz), 3.75-4.05 (6H, m), 6.38 (1 H, d, J=9Hz), 6.75-6.85 (2H, m), 6.9-7 (1H, m), 7.1-7.2 (3H, m), 7.25-7.4 (2H, m) |

(continued)

| Example No. | Chemical structure | $^1$H-NMR ($CD_3OD$) δ ppm: |
|---|---|---|
| Ex.83 | 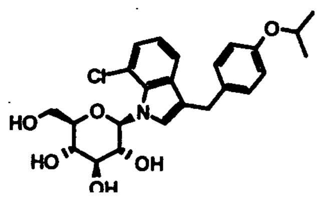 | 1.27 (6H, d, J=6.2Hz), 3.4-3.5 (1 H, m), 3.5-3.65 (2H, m), 3.69 (1 H, dd, J=12.2Hz, 5.1Hz), 3.8-3.95 (2H, m), 3.99 (2H, s), 4.45-4.6 (1H, m), 6.38 (1H, d, J=9.3Hz), 6.75-6.85 (2H, m), 6.9-7 (1H, m), 7.1-7.2 (3H, m), 7.25-7.4 (2H, m) |
| Ex.84 | 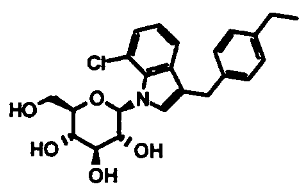 | 1.19 (3H, t, J=7.5Hz), 2.58 (2H, q, J=7.6Hz), 3.4-3.5 (1H, m), 3.5-3.6 (2H, m), 3.69 (1 H, dd, J=12.2Hz, 5Hz), 3.8-3.95 (2H, m), 4.02 (2H, s), 6.38 (1H, d, J=9.2Hz), 6.9-7 (1H, m), 7.05-7.1 (2H, m), 7.1-7.2 (3H, m), 7.25-7.4 (2H, m) |

Table 2-2

| Example No. | Chemical structure | $^1$H-NMR ($CD_3OD$) δ ppm: |
|---|---|---|
| Ex.85 | 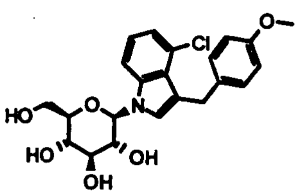 | 3.4-3.5 (1 H, m), 3.5-3.6 (2H, m), 3.68 (1 H, dd, J=12.2Hz, 5.8Hz), 3.75 (3H, s), 3.75-3.9 (2H, m), 4.25 (1H, d, J=16.3Hz), 4.29 (1H, d, J=16.3Hz), 5.4 (1 H, d, J=9Hz), 6.75-6.85 (2H, m), 6.99 (1 H, d, J=7.5Hz), 7-7.1 (2H, m), 7.1-7.15 (2H, m), 7.46 (1 H, d, J=8.5Hz) |
| Ex.86 | 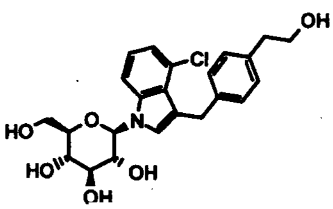 | 2.77 (2H, t, J=7.2Hz), 3.4-3.5 (1 H, m), 3.5-3.6 (2H, m), 3.65-3.75 (3H, m), 3.75-3.9 (2H, m), 4.28 (1H, d, J=16.2Hz), 4.32 (1H, d, J=16.2Hz), 5.4 (1H, d, J=9.1 Hz), 6.95-7.2 (7H, m), 7.4-7.5 (1H, m) |
| Ex.87 | 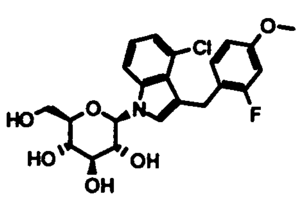 | 3.4-3.5 (1 H, m), 3.5-3.6 (2H, m), 3.69 (1 H, dd, J=12Hz, 5.8Hz), 3.75 (3H, s), 3.75-3.9 (2H, m), 4.24 (1H, d, J=16.3Hz), 4.29 (1H, d, J=16.3Hz), 5.41 (1H, d, J=9.1Hz), 6.55-6.7 (2H, m), 6.9-7.05 (2H, m), 7.05-7.15 (2H, m), 7.4-7.5 (1H, m) |

Example 88

4-Chloro-3-(4-ethylbenzyl)-1-(β-D-glucopyranosyl)-1H-indole

[Chem.21]

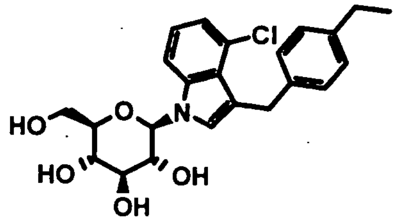

To a solution of 4-bromo ethylbenzene (0.1 g) in tetrahydrofuran (3 mL) was added n-butylllitium (2. 67 M, hexane solution, 0.2mL) under an argon atmosphere under dry ice-cooling in an acetone bath, and the resulting mixture was stirred for 20 minutes. To the reaction mixture was added a solution of 1-(2,3,4,6-tetra-*O*-benzyl-β-D-glucopyranosyl)-4-chloro-3-formyl-1H-indole (0.15 g) in tetrahydrofuran (1 mL), and the resulting mixture was stirred for 1 hour under ice-cooling and for 1 hour at room temperature. To the reaction mixture was added a aqueous ammonium chloride solution to quench the reaction, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: n-hexane/ethyl acetate = 5/1-1/1) to give the corresponding adduct compound, 1-(2,3,4,6-tetra-*O*-benzyl-β-D-glucopyranosyl)-4-chloro-3-[hydroxy-(4-ethylyphenyl)methyl]-1H-indole (0.2g).

To a solution of the obtained adduct compound (0.17 g) in dichloromethane (5 mL) were added dropwise triethylsilane (0.12 g) and boron trifluoride diethyl ether complex (0.15 g) successively, and the resulting mixture was stirred for 1 hour. To the reaction mixture was added a saturated aqueous potassium carbonate solution, and the resulting mixture was stirred for 10 minutes. The mixture was extracted with dichloromethane. The organic layer was washed with water and brine successively, and dried over anhydrous sodium sulfate. The solvent was removed under reduced pressure, and the residue was purified by column chromatography on silica gel (eluent: *n*-hexane/ethyl acetate = 10/1-2/1) to give the corresponding reduced compound, 1-(2,3,4,6-tetra-*O*-benzyl-β-D-glucopyranosyl)-4-chloro-3-(4-ethylbenzyl)-1H-indole (125 mg).

To a solution of the obtained reduced compound (125 mg) in tetrahydrofuran/methanol (= 1/1, 4 mL) was added 10% palladium-carbon powder (30 mg), and the mixture was stirred under a hydrogen atmosphere for 2 hour at room temperature. The insoluble material was removed by filtration, and the solvent of the filtrate was removed under reduced pressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (12 mg).

$^1$H-NMR ($CD_3OD$) δ ppm:

1.2 (3H, t, J=7.6Hz), 2.59 (2H, q, J=7.6Hz), 3.4-3.5 (1H, m), 3.5-3.6 (2H, m) , 3.68 (1H, dd, J=12. 1 Hz, 5.7 Hz) , 3.75-3.9 (2H, m), 4.27 (1H, d, J=16.3 Hz), 4.31 (1H, d, J=16.4Hz), 5.4 (1H, d, J=9.1 Hz), 6.95-7.2 (7H, m), 7.4-7.5 (1H, m).

Examples 89 to 91

The compounds described in Table 3 were prepared in a similar manner to that described in Example 88, using the corresponding 2-benzyloxy-6-bromonaphthalene instead of 4-bromoethylbenzene.

[Table 3]

| Example No. | Chemical structure | $^1$H-NMR ($CD_3OD$) δ ppm: |
|---|---|---|
| Ex.89 | 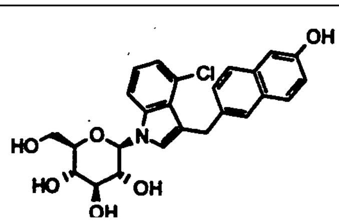 | 3.4-3.5 (1 H, m), 3.5-3.6 (2H, m), 3.68 (1H, dd, J=12.2Hz, 5.6Hz), 3.75-3.9 (2H, m), 4.35-4.55 (2H, m), 5.41 (1H, d, J=9.1 Hz), 6.95-7.05 (2H, m), 7.05-7.15 (3H, m), 7.25-7.35 (1 H, m), 7.45-7.6 (4H, m) |

(continued)

| Example No. | Chemical structure | $^1$H-NMR ($CD_3OD$) δ ppm: |
|---|---|---|
| Ex.90 | 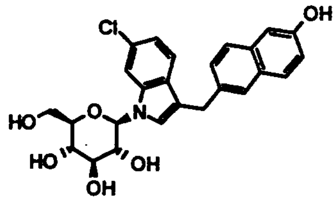 | 3.4-3.5 (1 H, m), 3.5-3.65 (2H, m), 3.69 (1H, dd, J=12.1 Hz, 5.8Hz), 3.8-3.95 (2H, m), 4.15 (2H, s), 5.39 (1H, d, J=9.2Hz), 6.9-7.1 (3H, m), 7.22 (1H, s), 7.25-7.4 (2H, m), 7.45-7.65 (4H, m) |
| Ex.91 | 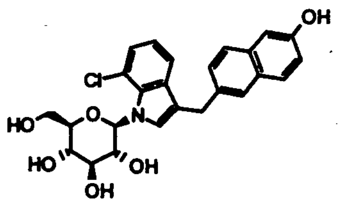 | 3.4-3.5 (1 H, m), 3.5-3.6 (2H, m), 3.69 (1H, dd, J=12.1 Hz, 5.3Hz), 3.8-3.95 (2H, m), 4.17 (2H, s), 6.39 (1 H, d, J=9.1Hz), 6.9-7.1 (3H, m), 7.1-7.15 (1 H, m), 7.25-7.4 (3H, m), 7.5-7.55 (1H, m), 7.55-7.65 (2H, m) |

Examples 92 to 316

The compounds described in Table 4 can be prepared in a similar manner to that described in Example 2, Example 77 or Example 88, by means of the reactions 1-(2,3,4,6-tetra-*O*-benzyl-β-D-glucopyranosyl)-3-formyl-1H-indole compounds with organometallic reagents such as Grignard reagents, alkyllitium reagents or the like.
[Table 4]

Table 4-1

| | | |
|---|---|---|
| 92 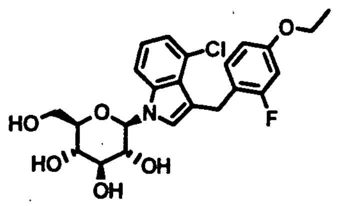 | 93 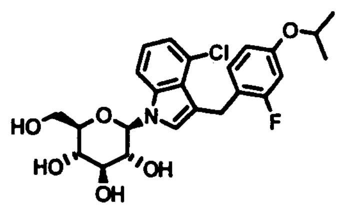 | 94 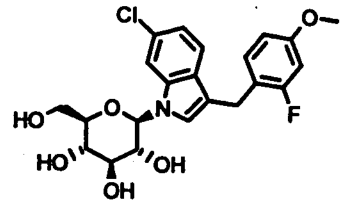 |
| 95 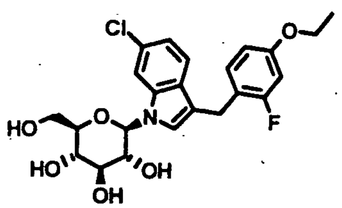 | 96 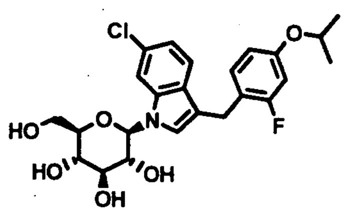 | 97 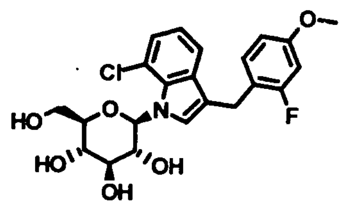 |
| 98 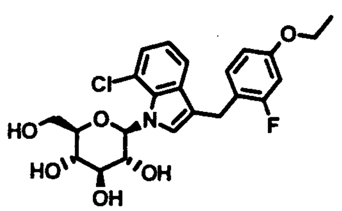 | 99 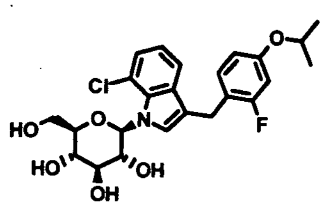 | 100 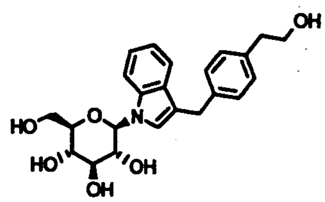 |
| 101 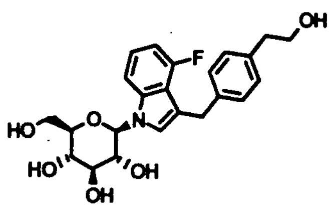 | 102 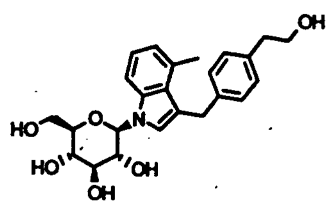 | 103 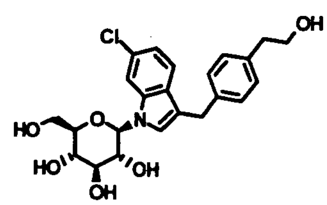 |
| 104 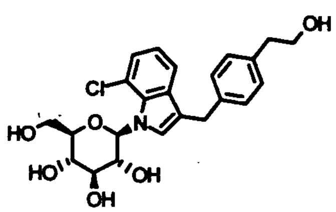 | 105 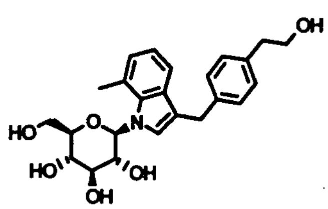 | 106 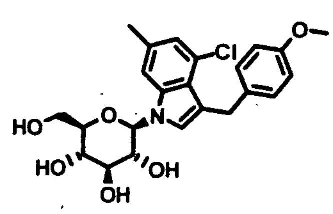 |
| 107 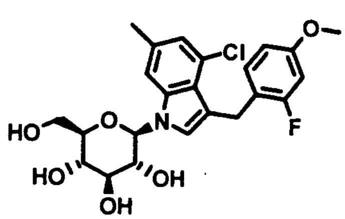 | 108 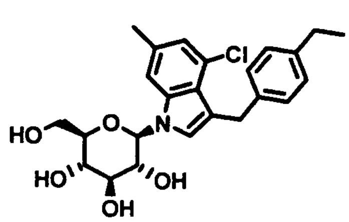 | 109 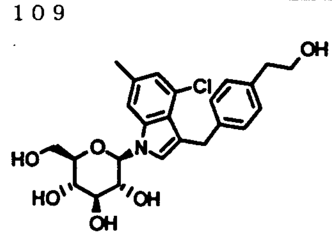 |

Table 4-2

| | | |
|---|---|---|
| 110 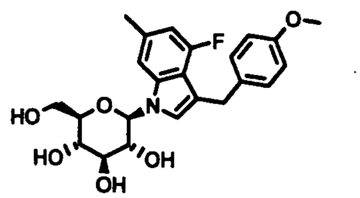 | 111 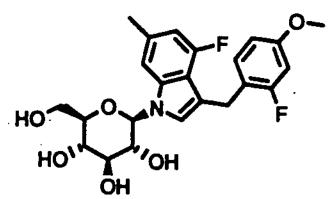 | 112 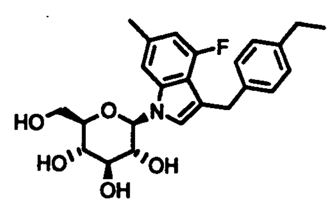 |
| 113 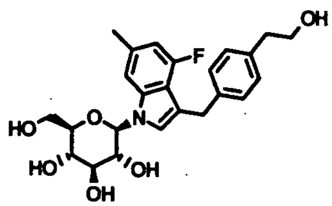 | 114 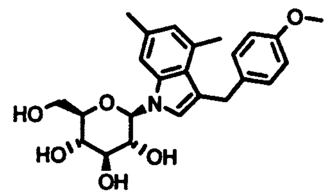 | 115 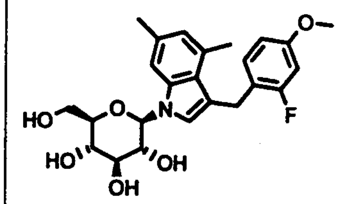 |
| 116 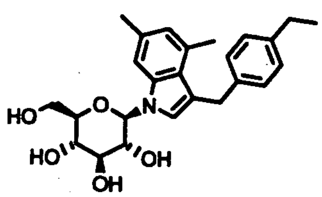 | 117 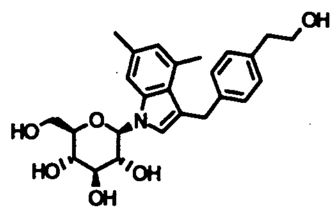 | 118 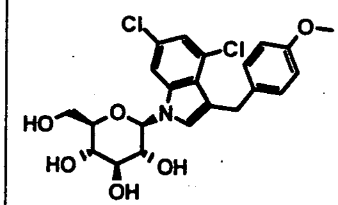 |
| 119 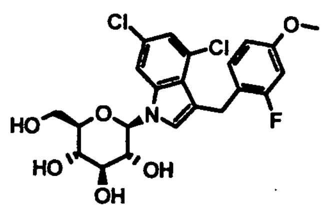 | 120 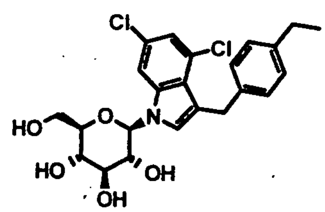 | 121 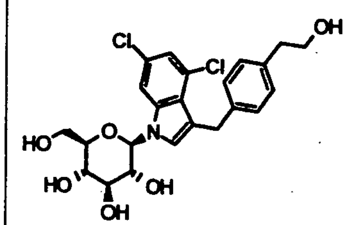 |
| 122 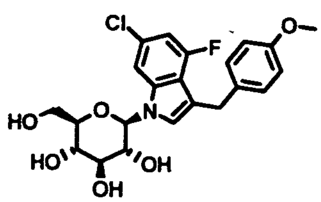 | 123 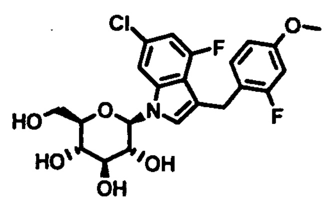 | 124 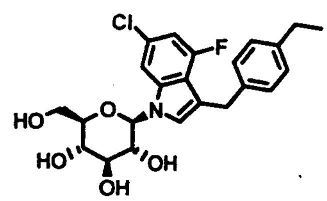 |
| 125 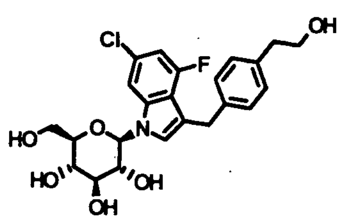 | 126 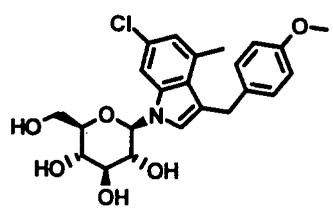 | 127 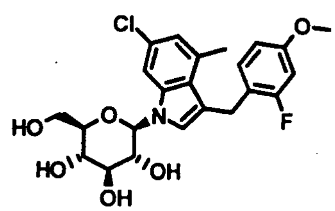 |

Table 4-3

| | | |
|---|---|---|
| 128 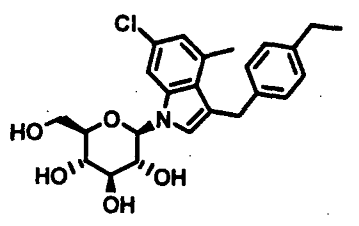 | 129 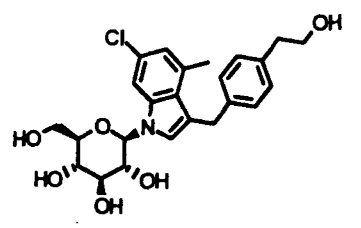 | 130 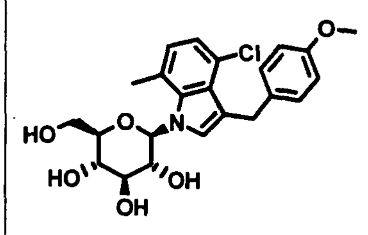 |
| 131 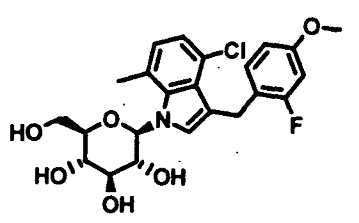 | 132 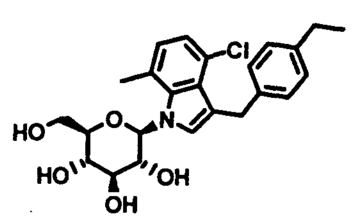 | 133 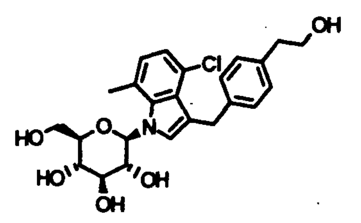 |
| 134 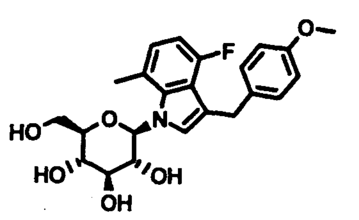 | 135 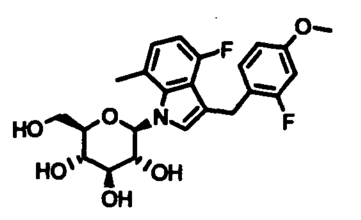 | 136 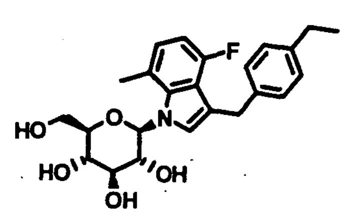 |
| 137 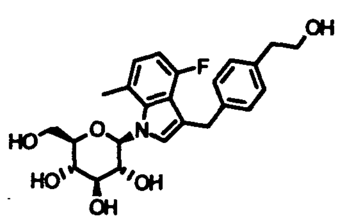 | 138 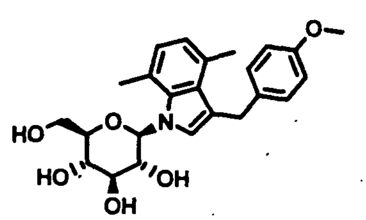 | 139 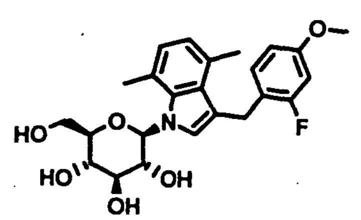 |
| 140 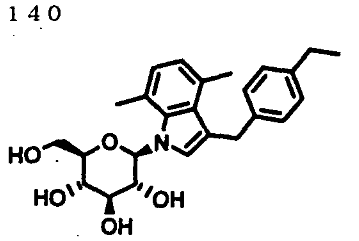 | 141 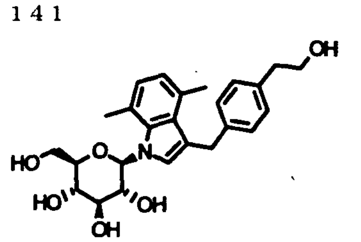 | 142 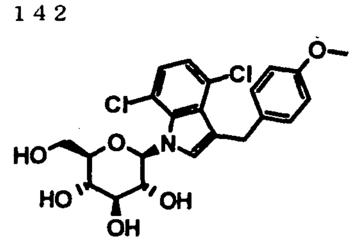 |
| 143 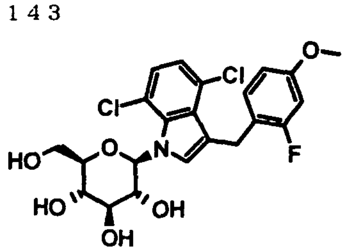 | 144 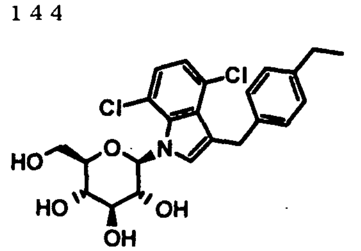 | 145 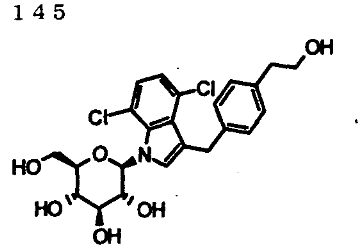 |

Table 4-4

| | | |
|---|---|---|
| 146 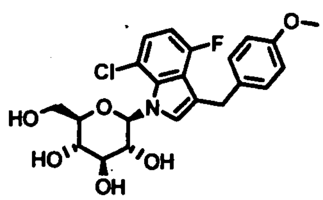 | 147 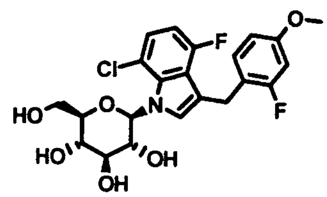 | 148 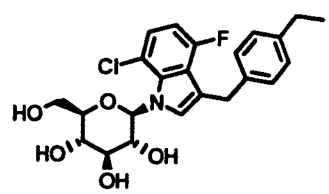 |
| 149 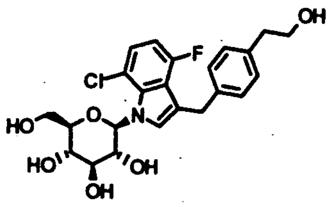 | 150 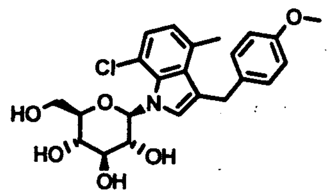 | 151 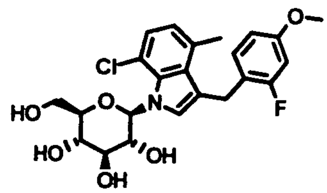 |
| 152 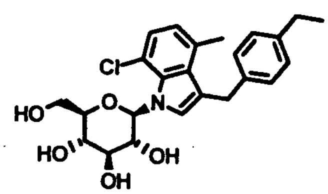 | 153 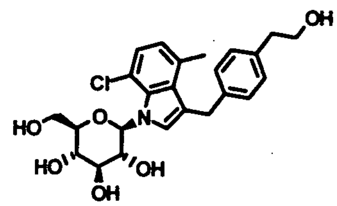 | 154 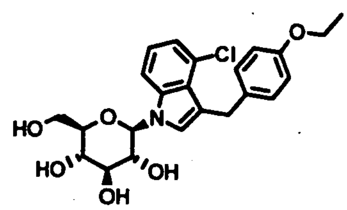 |
| 155 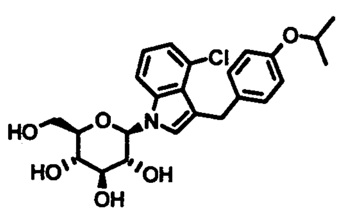 | 156 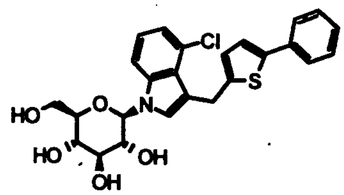 | 157 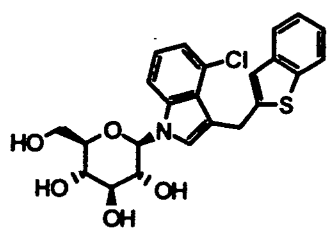 |
| 158 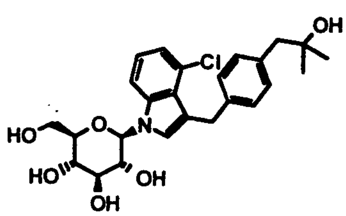 | 159 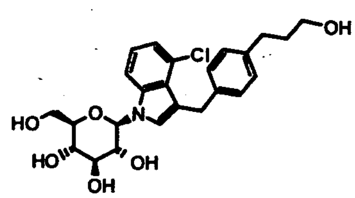 | 160 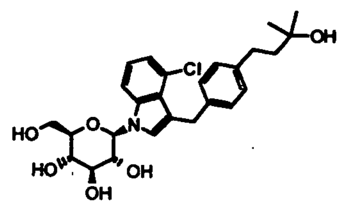 |
| 161 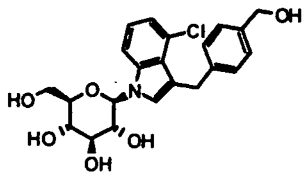 | 162 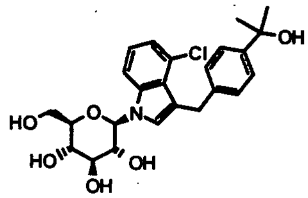 | 163 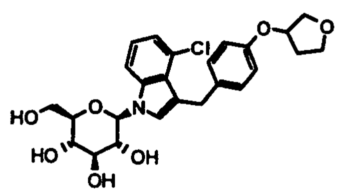 |

Table 4-5

| | | |
|---|---|---|
| 164 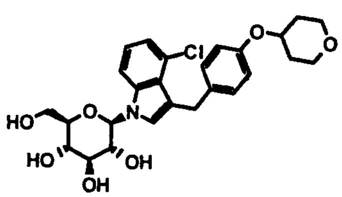 | 165 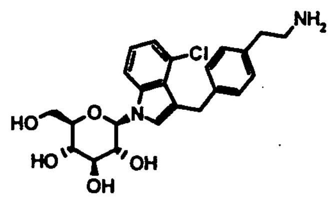 | 166 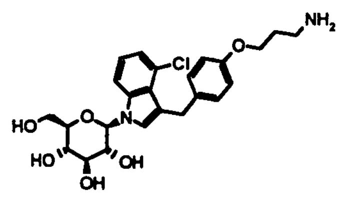 |
| 167 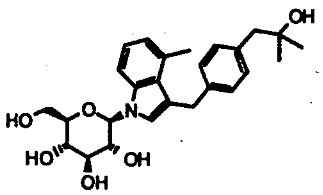 | 168 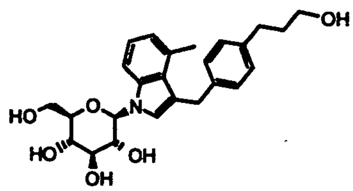 | 169 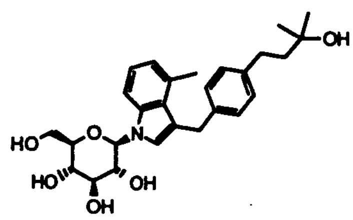 |
| 170 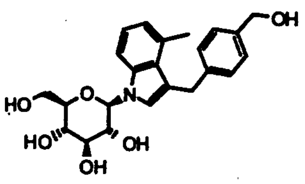 | 171 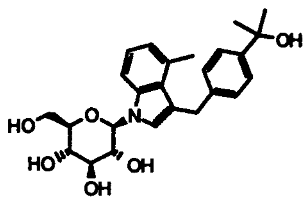 | 172 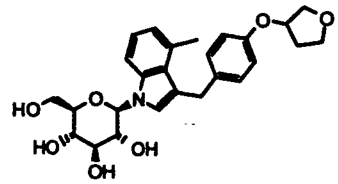 |
| 173 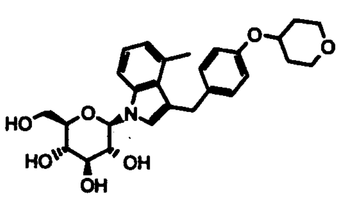 | 174 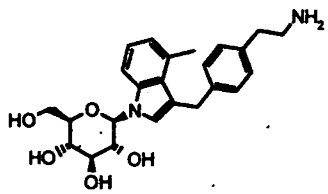 | 175 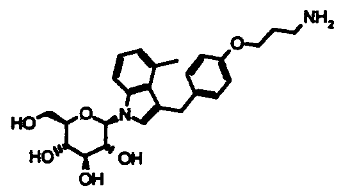 |
| 176 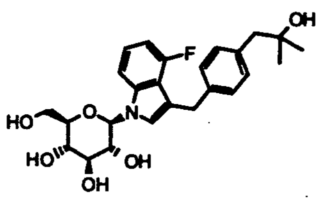 | 177 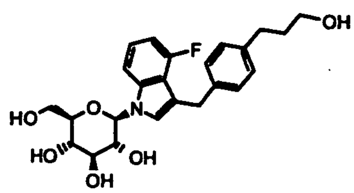 | 178 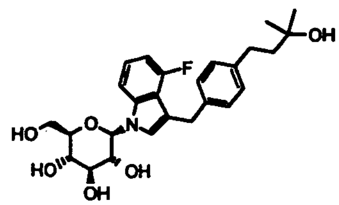 |
| 179 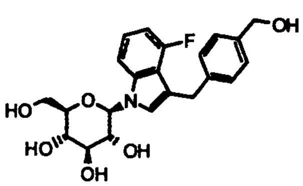 | 180 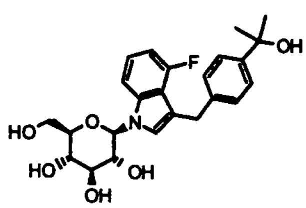 | 181 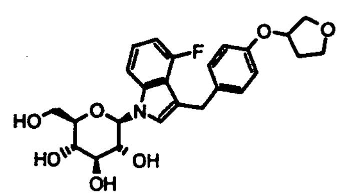 |

Table 4-6

| | | |
|---|---|---|
| 182 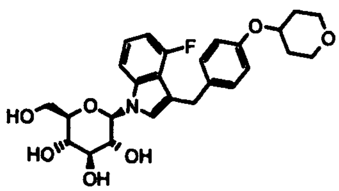 | 183 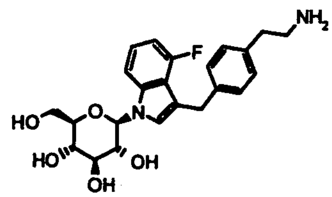 | 184 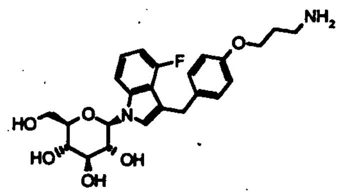 |
| 185 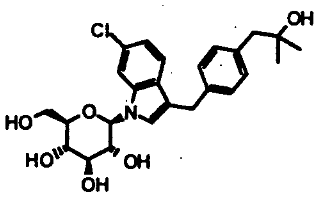 | 186 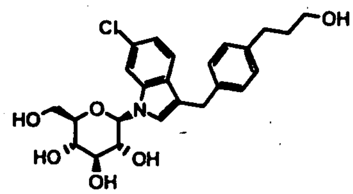 | 187 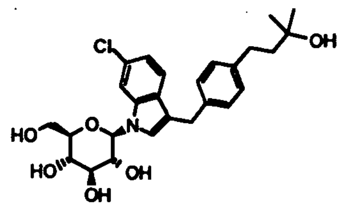 |
| 188 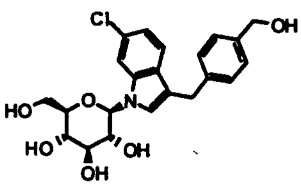 | 189 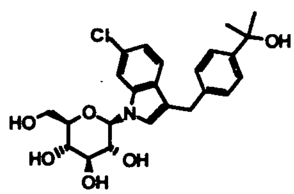 | 190 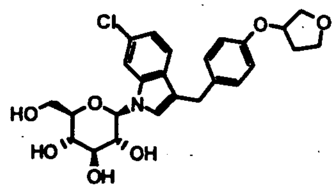 |
| 191 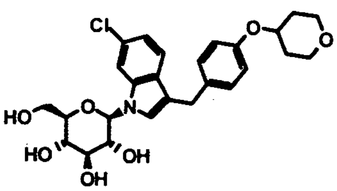 | 192 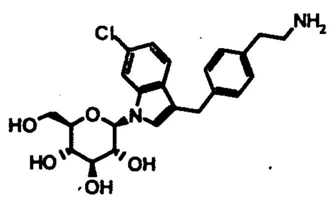 | 193 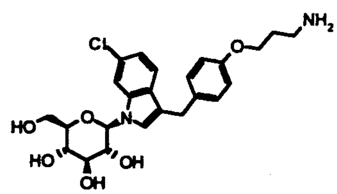 |
| 194 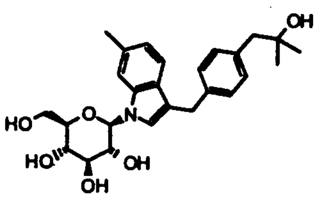 | 195 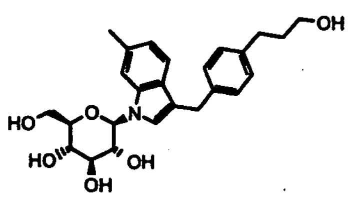 | 196 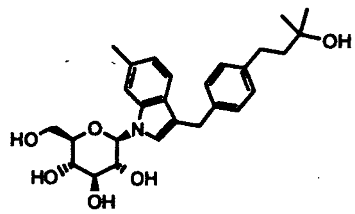 |
| 197 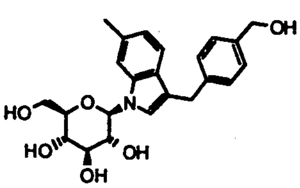 | 198 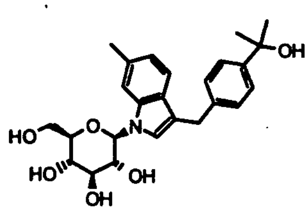 | 199 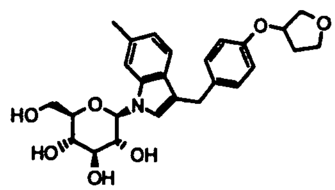 |

Table 4-7

| | | |
|---|---|---|
| 200 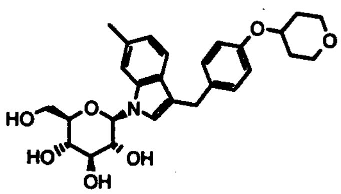 | 201 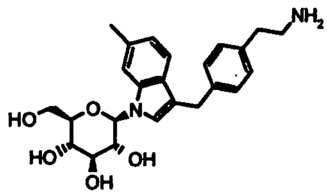 | 202 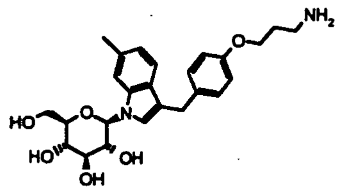 |
| 203 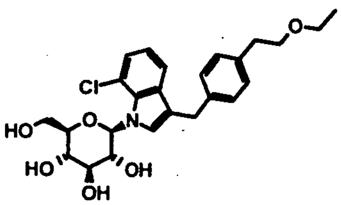 | 204 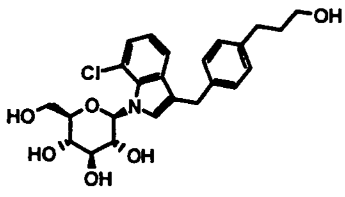 | 205 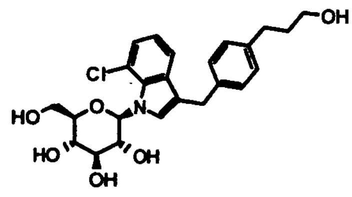 |
| 206 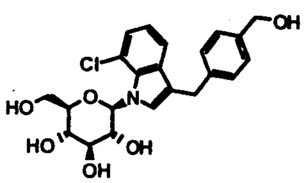 | 207 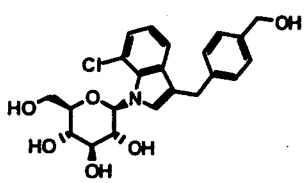 | 208 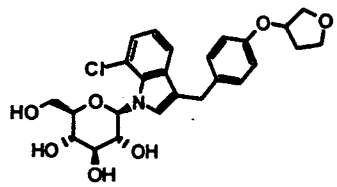 |
| 209 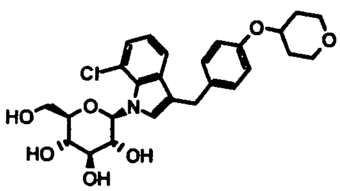 | 210 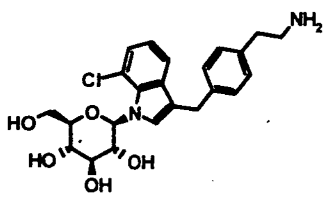 | 211 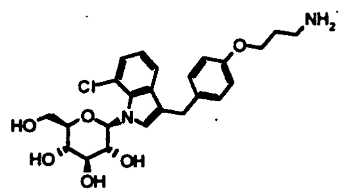 |
| 212 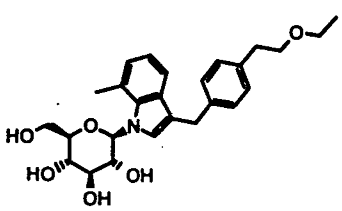 | 213 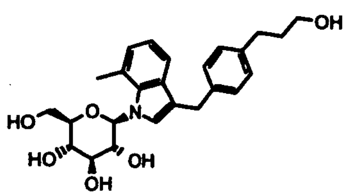 | 214 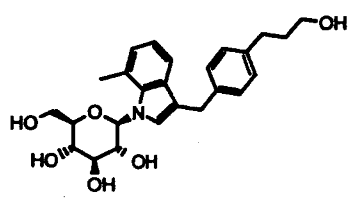 |
| 215 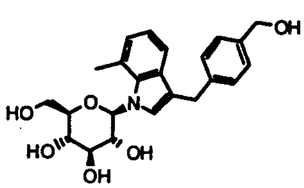 | 216 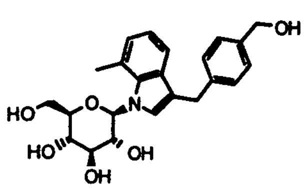 | 217 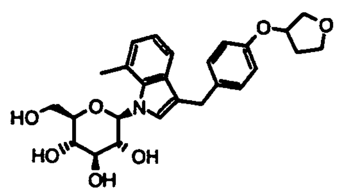 |

Table 4-8

| | | |
|---|---|---|
| 218 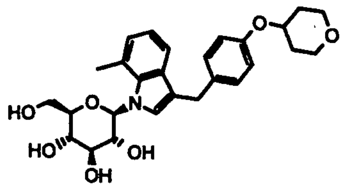 | 219 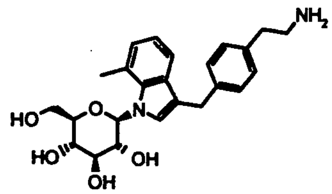 | 220 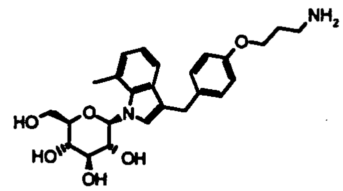 |
| 221 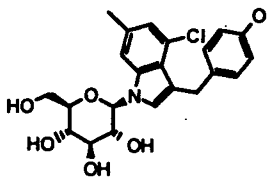 | 222 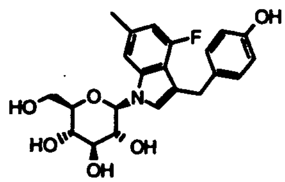 | 223 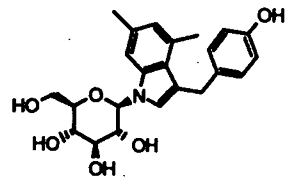 |
| 224 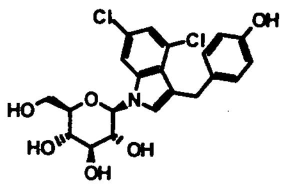 | 225 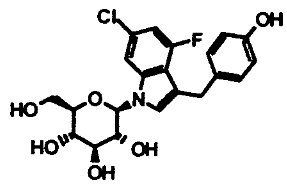 | 226 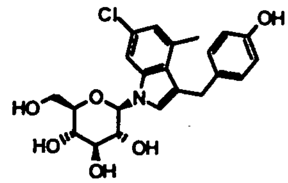 |
| 227 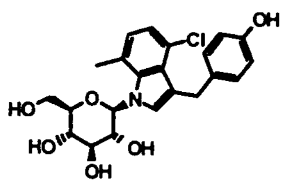 | 228 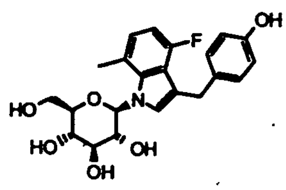 | 229 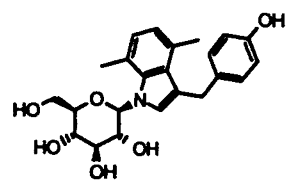 |
| 230 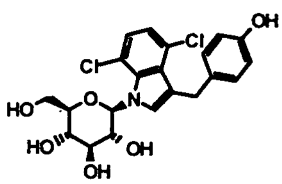 | 231 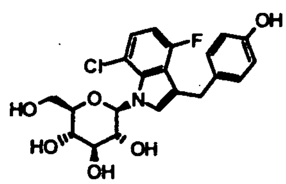 | 232 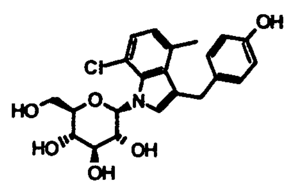 |
| 233 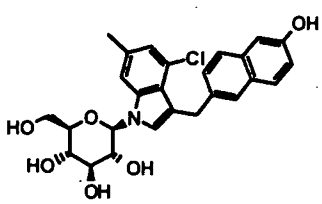 | 234 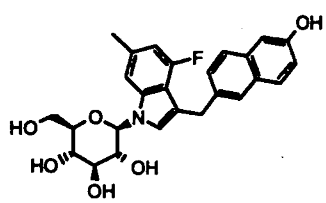 | 235 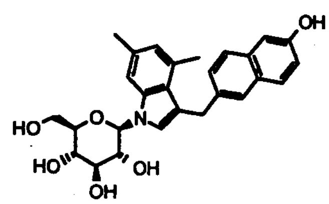 |

Table 4-9

| | | |
|---|---|---|
| 236 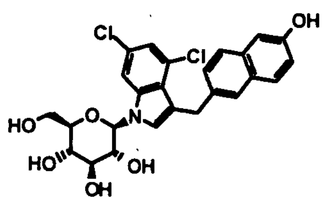 | 237 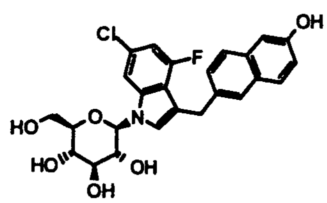 | 238 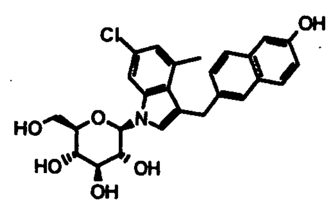 |
| 239 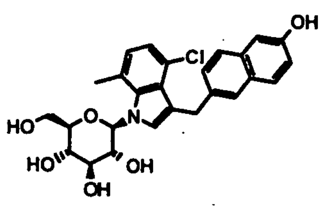 | 240 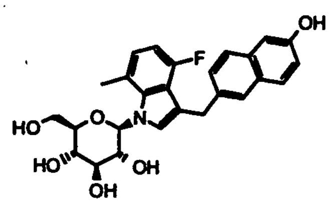 | 241 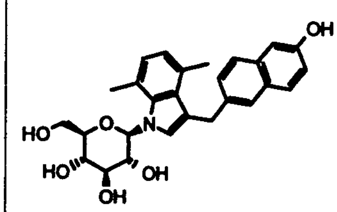 |
| 242 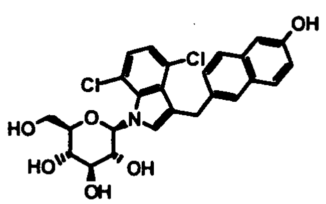 | 243 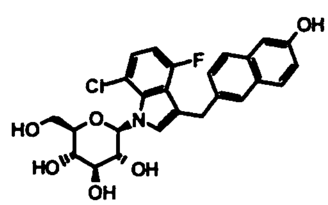 | 244 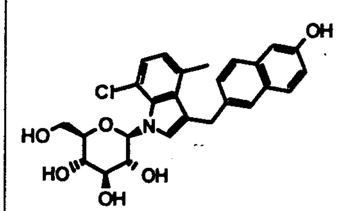 |
| 245 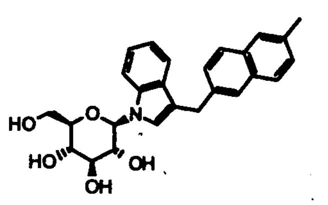 | 246 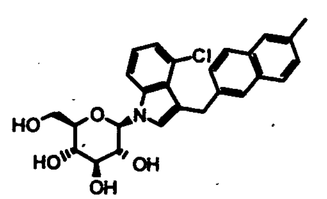 | 257 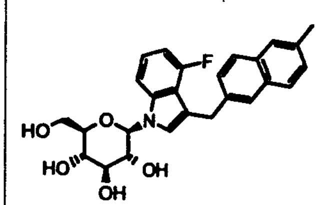 |
| 248 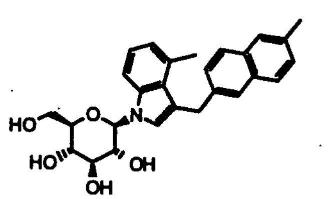 | 249 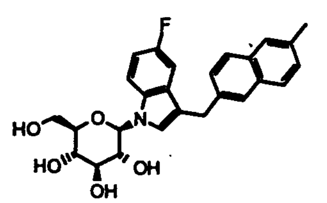 | 250 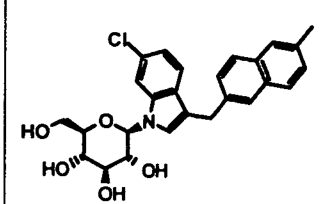 |
| 251 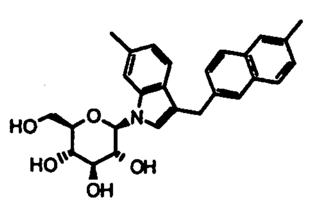 | 252 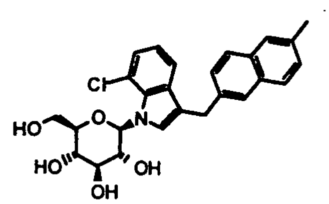 | 253 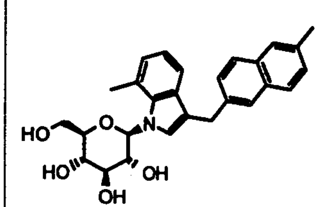 |

Table 4-10

| | | |
|---|---|---|
| 254 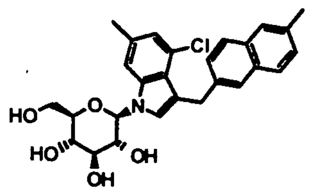 | 255 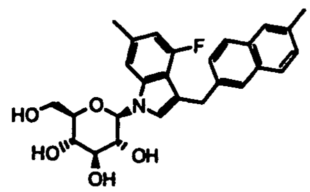 | 256 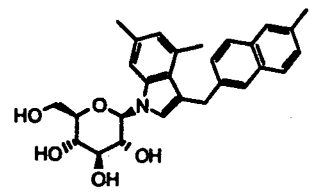 |
| 257 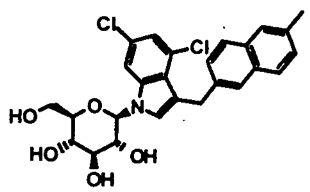 | 258 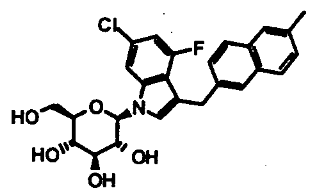 | 259 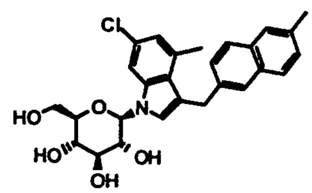 |
| 260 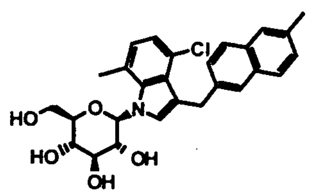 | 261 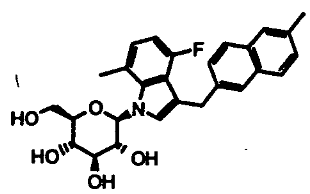 | 262 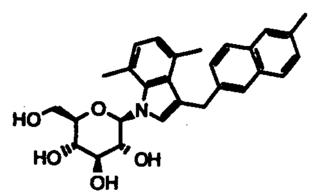 |
| 263 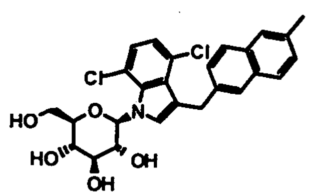 | 264 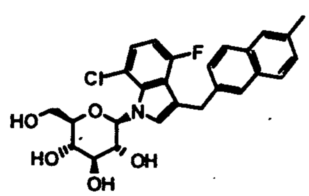 | 265 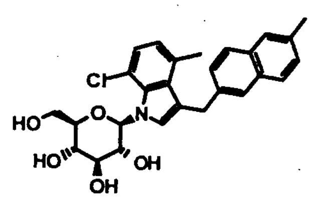 |
| 266 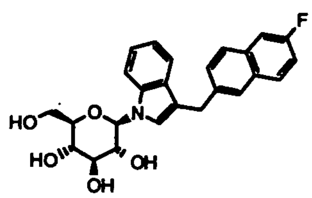 | 267 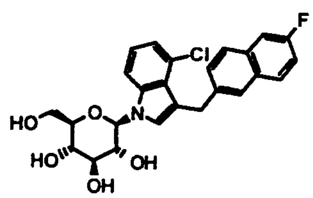 | 268 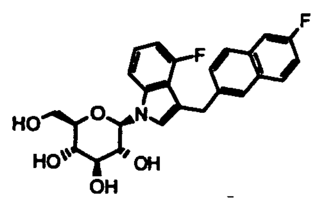 |
| 269 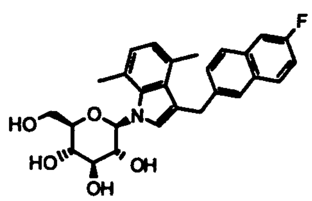 | 270 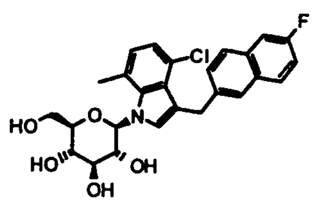 | 271 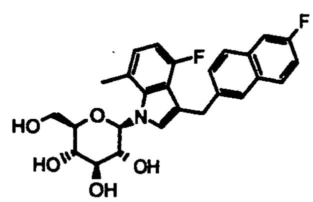 |

Table 4-11

| | | |
|---|---|---|
| 272 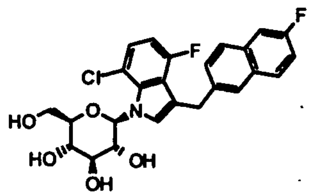 | 273 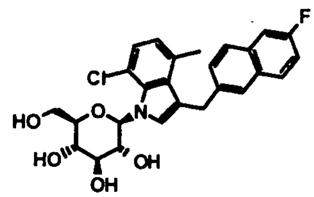 | 274 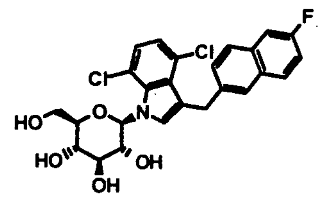 |
| 275 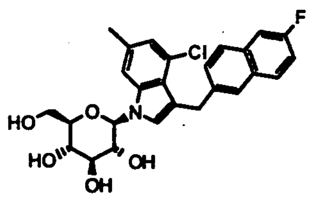 | 276 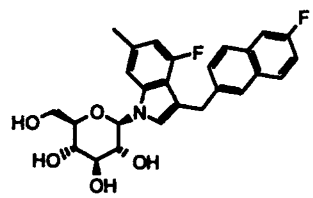 | 277 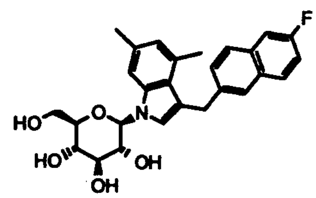 |
| 278 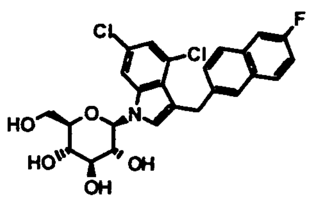 | 279 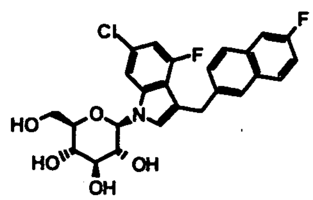 | 280 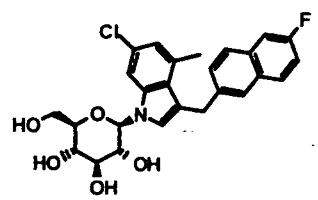 |
| 281 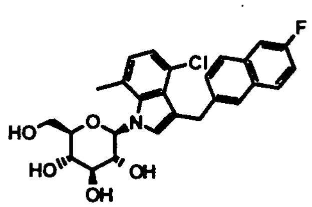 | 282 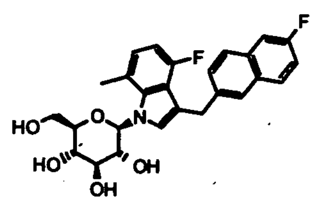 | 283 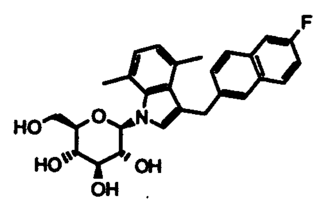 |
| 284 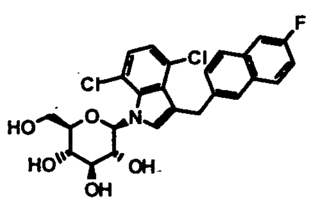 | 285 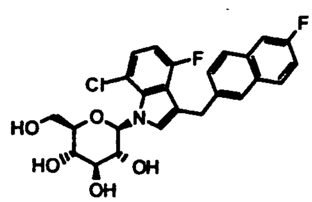 | 286 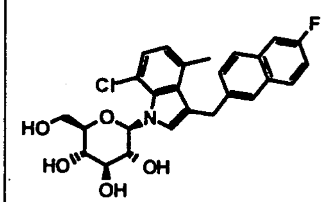 |
| 287 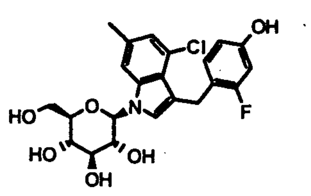 | 288 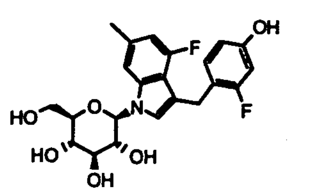 | 289 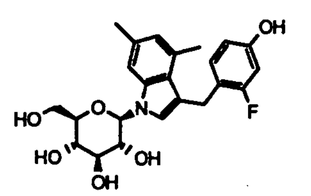 |

Table 4-12

| | | |
|---|---|---|
| 290 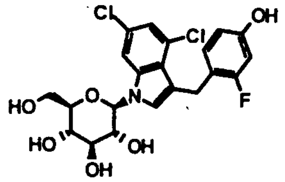 | 291 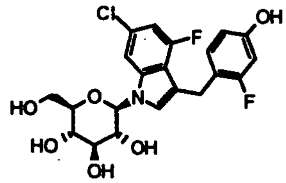 | 292 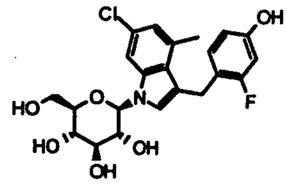 |
| 293 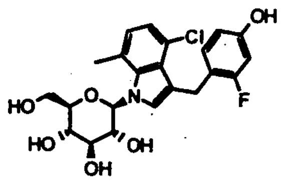 | 294 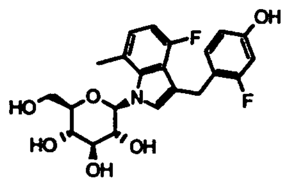 | 295 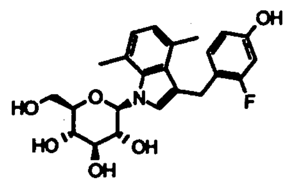 |
| 296 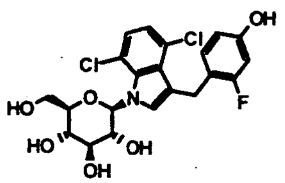 | 297 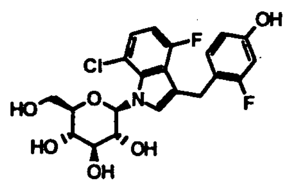 | 298 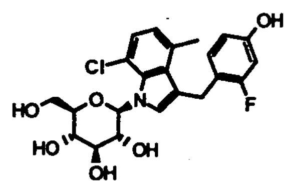 |
| 299 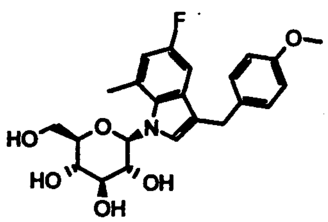 | 300 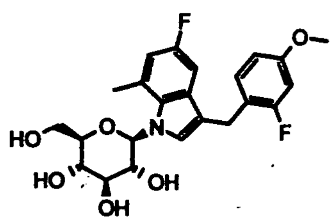 | 301 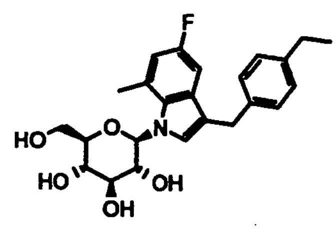 |
| 302 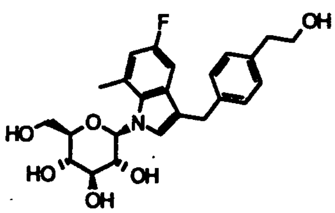 | 303 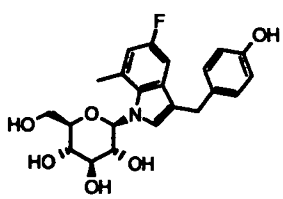 | 304 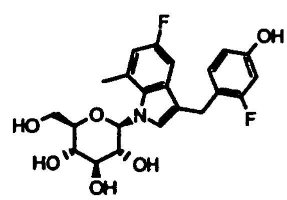 |
| 305 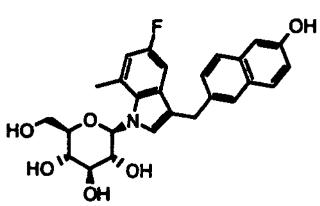 | 306 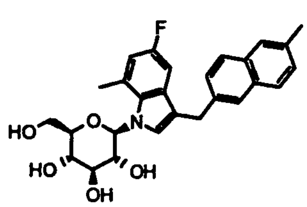 | 307 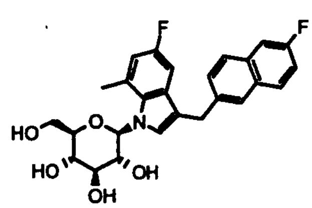 |

Table 4-13.

| | | |
|---|---|---|
| 308 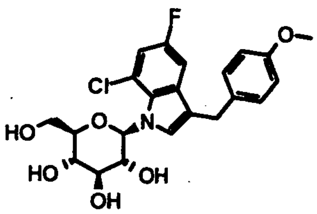 | 309 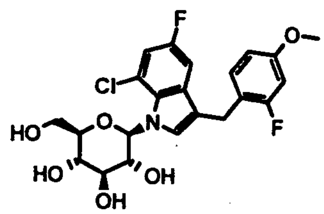 | 310 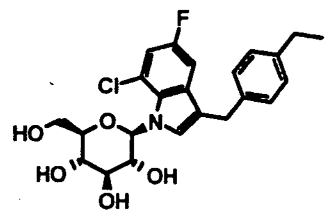 |
| 311 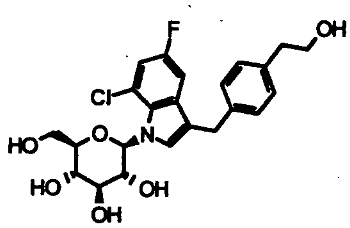 | 312 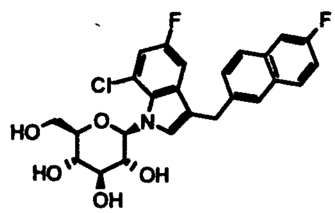 | 313 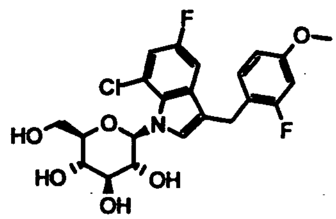 |
| 314 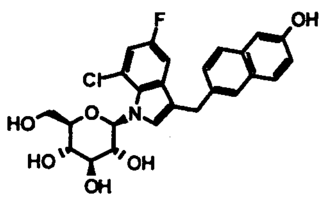 | 315 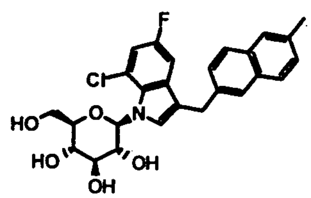 | 316 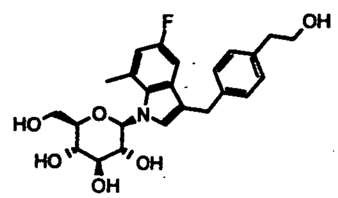 |

Example 317

3-[(4-{3-[2-(Carbamoyl)ethylamino]propoxy}phenyl)methyl]-1-(β-D-glucopyranosyl)-1H-indole

To a solution of the compound of Example 7 (0.1 g) in N,N-dimethylformamide (1 mL) were added sodium iodide (12 mg), 3-[N-benzyloxycarbonyl-N-(3-bromopropyl)amino]propionamide (refer to WO04018491 pamphlet)(0.18 g), potassium carbonate (0.11 g), and the resulting mixture was stirred at room temperature overnight. The insoluble material was removed by filtration, and the filtrate was diluted with water (1 mL) and methanol (1 mL). This solution was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the corresponding phenyl ether compound, 3-[(4-{3-[benzyloxycarbonyl-2-(carbamoylethyl)amino]propoxy}phenyl)methyl]-1-(β-D-glucopyranosyl) -1H-indole (59 mg) . To a solution of this phenyl ether compound (23 mg) in methanol (1 mL) was added 10% palladium-carbon powder (0.05 g), and the mixture was stirred under a hydrogen atmosphere overnight. The insoluble material was removed by filtration, and the solvent of the filtrate was removed under reduced pressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (5 mg).
$^1$H-NMR ($CD_3OD$) δ ppm:
1.9-2.0 (2H, m), 2.41 (2H, t, J=6. 9 Hz), 2.77 (2H, t, J=7.1 Hz), 2.84 (2H, t, J=7.0 Hz), 3.4-3.5 (1H, m), 3.5-3.65 (2H, m), 3.68 (1H, dd, J=12.2Hz, 5.8Hz), 3.8-3.95 (2H, m), 3.95-4.05 (4H, m), 5.41 (1H, d, J=9.1Hz), 6.75-6.85 (2H, m), 6.95-7.05 (1H, m), 7.1-7.2 (4H, m), 7.38 (1H, d, J=7.9Hz), 7.48 (1H, d, J=8.6Hz).

Example 318

3-[(4-{3-[2-(Carbamoyl)ethylamino]propoxy}phenyl)methyl]-1-(β-D-glucopyranosyl)-4-methyl-1H-indole

The title compound was prepared in a similar manner to that described in Example 317, using the compound

of Example 9 instead of the compound of Example 7.
$^{1}$H-NMR ($CD_3OD$) δ ppm:
1.95-2.05 (2H, m), 2.43 (3H, s), 2.47 (2H, t, J=6.9 Hz), 2.87 (2H, t, J=6.8 Hz), 2.93 (2H, t, J=6.8Hz), 3.4-3.5 (1H, m) , 3.5-3.6 (2H, m), 3.69 (1H, dd, J=12.1Hz, 5.8Hz), 3.8-3.9 (2H, m), 4.03 (2H, t, J=6.0Hz), 4.19 (2H, s), 5.4 (1H, d, J=9.1Hz), 6.72 (1H, d, J=7.2Hz), 6. 82 (2H, d, J=8. 4Hz) , 6.95-7.10 (4H, m) , 7.32 (1H, d, J=8.1Hz).

Example 319

1-(β-D-Glucopyranosyl)-3-[(4-{3-[(S)-2-hydroxy-1-(methyl)-ethylcarbamoyl]propyl}phenyl)methyl]-4-methyl-1H-indole

To a solution of 1-(2,3,4,6-tetra-*O*-benzyl-β-D-glucopyranosyl)-3-({4-[(1E)-3-carboxyprop-1-enyl]phenyl}-methyl)-4-methyl-1H-indole (50mg), (S)-2-amino-1-propanol(14 mg), 1-hydroxybenzotriazole (25 mg) and triethylamine (31 mg) in N,N-dimethylformamide (1 mL) was added 1-ethyl-3-(3-dimethylaminopropyl) carbodiimide hydrochloride (25 mg), and the resulting mixture was stirred at room temperature overnight. The reaction liquid was poured into water, and the resulting mixture was extracted with diethyl ether. The organic layer was washed with a saturated aqueous sodium hydrogen carbonate solution, water and brine successively, and dried over anhydrous magnesium sulfate. The solvent was removed under reduced pressure, and the residue was purified by column chromatography on silica gel (eluent: dichloromethane /methanol = 50/1-21/1) to give the corresponding amide compound, 1- (2, 3, 4, 6-tetra-*O*-benzyl-β-D-glucopyranosyl)-3-[(4-{(1E)-3-[(S)-2-hydroxy-1-(methyl)ethylcarbamoyl]prop-1-enyl}phenyl)methyl]-4-methyl-1H-indole (36 mg). To a solution of this amide compound (36 mg) in tetrahydrofuran (0.5 mL)/methanol (2 mL) was added 10% palladium-carbon powder (15 mg), and the mixture was stirred under a hydrogen atmosphere for 1.5 hours. The insoluble material was removed by filtration, and the solvent of the filtrate was removed under reduced pressure to give the title compound (20 mg).
$^{1}$H-NMR ($CD_3OD$) δ ppm:
1.11 (3H, d, J=5.9 Hz), 1.80-1.95 (2H, m), 2.19 (2H, t, J=7.4 Hz), 2.42 (3H, s), 2.59 (2H, t, J=7.4 Hz), 3.35-3.5 (3H, m), 3.5-3.6 (2H, m), 3.69 (1H, dd, J=12.1 Hz, 5.8 Hz), 3.80-3.95 (3H, m), 4.22 (2H, s), 5.40 (1H, d, J=9.1 Hz), 6.72 (1H, m), 6.95-7.05 (1H, m), 7.05-7.15 (5H, m), 7.3 (1H, d, J=8.5 Hz).

Examples 320 to 324

The compounds described in Table 5 were prepared in a similar manner to that described in Example 319, using the corresponding amine compounds instead of (S)-2-amino-1-propanol.
[Table 5]

| Example No. | Chemical structure | $^{1}$H-NMR ($CD_3OD$) δ ppm: |
|---|---|---|
| Ex.320 | 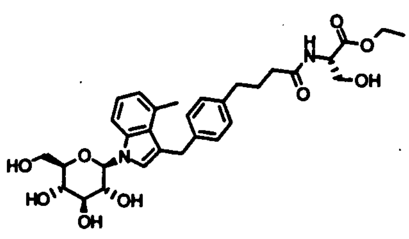 | 1.26 (3H, t, J=7.2 Hz), 1.85-2.0 (2H, m), 2.2-2.35 (2H, m), 2.43 (3H, s). 2.55-2.65 (2H, m), 3.4-3.5 (1H, m), 3.5-3.6 (2H, m), 3.69 (1H, dd, J=12.0 Hz, 5.8 Hz), 3.78 (1H, dd, J=11.5 Hz, 4.4 Hz), 3.8-3.95 (3H, m), 4.18 (2H, q, J=7.2 Hz), 4.22 (2H, s), 4.47 (1H, t, J=5.0 Hz), 5.40 (1H, d, J=9.1 Hz), 6.72 (1H, d, J=6.9 Hz), 6.95-7.05 (1H, m), 7.05-7.15 (5H, m), 7.33 (1H, d, J=8.0 Hz) |
| Ex.321 | 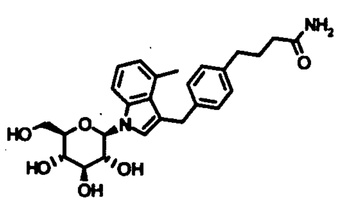 | 1.80-1.95 (2H, m), 2.20 (2H, t, J=7.6 Hz), 2.42 (3H, s), 2.60 (2H, t, J=7.6 Hz), 3.4-3.5 (1H, m), 3.5-3.6 (2H, m), 3.69 (1H, dd, J=12.8 Hz, 5.8 Hz), 3.80-3.95 (2H, m), 4.22 (2H, s), 5.40 (1H, d, J=9.1 Hz). 6.72 (1H, d. J=6.7 Hz). 6.95-7.05 (1H, m), 7.05-7.15 (5H, m), 7.33 (1H, d, J=8.1 Hz). |

(continued)

| Example No. | Chemical structure | $^1$H-NMR ($CD_3OD$) δ ppm: |
|---|---|---|
| Ex.322 | 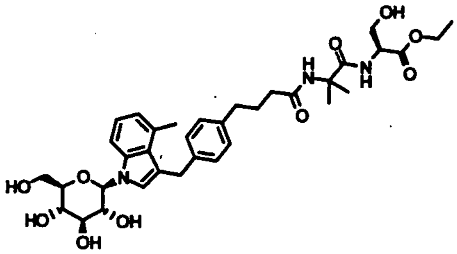 | 1.24 (3H, t, J=7.2 Hz), 1.45 (6H, d, J=2.7 Hz), 1.80-1.95 (2H, m), 2.22 (2H, t, J=7.6 Hz), 2.42 (3H. s), 2.60 (2H, t, J=7.8 Hz), 3.4-3.5 (1H, m), 3.5-3.6 (2H, m), 3.69 (1H, dd, J=11.9 Hz, 5.8 Hz), 3.79 (1H, dd, J=11.1 Hz, 4.0 Hz), 3.8-3.95 (3H, m), 4.17 (2H, q, J=7.2 Hz), 4.22 (2H, s), 4.43 (1H, t, J=4.2 Hz), 5.40 (1H, d, J=9.2 Hz). 6.72 (1H. d, J=7.5 Hz). 6.95-7.05 (1H, m), 7.05-7.15 (5H, m), 7.33 (1H, d, J=8.2 Hz) |
| Ex.323 | 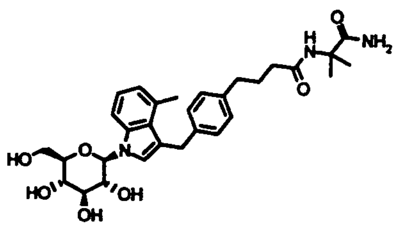 | 1.44 (6H, s), 1.80-1.95 (2H, m), 2.20 (2H, t, J=7.6 Hz), 2.42 (3H, s), 2.59 (2H, t, J=7.7 Hz), 3.4-3.5 (1H, m), 3.5-3.6 (2H, m), 3.69 (1H, dd, J=12.3 Hz, 5.8 Hz), 3.8-3.90 (2H, m), 4.21 (2H, s), 5.40 (1H, d, J=9.2 Hz). 6.72 (1H, d, J=6.9 Hz), 6.95-7.05 (1H, m), 7.05-7.15 (5H, m), 7.33 (1H, d, J=8.5 Hz) |
| Ex.324 | 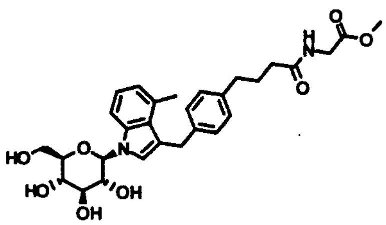 | 1.85-1.95 (2H, m), 2.25 (2H, t, J=7.4 Hz), 2.42 (3H, s), 2.61 (2H, t, J=7.4 Hz), 3.4-3.5 (1H, m), 3.5-3.6 (2H, m), 3.65-3.7 (4H, m), 3.8-3.9 (4H, m), 4.22 (2H, s), 5.40 (1H, d, J=9.1 Hz), 6.72 (1H, d, J=7.3 Hz), 6.95-7.05 (1H, m), 7.05-7.15 (5H, m), 7.33 (1H, d, J=8.4 Hz) |

Example 325

1-(β-D-Glucopyranosyl)-3-(4-{[3-(methoxycarbonyl)propyl]-phenyl}methyl)-4-methyl-1H-indole

To a suspension of 1-(2,3,4,6-tetra-*O*-benzyl-β-D-glucopyranosyl)-3-({4-[(1E)-3-carboxyprop-1-enyl]phenyl} methyl)-4-methyl-1H-indole (30 mg) and potassium carbonate (10 mg) in N,N-dimethylformamide (1 mL) was added methyl iodide (8 mg), and the resulting mixture was stirred at room temperature for 4 hours. The reaction liquid was poured into water, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: n-hexane/ethyl acetate = 5/1) to give the corresponding ester compound, 1-(2,3,4,6-tetra-*O*-benzyl-β-D-glucopyranosyl)-3-({4-[(1E)-3-(methoxyca rbonyl)prop-1-enyl]phenyl}methyl)-4-methyl-1H-indole (9 mg). To a solution of this ester compound (9 mg) in tetrahydrofuran (0.25 mL)/methanol (1 mL) was added 10% palladium-carbon powder (10 mg), and the mixture was stirred under a hydrogen atmosphere for 1.5 hours. The insoluble material was removed by filtration, and the solvent of the filtrate was removed under reduced pressure to give the title compound (4 mg).
$^1$H-NMR ($CD_3OD$) δ ppm:
1.80-1.95 (2H, m), 2.30 (2H, t, J=7.0 Hz), 2.42 (3H, s), 2.59 (2H, t, J=7.5 Hz), 3.4-3.5 (1H, m), 3.5-3.6 (2H, m), 3.62 (3H, s), 3.69 (1H, dd, J=12.3 Hz, 5.8 Hz), 3.80-3.95 (2H, m), 4.22 (2H, s), 5.40 (1H, d, J=9.2 Hz), 6.72 (1H, d, J=7.5 Hz), 6.95-7.05 (1H, m), 7.00-7.15 (5H, m), 7.33 (1H, d, J=8.0 Hz).

Example 326

1-(β-D-Glucopyranosyl)-3-({4-[2-(methoxycarbonyl)ethyl]-phenyl}methyl)-4-methyl-1H-indole

A suspension of 1-(2,3,4,6-tetra-*O*-benzyl-β-D-glucopyranosyl)-3-(4-bromobenzyl)-4-methyl-1H-indole (0.107 g), methyl acrylate (26 mg), palladium (II) acetate (3 mg), tris (2-methylphenyl) phosphine (8 mg) and triethylamine (66 mg) in acetonitrile (1.5 mL) was heated under reflux for 6 hours. The reaction mixture was diluted with ethyl acetate and the insoluble material was removed by filtration on celite. The filtrate was poured into 1M hydrochloric acid, and the resulting

mixture was extracted with diethyl ether. The organic layer was washed with water and brine successively, and dried over anhydrous magnesium sulfate. The solvent was removed under reduced pressure, and the residue was purified by column chromatography on silica gel (eluent: n-hexane/ethyl acetate = 8/1-5/1) to give 1-(2,3,4,6-tetra-*O*-benzyl-β-D-glucopyranosyl)-3-({4-[(1E)-2-(methoxycarbonyl)vinyl]phenyl}-methyl)-4-methyl-1H-indole (82 mg). To a solution of the obtained 1-(2,3,4,6-tetra-*O*-benzyl-β-D-glucopyranosyl)-3-({4-[(1E)-2-(methoxycarbonyl)vinyl]phenyl}methyl)-4-methyl-1H-indole (80 mg) in tetrahydrofuran (0.5 mL)/methanol (2 mL) was added 10% palladium-carbon powder (40 mg), and the mixture was stirred under a hydrogen atmosphere for 2 hours. The insoluble material was removed by filtration, and the solvent of the filtrate was removed under reduced pressure to give the title compound (45 mg).
$^1$H-NMR ($CD_3OD$) δ ppm:
2.41 (3H, s), 2.60 (2H, t, J=7.5 Hz), 2.87 (2H, t, J=7.5 Hz), 3.4-3.5 (1H, m), 3.5-3.6 (2H, m), 3.62 (3H, s), 3.69 (1H, dd, J=12.3 Hz, 6.1 Hz), 3.80-3.95 (2H, m), 4.22 (2H, s), 5.40 (1H, d, J=9.1Hz), 6.72 (1H, d, J=7.0Hz), 6.95-7.05 (1H, m), 7.05-7.15 (5H, m), 7.33 (1H, d, J=8.0 Hz).

Example 327

<u>3-{[4-(Ethoxycarbonylmethoxy)phenyl]methyl}-1-(β-D-glucopyranosyl)-4-methyl-1H-indole</u>

To a suspension of the compound of Example 9 (21 mg) and cesium carbonate (51 mg) in *N,N*-dimethylformamide (1 mL) were added a catalytic amount of sodium iodide and ethyl bromoacetate (26 mg), and the resultingmixturewas stirred at roomtemperature for 6 hours. The reaction mixture was diluted with dichloromethane/tetrahydrofuran (50/1), and the resulting mixture was purified by column chromatography on silica gel (eluent: n-hexane/ethyl acetate = 5/1) to give the title compound (13 mg).
$^1$H-NMR ($CD_3OD$) δ ppm:
1.26 (3H, t, J=7.1 Hz), 2.42 (3H, s), 3.4-3.5 (1H, m), 3.5-3.6 (2H, m), 3.69 (1H, dd, J=12.3 Hz, 5.9 Hz), 3.8-3.9 (2H, m), 4.15-4.25 (4H, m), 4.64 (2H, s), 5.40 (1H, d, J=9.1 Hz), 6.72 (1H, d, J=6.8 Hz) , 6.75-6.85 (2H, m) , 6.95-7.05 (1H, m) , 7.05-7.15 (3H, m), 7.33 (1H, d, J=8.4 Hz).

Example 328

<u>3-{4-[(3-Carboxypropyl)phenyl]methyl}-1-(β-D-glucopyranosyl)-4-methyl-1H-indole</u>

To a solution of 1-(2,3,4,6-tetra-*O*-benzyl-β-D-glucopyranosyl)-3-({4-[(1E)-3-carboxyprop-1-enyl]phenyl}methyl)-4-methyl-1H-indole (42 mg) in tetrahydrofuran (1 mL)/methanol (2 mL) was added 10% palladium-carbon powder (20 mg), and the mixture was stirred under a hydrogen atmosphere for 1.5 hours. The insoluble material was removed by filtration, and the solvent of the filtrate was removed under reduced pressure to give the title compound (21 mg).
$^1$H-NMR ($CD_3OD$) δ ppm:
1.80-1.95 (2H, m), 2.26 (2H, t, J=7.0 Hz), 2.42 (3H, s), 2.60 (2H, t, J=7.2 Hz), 3.4-3.5 (1H, m), 3.5-3.6 (2H, m), 3.69 (1H, dd, J=12.2 Hz, 5.8 Hz), 3.80-3.95 (2H, m), 4.22 (2H, s), 5.40 (1H, d, J=8.9 Hz), 6.73 (1H, d, J=7.4 Hz), 6.95-7.05 (1H, m), 7.00-7.15 (5H, m), 7.32 (1H, d, J=8.6 Hz).

Example 329

<u>1-(β-D-Glucopyranosyl)-3-(4-[3-{1-(piperazin-1-yl-carbonyl)-1-(methyl)ethylcarbamoyl}propyl]phenyl)methyl-4-methyl-1H-indole</u>

A solution of 3-{4-[(3-carboxypropyl)phenyl]methyl}-1-(β-D-glucopyranosyl)-4-methyl-1H-indole (21 mg), 1-(2-amino-2-methylpropionyl)-4-(benzyloxycarbonyl)piperazine (refer to WO0414932 pamphlet) (41 mg), 1-hydroxybenzotriazole (18 mg) and triethylamine (23 g) in N,N-dimethylformamide (1 mL) was added 1-ethyl-3-(3-dimethylaminopropyl) carbodiimide hydrochloride (26mg), and the resulting mixture was stirred at room temperature for 6 hours. The reaction liquid was purified directly by column chromatography on silica gel (eluent: dichloromethane/methanol = 50/1-10/1) to give 3-[(4-{3-[1-{[4-(benzyloxycarbonyl)-piperazin-1-yl]carbonyl}-1-(methyl)ethylcarbamoyl]-propyl}phenyl)methyl-1-(β-D-glucopyranosyl)-4-methyl-1H-indole (24 mg). To a solution of the obtained 3-[(4-{3-[1-{[4-(benzyloxycarbonyl)piperazin-1-yl]carbonyl}-1-(methyl)-ethylcarbamoyl]propyl}phenyl)methyl-1-(β-D-glucopyranosyl)-4-methyl-1H-indole (23 mg) in tetrahydrofuran (1 mL) /methanol (2 mL) was added 10% palladium-carbon powder (20 mg), and the mixture was stirred under a hydrogen atmosphere for 1.5 hours. The insoluble material was removed by filtration, and the solvent of the filtrate was removed under reduced pressure, and the residue was purified by solid phase extraction on ODS (washing solvent: distilled water and a saturated aqueous sodium hydrogen carbonate solution, eluent: methanol) to give the title compound (12 mg).

$^1$H-NMR ($CD_3OD$) δ ppm:
1.42 (6H, s), 1.80-1.95 (2H, m), 2.17 (2H, t, J=7.6 Hz), 2.43 (3H, s), 2.60 (2H, t, J=7.5 Hz), 2.65-2.75 (4H, brs), 3.4-3.5 (1H, m), 3.5-3.65 (6H, m), 3.69 (1H, dd, J=12.1 Hz, 5.8 Hz), 3.8-3.9 (2H, m), 4.22 (2H, s), 5.40 (1H, d, J=9.1 Hz), 6.72 (1H, d, J=7.4 Hz), 6.95-7.05 (1H, m), 7.05-7.15 (5H, m), 7.33 (1H, d, J=8.5 Hz).

Example 330

1-(β-D-Glucopyranosyl)-3-([4-{2-(2-hydroxyethoxycarbonyl)-ethyl}phenyl]methyl)-4-methyl-1H-indole

[Chem.22]

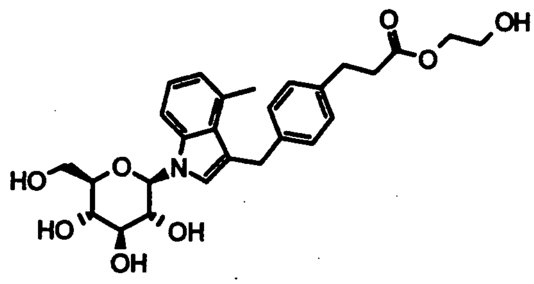

To a suspension of a carboxylic acid compound, 1-(2,3,4,6-tetra-*O*-benzyl-β-D-glucopyranosyl)-3-([4-{(1E)-2-carboxyvinyl}phenyl]methyl)-4-methyl-1H-indole (26 mg) and potassium carbonate (13 mg) in N, N-dimethylformamide (1 mL) were added a halide compound (benzyl 2-bromoethyl ether) (14 mg), and the result ing mixture was stirred at room temperature for 3 hours. The reaction mixture was diluted with water, and the resulting mixture was extracted with diethyl ether. The organic layer was washed with water and brine successively, and dried over anhydrous magnesium sulfate. The solvent was removed under reduced pressure, and the residue was purified by column chromatography on silica gel (eluent: n-hexane/ethyl acetate = 4/1) to give the corresponding ester compound, 1-(2,3,4,6-tetra-*O*-benzyl-β-D-glucopyranosyl)-3-([4-{2-(2-hydroxy-ethoxycarbonyl)ethyl}phenyl]methyl)-4-methyl-1H-indole (25 mg) .
To a solution of this ester compound (25 mg) in tetrahydrofuran (1 mL) /methanol (1 mL) was added 10% palladium-carbon powder (10 mg), and the mixture was stirred under a hydrogen atmosphere for 2 hours. The insoluble material was removed by filtration, and the solvent of the filtrate was removed under reduced pressure to give the title compound (11 mg).
$^1$H-NMR ($CD_3OD$) δ ppm:
2.41 (3H, s), 2.64 (2H, t, J=7.7 Hz), 2.89 (2H, t, J=7.7 Hz), 3.40-3.75 (6H, m), 3.80-3.95 (2H, m), 4.00-4.15 (2H, m), 4.22 (2H, s), 5.40 (1H, d, J=9.1Hz), 6.72 (1H, d, J=7.0Hz), 6.95-7.15 (6H, m), 7.33 (1H, d, J=8.2 Hz).

Examples 331 to 333

The compounds described in Table 6 were prepared in a similar manner to that described in Example 330, using the corresponding carboxylic acid compounds and halide compounds instead of 1-(2,3,4,6-tetra-*O*-benzyl-β-D-glucopyranosyl)-3-([4-{(1E)-2-carboxyvinyl}phenyl]methyl)-4-methyl-1H-indole and benzyl 2-bromoethyl ether.
[Table 6]

| Example No. | Chemical structure | $^1$H-NMR ($CD_3OD$) δ ppm: |
|---|---|---|
| Ex.331 | 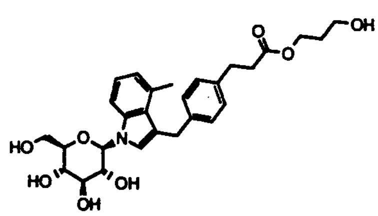 | 1.70-1.85 (2H, m), 2.41 (3H, s), 2.60 (2H, t, J=7.5 Hz), 2.88 (2H, t, J=7.5 Hz), 3.40-3.75 (6H, m), 3.80-3.95 (2H, m), 4.00-4.15 (2H, m), 4.22 (2H, s), 5.40 (1H, d, J=8.8 Hz), 6.72 (1H, d, J=6.9 Hz), 6.95-7.15 (6H, m), 7.33 (1H, d, J=8.4 Hz) |

(continued)

| Example No. | Chemical structure | $^1$H-NMR ($CD_3OD$) δ ppm: |
|---|---|---|
| Ex.332 | 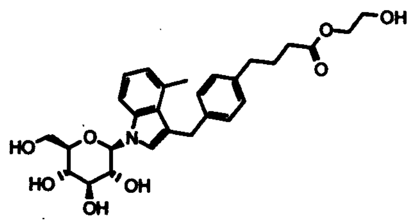 | 1.85-1.95 (2H, m), 2.34 (2H, t, J=7.4 Hz), 2.42 (3H, s), 2.61 (2H, t, J=7.4 Hz), 3.40-3.50 (1 H, m), 3.50-3.65 (2H, m), 3.65-3.75 (3H, m), 3.80-3.90 (2H, m), 4.05-4.15 (2H, m), 4.22 (2H, s), 5.40 (1 H, d, J=9.1 Hz), 6.72 (1 H, d, J=7.4 Hz), 6.95-7.05 (1H, m), 7.05-7.15 (5H, m), 7.33 (1H, d, J=8.1 Hz) |
| Ex.333 | 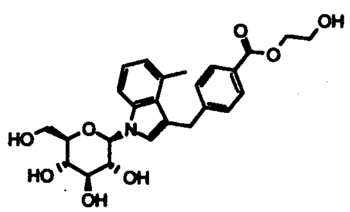 | 2.38 (3H, s), 3.40-3.50 (1H, m), 3.50-3.65 (2H, m), 3.69 (1H, dd, J=12.4 Hz, 5.9 Hz), 3.80-3.95 (4H, m), 4.30-4.40 (4H, m), 5.42 (1 H, d, J=9.2 Hz), 6.73 (1 H, d, J=7.0 Hz), 6.95-7.05 (1H, m), 7.16 (1H, s), 7.30 (2H, d, J=8.5 Hz), 7.35 (1H, d, J=8.5 Hz), 7.98 (2H, d, J=8.5 Hz). |

Example 334

3-([4-{3-((S)-2,3-Dihydroxypropoxycarbonyl)propyl}phenyl]-methyl)-1-(β-D-glucopyranosyl)-4-methyl-1H-indole

[Chem.23]

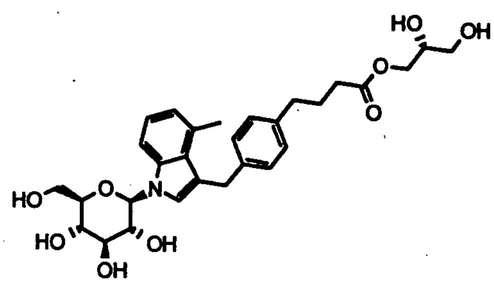

To a solution of 1-(2,3,4,6-tetra-*O*-benzyl-β-D-glucopyranosyl)-3-({4-[(1E)-3-carboxyprop-1-enyl]phenyl}methyl)-4-methyl-1H-indole (150 mg), dicyclohexylcarbodiimide (56 mg) and 4-dimethylamino pyridine (11 mg) in dichloromethane (2 mL) was added (S)-2,2-dimethyl-1,3-dioxolane-4-mehtanol (29 mg), and the resulting mixture was stirred at room temperature. The reaction mixture was purified directly by column chromatography on silica gel (eluent: *n*-hexane/ethyl acetate = 2/1) to give 1-(2,3,4,6-tetra-*O*-benzyl-β-D-glucopyranosyl)-3-([4-{(1E)-3-[{(S)-2,2-dimethyl-1,3-dioxolane-4-yl} methoxycarbonyl]-prop-1-enyl}phenyl]methyl)-4-methyl-1H-indole (105 mg).
To a solution of 1- (2, 3, 4, 6-tetra-*O*-benzyl-β-D-glucopyranosyl)-3-([4-{(1E)-3-[{(S)-2,2-dimethyl-1,3-dioxolane-4-yl} methoxycarbonyl]prop-1-enyl}phenyl]methyl)-4-methyl-1H-indole (85 mg) in dichloromethane/methanol (= 1/3, 1.8 mL) was added Amberlyst ® 15 ion-exchange resin (150 mg), and the resulting mixture was stirred at 50˚C for 4 hours. The insoluble material was removed by filtration, and the solvent of the filtrate was removed under reduced pressure, and the residue was purified by column chromatography on silica gel (eluent: dichloromethane/methanol = 20/1) to give 1-(2,3,4,6-tetra-O-benzyl-β-D-glucopyranosyl)-3-{[4-[(1E)-3-{(S)-2,3-dihydro xypropoxycarbonyl}prop-1-enyl]phenyl] methyl}-4-methyl-1H-indole (62 mg).
To a solution of 1-(2,3,4,6-tetra-*O*-benzyl-β-D-glucopyranosyl)-3-{[4-[(1E)-3-{(S)-2,3-dihydroxypropoxy-carbonyl}prop-1-enyl]phenyl]methyl}-4-methyl-1H-indole (60 mg) in tetrahydrofuran (1 mL) /methanol (3 mL) was added 10% palladium-carbon powder (30 mg), and the mixture was stirred under a hydrogen atmosphere for 2 hours. The insoluble material was removed by filtration, and the solvent of the filtrate was removed under reduced pressure to give the title compound (36 mg).
$^1$H-NMR ($CD_3OD$) δ ppm:

1.85-1.95 (2H, m), 2.34 (2H, t, J=7.4 Hz), 2.42 (3H, s), 2.61 (2H, t, J=7.4 Hz), 3.40-3.60 (5H, m), 3.69 (1H, dd, J=12.1 Hz, 5.8 Hz), 3.75-3.95 (3H, m), 4.03 (1H, dd, J=11.3 Hz, 6.3 Hz), 4.12 (1H, dd, J=11.3 Hz, 4.2 Hz), 4.22 (2H, s), 5.40 (1H, d, J=9.3 Hz), 6.72 (1H, d, J=7.2 Hz), 6.95-7.15 (6H, m), 7.33 (1H, d, J=8.0 Hz).

Example 335

3-([4-{3-((R)-2,3-Dihydroxypropoxycarbonyl)propyl}phenyl]-methyl)-1-(β-D-glucopyranosyl)-4-methyl-1H-indole

[Chem.24]

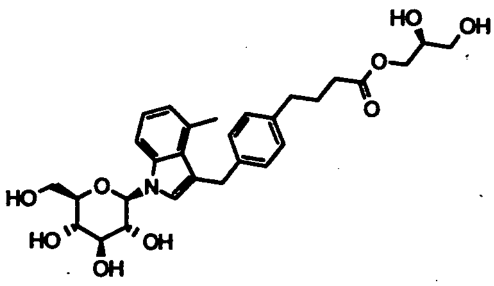

The title compound was prepared in a similar manner to that described in Example 334, using (R)-2,2-dimethyl-1,3-dioxolane-4-mehtanol instead of (S)-2,2-dimethyl-1,3-dioxolane-4-mehtanol.
$^1$H-NMR ($CD_3OD$) δ ppm:
1.85-1.95 (2H, m), 2.34 (2H, t, J=7.4 Hz), 2.42 (3H, s), 2.61 (2H, t, J=7.4 Hz), 3.40-3.60 (5H, m), 3.69 (1H, dd, J=11.9 Hz, 5.2 Hz), 3.75-3.95 (3H, m), 4.03 (1H, dd, J=11.3 Hz, 6.3 Hz), 4.12 (1H, dd, J=11.3 Hz, 4.3 Hz), 4.22 (2H, s), 5.40 (1H, d, J=8.9 Hz), 6.72 (1H, d, J=7.2 Hz), 6.95-7.15 (6H, m), 7.33 (1H, d, J=8.1 Hz).

Example 336

1-(β-D-Glucopyranosyl)-3-[{4-(2-hydroxy-1-hydroxymethyl-ethoxycarbonyl)phenyl}methyl]-4-methyl-1H-indole

[Chem.25]

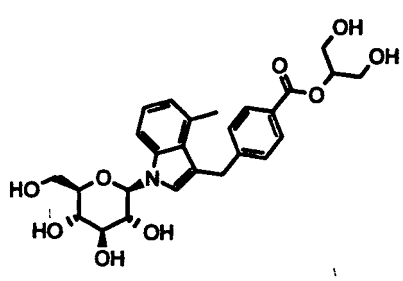

A mixture of 1-(2,3,4,6-tetra-*O*-benzyl-β-D-glucopyranosyl)-3-([4-{(1E)-2-carboxyvinyl}phenyl]methyl)-4-methyl-1H-indole (25 mg), 1,3-benzylideneglycerol (7 mg), triphenylphosphine (9 mg) and diethyl azodicarboxylate (40% toluene solution, 0.02 mL) in tetrahydrofuran (0.1 mL) was stirred at room temperature for 2 hours. The reaction mixture was purified directly by column chromatography on silica gel (eluent: *n*-hexane/ethyl acetate = 5/1) to give 1- (2, 3, 4, 6-tetra-O-benzyl-β-D-glucopyranosyl)-3-{4-(2-phenyl-1,3-dioxolane-5-yloxycarbonyl)phenyl}methyl-4-methyl-1H-indole (17 mg).
To a solution of 1-(2,3,4,6-tetra-*O*-benzyl-β-D-glucopyranosyl)-3-{4-(2-phenyl-1,3-dioxolane-5-yloxycarbonyl)-phenyl}methyl-4-methyl-1H-indole (17 mg) in tetrahydrofuran (0.5 mL) /methanol (1 mL) was added 10% palladium-carbon

powder (13 mg), and the mixture was stirred under a hydrogen atmosphere overnight. The insoluble material was removed by filtration, and the solvent of the filtrate was removed under reduced pressure to give the title compound (9 mg).
$^1$H-NMR ($CD_3OD$) δ ppm:
2.37 (3H, s), 3.40-3.50 (1H, m), 3.50-3.65 (2H, m), 3.70 (1H, dd, J=12.1Hz, 5.6Hz), 3.75-3.95 (6H, m), 4.34 (2H, s), 5.05-5.15 (1H, m), 5.42 (1H, d, J=9.0Hz), 6.73 (1H, d, J=6.8Hz), 6.95-7.05 (1H, m), 7.15 (1H, s), 7.29 (2H, d, J=8.5Hz), 7.35 (1H, d, J=8.3 Hz), 7.97 (2H, d, J=8.5 Hz).

Example 337

1-(β-D-Glucopyranosyl)-3-[4-{3-(2-hydroxy-1-hydroxymethyl-ethoxycarbonyl)propyl}phenyl]methyl-4-methyl-1H-indole

[Chem. 26]

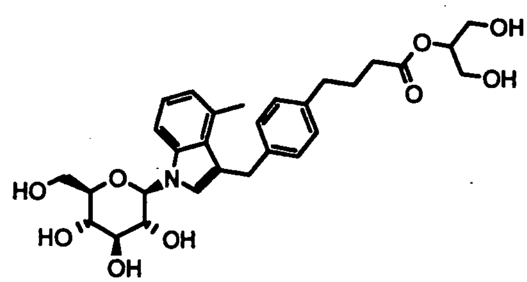

The title compound was prepared in a similar manner to that described in Example 336, using 1-(2, 3, 4, 6-tetra-*O*-benzyl-β-D-glucopyranosyl)-3-({4-[(1E)-3-carboxyprop-1-enyl]phenyl }methyl)-4-methyl-1H-indole and diisopropyl azodicarboxylate instead of 1-(2,3,4,6-Tetra-*O*-benzyl-β-D-glucopyranosyl)-3-([4-{(1E)-2-carboxyvinyl}phenyl] methyl)-4-methyl-1H-indole and diethyl azodicarboxylate.
$^1$H-NMR ($CD_3OD$) δ ppm:
1.85-1.95 (2H, m), 2.36 (2H, t, J=7.4 Hz), 2.42 (3H, s), 2.62 (2H, t, J=7. 4 Hz), 3.40-3.50 (1H, m), 3.50-3.70 (7H, m), 3. 80-3. 95 (2H, m), 4.22 (2H, s), 4.80-4.95 (1H, m), 5.40 (1H, d, J=9.1 Hz), 6.72 (1H, d, J=7.0 Hz), 6.95-7.05 (1H, m), 7.05-7.15 (5H, m), 7.33 (1H, d, J=8.3 Hz.

Example 338

1-(β-D-Glucopyranosyl)-3-(4-[3-{1-(4-isopropylpiperazin-1-ylcarbonyl)-1-(methyl)ethylcarbamoyl}propyl]phenyl)methyl-4-methyl-1H-indole

[Chem. 27]

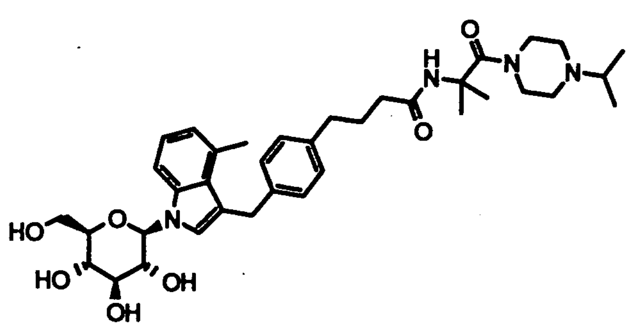

To a solution of 1-(β-D-Glucopyranosyl)-3-(4-[3-{1-(piperazin-1-ylcarbonyl)-1-(methyl)ethylcarbamoyl}-4-methyl-1H-indole (10 mg) and N,N-diisopropylethylamine (10 mg) in acetonitrile (1 mL)/methanol (1 mL) was added 2-iodopropane (14 mg), and the resulting mixture was stirred at 60°C. The reaction mixture was purified directly by column chromatography on silica gel (eluent: dichloromethane/ methanol = 5/1-2/1) to give the title compound (11 mg).
$^1$H-NMR ($CD_3OD$) δ ppm:
1.02 (6H, d, J=6.4 Hz), 1.42 (6H, s), 1.80-1.95 (2H, m), 2.18 (2H, t, J=7.3 Hz), 2.41 (3H, s), 2.48 (4H, brs), 2.55-2.70 (3H, m), 3.40-3.50 (1H, m), 3.50-3.75 (7H, m), 3.80-3.95 (2H, m), 4.22 (2H, s), 5.41 (1H, d, J=9.1 Hz), 6.72 (1H, d, J=7.2 Hz), 6.95-7.05 (1H, m), 7.05-7.15 (5H, m), 7.33 (1H, d, J=8.0 Hz).

Example 339

<u>1-(β-D-Glucopyranosyl)-3-(4-[3-{1-(4-isobutylpiperazin-1-ylcarbonyl)-1-(methyl)ethylcarbamoyl}propyl]phenyl)-methyl-4-methyl-1H-indole</u>

[Chem.28]

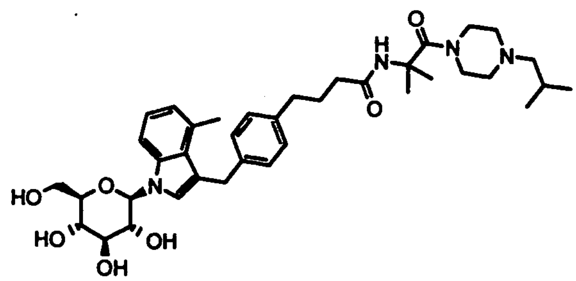

The title compound was prepared in a similar manner to that described in Example 338, using 1-iodo-2-methylpropane instead of 2-iodopropane.
$^1$H-NMR ($CD_3OD$) δ ppm:
0.89 (6H, d, J=6.5 Hz), 1.42 (6H, s), 1.70-1.95 (3H, m), 2.09 (2H, d, J=7. 3 Hz) , 2.17 (2H, t, J=7. 4 Hz) , 2.36 (4H, brs), 2.41 (3H, s), 2.60 (2H, t, J=7.4 Hz), 3.40-3.50 (1H, m), 3.50-3.75 (7H, m), 3.80-3.95 (2H, m), 4.22 (2H, s), 5.41 (1H, d, J=9.1 Hz), 6.72 (1H, d, J=7.0 Hz), 6.95-7.05 (1H, m), 7.05-7.15 (5H, m), 7.33 (1H, d, J=8.5 Hz).

Example 340

<u>3-(4-Carboxyphenyl)methyl-1-(β-D-glucopyranosyl)-4-methyl-1H-indole</u>

[Chem.29]

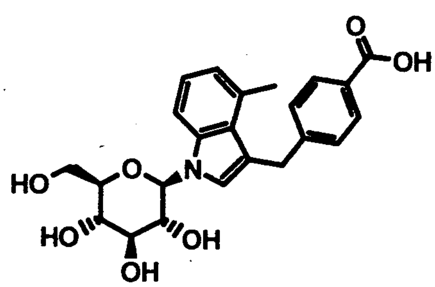

The title compound was prepared in a similar manner to that described in Example 328, using 1- (2, 3, 4, 6-tetra-*O*-benzyl-β-D-glucopyranosyl)-3-(4-carboxyphenyl)methyl-4-methyl-1H-indole instead of 1-(2,3,4,6-tetra-*O*-benzyl-β-D-glucopyranosyl)-3-({4-[(1E)-3-carboxyprop-1-enyl]phenyl}methyl)-4-methyl-1H-indole.
$^{1}$H-NMR ($CD_3OD$) δ ppm:
2.39 (3H, s), 3.40-3.50 (1H, m), 3.50-3.65 (2H, m), 3.70 (1H, dd, J=12.1 Hz, 5.6 Hz), 3.80-3.95 (2H, m), 4.33 (2H, s), 5.42 (1H, d, J=9.2 Hz), 6.74 (1H, d, J=7.8 Hz), 6.95-7.05 (1H, m), 7.14 (1H, s), 7.28 (2H, d, J=8.2 Hz), 7.35 (1H, d, J=7.8 Hz), 7.90 (2H, d, J=8.2 Hz).

Example 341

1-(β-D-Glucopyranosyl)-3-{4-(2-carboxyethyl)phenyl}methyl-4-methyl-1H-indole

[Chem.30]

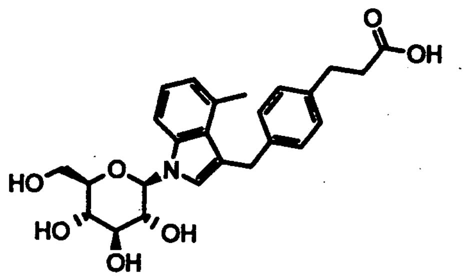

To a solution of the compound of the Example 326 was added 1M aqueous sodium hydroxide solution (0.23 mL), and the resulting mixture was stirred at 40°C overnight. To the reaction mixture was added 1M hydrochloric acid (0.25 mL), and the resulting mixture was concentrated under reduced pressure. The residue was purified by solid phase extraction on ODS (washing solvent: distilled water, eluent: methanol) to give the title compound (12 mg).
$^{1}$H-NMR ($CD_3OD$) δ ppm:
2.42 (3H, s), 2.55 (2H, t, J=7.6 Hz), 2.87 (2H, t, J=7.6 Hz), 3.40-3.50 (1H, m), 3.50-3.65 (2H, m), 3.69 (1H, dd, J=12.3 Hz, 5.8 Hz), 3.80-3.95 (2H, m), 4.22 (2H, s), 5.40 (1H, d, J=9.2 Hz), 6.72 (1H, d, J=7.4 Hz), 6.95-7.15 (6H, m), 7.33 (1H, d, J=8.0 Hz).

Example 342

3-(4-Methoxybenzyl)-1-(β-D-glucopyranosyl)-7-azaindole

[Chem.31]

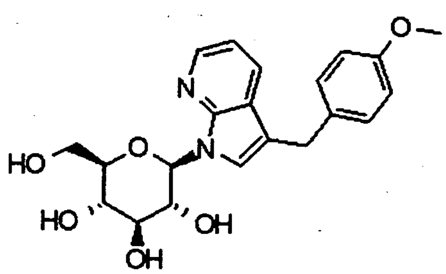

To a solution of 3- (4-methoxybenzoyl) -7-azaindole (0.12 g) in N, N-dimethylformamide (8 mL) was added 55% sodium hydride (27 mg) under ice-cooling, and the resulting mixture was stirred for 1 hour. To the reaction mixture was added 2,3,4,6,-tetra-*O*-benzyl-α-D-glucopyranosylchloride (306 mg) at the same temperature, and the mixture was stirred at room temperature overnight. To the reaction mixture was added ice-water, and the resulting mixture was extracted with ethyl acetate. The organic layer was washed with water and brine successively, and dried over anhydrous magnesium

sulfate. The solvent was removed under reduced pressure, and the residue was purified'by column chromatography on silica gel (eluent: *n*-hexane/ethyl acetate =3/1-1/1) to give the glycoside compound, 1-(2,3,4,6-tetra-*O*-benzyl-β-D-glucopyranosyl)-3-(4-methoxybenzoyl)-7-azaindole (215 mg). To a solution of this glycoside compound (315 mg) in tetrahydrofuran (4 mL) was added lithium aluminium hydride (1M tetrahydrofuran solution, 2.0 mL) at room temperature, and the mixture was stirred at same temperature for 30 minutes. To the reaction liquid was added dropwise water under ice-cooling, and the resulting mixture was extracted with ethyl acetate. The organic layer was washed with brine, and dried over anhydrous magnesium sulfate. The solvent was removed under reduced pressure, and the residue was purified by column chromatography on silica gel (eluent: n-hexane/ethyl acetate = 2/1) to give the alcohol compound 1-(2,3,4,6-tetra-*O*-benzyl-β-D-glucopyranosyl)-3-[hydroxy-(4-methoxyphenyl)-methyl]-7-azaindole (295 mg). To a solution of this alcohol compound (0.28 g) in dichloromethane (4 mL) were added dropwise triethylsilane (0.21 g)and boron trifluoride diethyl ether complex (0.26 g) successively under ice-cooling in a methanol bath, and the resulting mixture was stirred for 1 hour. To the reaction mixture was added a saturated aqueous potassium carbonate solution, and the resulting mixture was stirred for 10 minutes. The mixture was poured into water, and the resulting mixture was extracted with ethyl acetate. The organic layer was washed with water and brine successively, and dried over anhydrous magnesium sulfate. The solvent was removed under reduced pressure, and the residue was purified by column chromatography on silica gel (eluent: n-hexane/ethyl acetate - 4/1) to give 1-(2,3,4,6-tetra-*O*-benzyl-β-D-glucopyranosyl)-3-(4-methoxybenzyl)- 7-azaindole (0.24 g). $^1$H-NMR ($CDCl_3$) δ ppm: 3.7-3.95 (10H, m), 4.01 (2H, s), 4.30 (1H, d, J=10.6 Hz), 4.49 (1H, d, J=12.4 Hz), 4.59 (1H, d, J=12.4 Hz), 4.63 (1H, d, J=10.6 Hz), 4.8-4.95 (3H, m), 6.05 (1H,brs), 6.57(2H, d, J=7.4 Hz), 6.7-6.8 (2H, m) , 6.95-7.15 (7H, m) , 7.15-7.25 (2H,m), 7.25-7.35 (13H, m), 7.73 (1H, dd, J=7.6 Hz, 1.5 Hz), 8.33 (1H, dd, J=4.7 Hz, 1.5 Hz).

To a solution of 1-(2,3,4,6-tetra-*O*-benzyl-β-D-glucopyranosyl)-3-(4-methoxybenzyl)-7-azaindole (50 mg) in dichloromethane (1 mL) was added boron trichloride (1M dichloromethane solution, 0.33 mL) under ice-cooling, and the resulting mixture was stirred at room temperature for 2 hours. To the reaction liquid was added ethanol, and the solvent was removed under reduced pressure. The residue was purified by column chromatography on silica gel (eluent: dichloromethane/methanol = 7/1) to give the title compound (12 mg).

$^1$H-NMR ($CD_3OD$) δ ppm:

3.50-3.70 (3H, m), 3.70-3.85 (5H, m), 3.92 (1H, dd, J=12.0 Hz, 2.4 Hz), 4.12 (2H, s) , 5.69 (1H, d, J=9.0 Hz) , 6.85 (2H, d, J=8.7 Hz), 7.22 (2H, d, J=8.8 Hz), 7.53 (1H, dd, J=7.9 Hz, 5.6 Hz), 7.69 (1H, s), 8.41 (1H, d, J=5.6 Hz), 8.49 (1H, dd, J=7.9 Hz, 0.8 Hz).

Example 343

4-Chloro-1-(β-D-glucopyranosyl)-3-{2-(4-methoxyphenyl)-prop-2-yl}-1H-indole

[Chem.32]

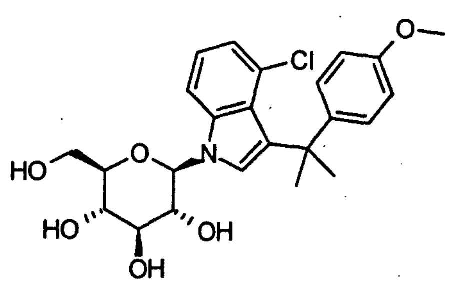

To a solution of 4-chloro-3-{2-(4-methoxyphenyl)-prop-2-yl}-1H-indole (0.15 g) in acetonitrile (6 mL) were added potassium hydroxide (0.22 g) and anhydrous sodium sulfate (1. 42 g) at room temperature, and the mixture was stirred for 30 minutes. To the reaction mixture was added a solution of 2,3,4,6-tetra-*O*-benzyl-α-D-glucopyranosylchloride (0.7 g) in acetonitrile (2 mL), and the mixture was stirred at room temperature overnight. The reaction mixture was poured into water, and the resultingmixture was extracted with ethyl acetate. The organic layer was washed with water and brine

successively, and dried over anhydrous sodium sulfate. The solvent was removed under reduced pressure, and the residue was purified by column chromatography on aminopropylated silica gel (eluent: *n*-hexane/ethyl acetate = 10/1-2/1) to give 1-(2,3,4,6-tetra-*O*-benzyl-β-D-glucopyranosyl)-4-chloro-3-{2-(4-methoxyphenyl)-prop-2-yl}-1H-indole (0.28 g). To a solution of the obtained adduct compound (0.27 g) in tetrahydrofuran/methanol (= 1/1, 3 mL) was added 10% palladium-carbon powder (50 mg), and the mixture was stirred under a hydrogen atmosphere overnight. The insoluble material was removed by filtration, and the solvent of the filtrate was removed under reduced pressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 80/20-40/60) to give the title compound (48 mg).
$^1$H-NMR ($CD_3OD$) δ ppm:
1.77 (3H, s), 1.78 (3H, s), 3.45-3.7 (3H, m), 3.7-3.8 (4H, m), 3.85-4.05 (2H, m), 5.06 (1H, d, J=9.3Hz), 6.65-6.75 (2H, m), 6.85-6.9 (1H, m), 6.95-7.15 (3H, m), 7.45-7.55 (2H, m).

Example 344

<u>3-(4-Ethylphenyl)oxy-1-(β-D-glucopyranosyl)-1H-indole</u>

[Chem. 33]

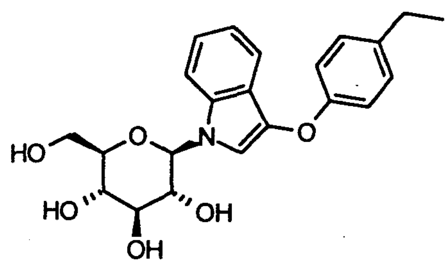

The title compound was prepared in a similar manner to that described in Example 343, using 3-(4-ethylphenyl) oxy-1H-indole instead of 4-chloro-3-{2-(4-methoxyphenyl)-prop-2-yl}-1H-indole.
$^1$H-NMR ($CD_3OD$) δ ppm:
1.2 (3H, t, J=7.6Hz), 2.58 (2H, q, J=7.6Hz), 3.4-3.55 (1H, m), 3.55-3.65 (2H, m), 3.71 (1H, dd, J=12.2Hz, 5.6Hz), 3.8-3.95 (2H, m), 5.48 (1H, d, J=9Hz), 6.85-6.95 (2H, m), 6.95-7.05 (1H, m), 7.05-7.15 (2H, m), 7.15-7.3 (3H, m), 7.5-7.6 (1H, m).

Example 345

<u>1-(6-Deoxy-6-fluoro-β-D-glucopyranosyl)-3-(4-methoxybenzyl)-6-methyl-1H-indole</u>

[Chem. 34]

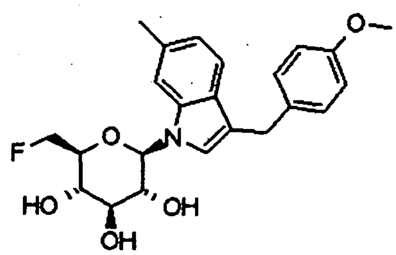

To a solution of the compound of Example 12 (1-(β-D-glucopyranosyl)-3-(4-methoxybenzyl)-6-methyl-1H-indole, 1.5 g) and imidazole (1.2 g) in N,N-dimethylformamide (15 mL) was added *t*-butyldimethylsilyl chloride (0.71 g) under ice-cooling, and the resulting mixture was stirred at room temperature for 1 hour. The reaction mixture was poured

into water, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: methanol/ethyl acetate = 1/10) to give 1-(6-*O*-*t*-butyldimethylsilyl-β-D-glucopyranosyl)-3-(4-methoxybenzyl)-6-methyl-1H-indole (1.35 g).

To a solution of 1-(6-*O*-*t*-butyldimethylsilyl-β-D-glucopyranosyl)-3-(4-methoxybenzyl)-6-methyl-1H- indole (1.35 g) and tetrabutylammonium iodide (0.05 g) was added 55% sodium hydride (0.2 g) under ice-cooling, and the resulting mixture was stirred for 20 minutes. To the reaction mixture was added benzyl bromide(1.5 g) at the same temperature, and the mixture was stirred at room temperature overnight. The reaction mixture was poured into water, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: n-hexane/ethyl acetate = 10/1-1/1) to give 1-(2,3,4-tri-*O*-benzyl-6-*O*-*t*-butyldimethylsilyl-β-D-glucopyranosyl)-3-(4-methoxybenzyl)-6-methyl-1H-indole (1.8 g).

To a solution of 1-(2,3,4-tri-*O*-benzyl-6-*O*-*t*-butyldimethylsilyl-β-D-glucopyranosyl)-3-(4-methoxybenzyl)-6-methyl-1H-indole (1.27 g) in tetrahydrofuran (2 mL) was added tetrabutylammonium fluoride (1M tetrahydrofuran solution, 2.4 mL) at room temperature, and the mixture was stirred at room temperature overnight. The reaction mixture was poured into water, and the resulting mixture was extracted with ethyl acetate. The organic layer was washed with water and brine successively, and dried over anhydrous sodium sulfate. The solvent was removed under reduced pressure, and the residue was purified by column chromatography on silica gel (eluent: n-hexane/ethyl acetate = 5/1-1/1) to give 1- (2, 3,4-tri-*O*-benzyl-β-D-glucopyranosyl)-3-(4-methoxybenzyl)-6-methyl-1H-indole (1.01 g).

To a solution of 1-(2,3,4-tri-*O*-benzyl-β-D-glucopyranosyl)-3-(4-methoxybenzyl)-6-methyl-1H-indole (0.23 g) in 1,2-dimethoxyethane (1.7 mL) was added (diethylamino)sulfur trifluoride (0.11 g) at room temperature, and the mixture was stirred at 65˚C for 1 hour. The reaction mixtue was cooled to room temperature, and then poured into a saturated aqueous sodium hydrogen carbonate solution. The resulting mixture was extracted with ethyl acetate. The organic layer was washed with water, a saturated aqueous sodium hydrogen carbonate solution and brine successively, and dried over anhydrous sodium sulfate. The solvent was removed under reduced pressure, and the residue was purified by column chromatography on silica gel (eluent: n-hexane/ethyl acetate = 5/1-1/1) to give 1-(2,3,4-triO-benzyl-6-deoxy-6-fluoro-β-D-glucopyranosyl)-3-(4-methoxybenzyl)-6-methyl-1H-indole (0.1 g).

To a solution of 1-(2,3,4-tri-*O*-benzyl-6-deoxy-6-fluoro-β-D-glucopyranosyl)-3-(4-methoxybenzyl)-6-methyl-1H-indole (0.1g) in tetrahydrofuran/methanol (= 1/1 (v/v), 4 mL) was added 10% palladium-carbon powder (0.1g), and the mixture was stirred under a hydrogen atmosphere overnight. The insoluble material was removed by filtration, and the solvent of the filtrate was removed under reduced pressure, and the residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (6 mg).

$^1$H-NMR ($CD_3OD$) δ ppm:

2.42 (3H, s), 3.45-3.8 (6H, m), 3.8-3.9 (1H, m), 3.97 (2H, s), 4.5-4.6(1H, m), 4.6-4.7 (1H, m), 5.39 (1H, d, J=9.1Hz), 6.7-6.9 (3H, m), 7.02 (1H, s), 7.1-7.2 (2H, m), 7.2-7.3 (2H, m).

Example 346

1-(β-D-Glucopyranosyl)-3-{(4-methoxyphenyl)sulfany}-6-methyl-1H-indole

[Chem.35]

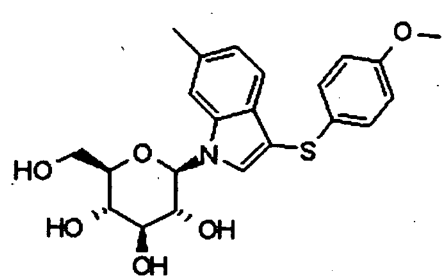

To a solution of bis(4-methoxyphenyl)disulfide (0.3 g) in 1,2-dichloroethane (4 mL) was added sulfuryl chloride (0.16 g) at room temperature, and the mixture was stirred for 5 minutes to prepare a sulfenyl chloride solution.

To a solution of 1-(2,3,4,6-tetra-*O*-acetyl-β-D-glucopyranosyl)-6-methyl-1H-indole(1g) in 1,2-dichloroethane (4 mL) and

N,N-dimethylformamide (4 mL) was added the above sulfenyl chloride solutionatroomtemperature, and the resulting mixture was stirred for 1 hour. The reaction mixture was poured into ice-water, and the resulting mixture was extracted with ethyl acetate. The organic layer was washed with a saturated aqueous sodium hydrogen carbonate solution, water and brine successively, and dried over anhydrous sodium sulfate. The solvent was removed under reduced pressure, and the residue was purified by column chromatography on silica gel (eluent: *n*-hexane/ethyl acetate = 3/1-1/3) to give 1-(2,3,4,6-tetra-*O*-acetyl-β-D-glucopyranosyl)-3-{(4-methoxyphenyl)-sulfany}-6-methyl-1H-indole (1.03'g).
To a solution of 1-(2,3,4,6-tetra-*O*-acetyl-β-D-glucopyranosyl)-3-{(4-methoxyphenyl)sulfany}-6-methyl-1H-indole (0.36 g) in dichloromethane (2 mL) and methanol (2 mL) was added sodium methoxide (28% methanol solution, 0.11 mL) at room temperature, and the resulting mixture was stirred for 1 hour. To the reaction mixture was added acetic acid (39 mg) , and the solvent was removed under reduced pressure. The residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (0.13 g).
$^1$H-NMR ($CD_3OD$) δ ppm:
2.44 (3H, s), 3.45-3.55 (1H, m), 3.55-3.65 (2H, m), 3.65-3.8 (4H, m), 3.85-3.95 (2H, m), 5.46 (1H, d, J=9.2Hz), 6.65-6.8 (2H, m), 6.85-6.95 (1H, m), 7.0-7.15 (2H, m), 7.31 (1H, d, J=8.2Hz), 7.39 (1H, br s), 7.6 (1H, s).

Example 347

1-(β-D-Glucopyranosyl)-3-{(2-fluoro-4-isopropyloxyphenyl)-sulfany}-1H-indole

[Chem. 36]

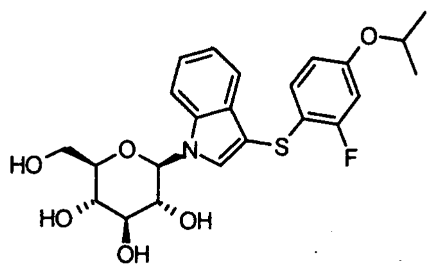

To a solution of 2-fluoro-4-isopropyloxy bromobenzene (0.5 g) in tetrahydrofuran (5 mL) was added of n-butyllitium (hexane solution 2.67 mol/L, 0.8 mL) under a argon atmosphere under dry ice-cooling in an acetone bath, and the resulting mixture was stirred for 20 minutes. To the reaction solution was added sulfur (69 mg) , and the mixture was stirred for 1 hour under ice-cooling and additional 1 hour at room temperature. To the reaction mixture was added an aqueous ammonium chloride solution to quench the reaction, and the resulting mixture was extracted with ethyl acetate. The organic layer was washed with water and brine successively, and dried over anhydrous sodium sulfate. The solvent was removed under reduced pressure to give the corresponding thiophenol compound, 2-fluoro-4-isopropyloxy thiophenol (385 mg).
To a solution of the obtained thiophenol compound (42 mg) in dichloromethane (1 mL) was added sulfuryl chloride (30 mg) under ice-cooling, and the mixture was stirred for 1 hour at room temperature to prepare a sulfenyl chloride solution. To a solution of 1-(2,3,4,6-tetra-*O*-acetyl-β-D-glucopyranosyl)-1H-indole (50 mg) in dichloromethane (1 mL) was added the above sulfenyl chloride solution at room temperature, and the resulting mixture was stirred overnight. The reaction mixture was purified directly by column chromatography on silica gel (eluent: n-hexane/ethyl acetate = 3/1-1/3) to give 1-(2,3,4,6-tetra-*O*-acetyl-β-D-glucopyranosyl)-3-{(2-fluoro-4-isopropyloxyphenyl)sulfany}-1H-indole (28 mg).
To a solution of 1-(2,3,4,6-tetra-*O*-acetyl-β-D-glucopyranosyl)-3-((2-fluoro-4-isopropyloxyphenyl)sulfany)-1H-indole (28 mg) in methanol (2 mL) was added sodium methoxide (28% methanol solution, 0.11 mL) at room temperature, and the resulting mixture was stirred for 1 hour. To the reaction mixture was added acetic acid (0.02 mL), and the solvent was removed under reduced pressure. The residue was purified by preparative reverse phase column chromatography (Shiseido CAPCELL PAK UG80 ODS, 5 μm, 20 x 50 mm, flow rate 30 mL/minute, linear gradient, water/acetonitrile = 90/10-40/60) to give the title compound (5 mg).
$^1$H-NMR ($CD_3OD$) δ ppm:

1.24 (6H, d, J=6.2Hz), 3.45-3.55 (1H, m), 3.55-3.65 (2H, m), 3.72 (1H, dd, J=12.1Hz, 5.4Hz), 3.85-3. 95 (2H, m), 4. 4-4.55 (1H, m), 5.49 (1H, d, J=9.1Hz), 6.45-6.55 (1H, m), 6.6-6.7 (1H, m), 6.8-6.95 (1H, m), 7.05-7.15 (1H, m), 7.2-7.25 (1H, m), 7.45-7.55 (1H, m), 7.55-7.65 (1H, m), 7.72 (1H, s).

Examples 348 to 611

The compounds described in Table 7 can be prepared in a similar manner to that described in Example 346 or Example 347, by means of reactions with 1-(2,3,4,6-tetra-*O*-acetyl-β-D-glucopyranosyl)-1H-indole compounds and sulfuryl chloride compounds which are prepared from the corresponding disulfide compounds or thiophenol compounds.

[Table 7]

Table 7-1

| | | |
|---|---|---|
| 348 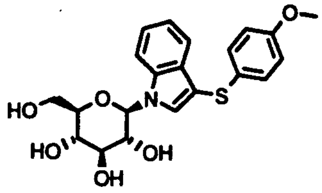 | 349 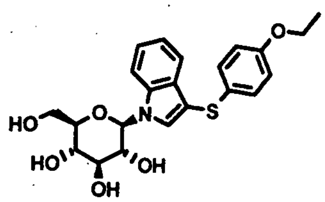 | 350 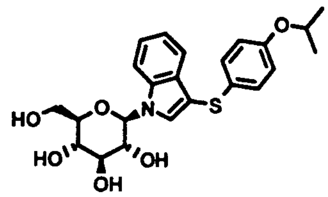 |
| 351 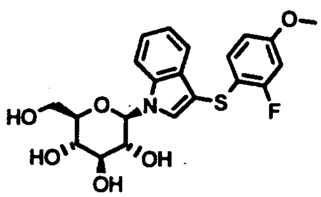 | 352 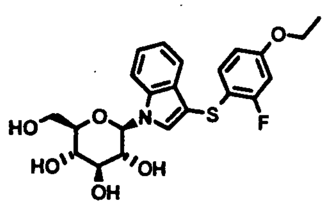 | 353 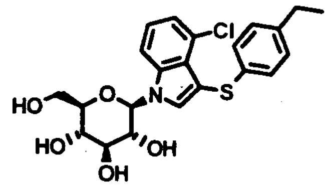 |
| 354 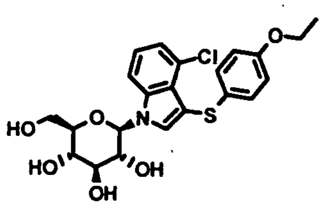 | 355 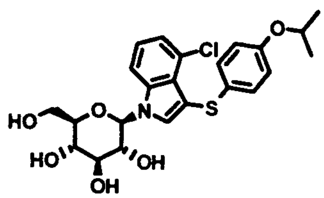 | 356 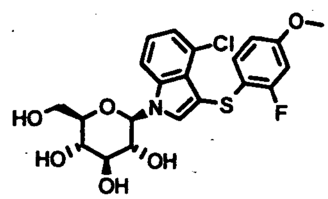 |
| 357 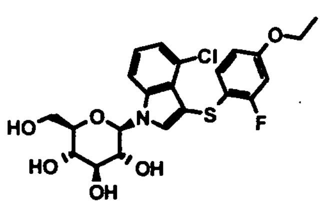 | 358 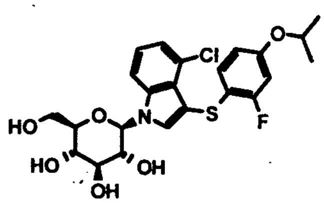 | 359 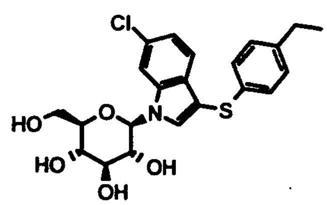 |
| 360 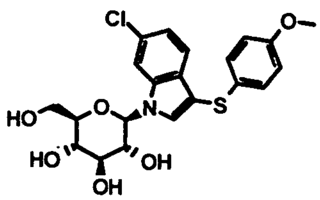 | 361 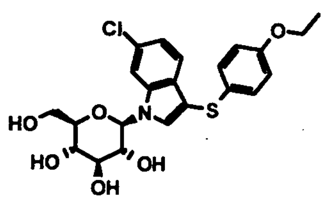 | 362 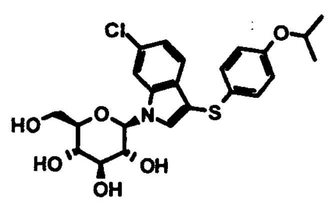 |
| 363 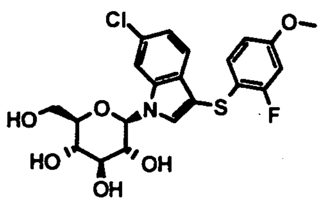 | 364 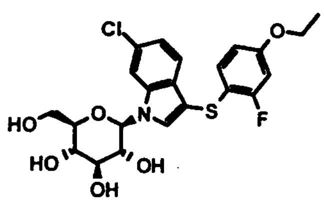 | 365 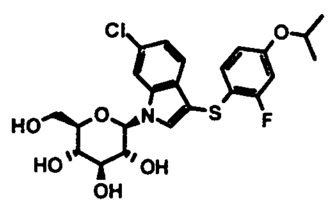 |

Table 7-2

| | | |
|---|---|---|
| 366 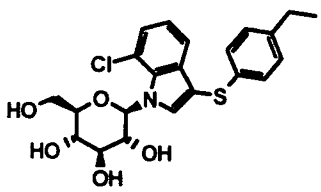 | 367 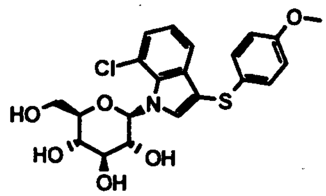 | 368 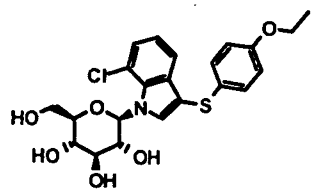 |
| 369 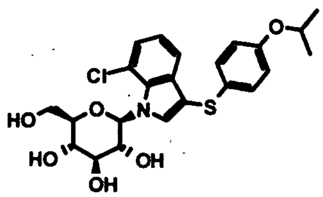 | 370 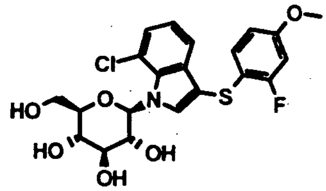 | 371 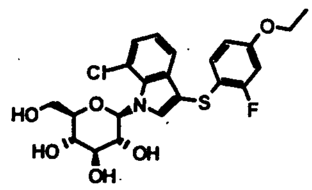 |
| 372 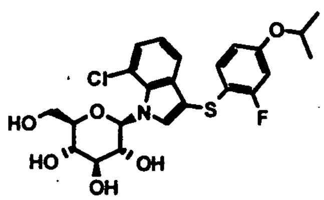 | 373 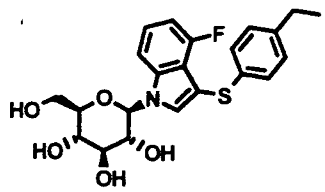 | 374 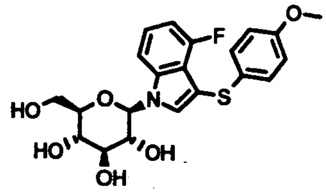 |
| 375 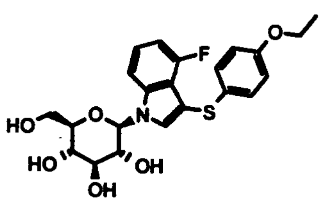 | 376 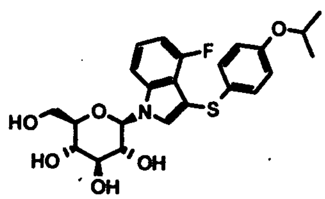 | 377 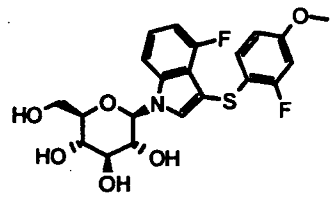 |
| 378 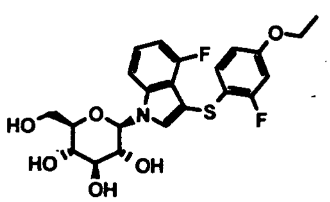 | 379 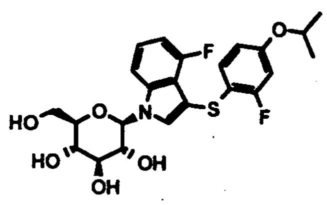 | 380 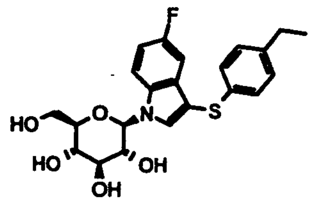 |
| 381 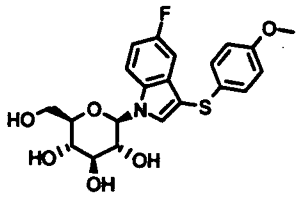 | 382 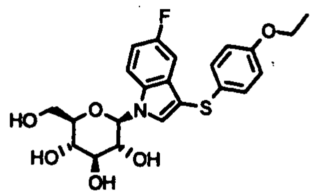 | 383 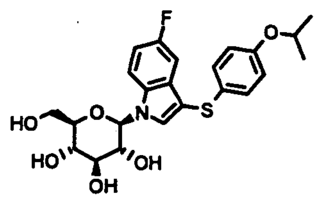 |

Table 7-3

| | | |
|---|---|---|
| 384 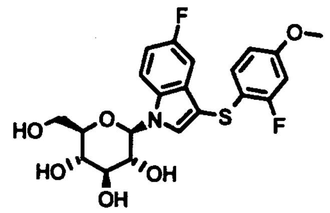 | 385 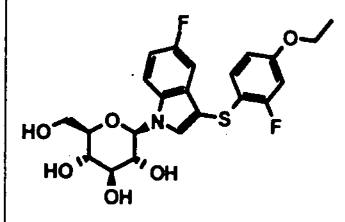 | 386 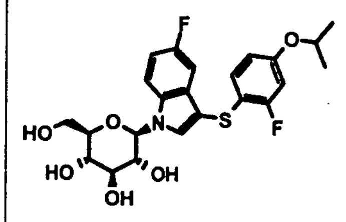 |
| 387 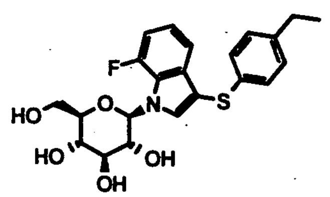 | 388 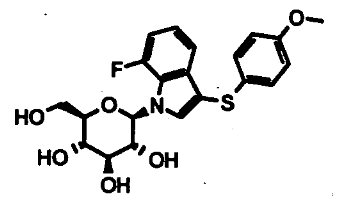 | 389 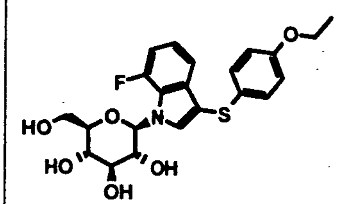 |
| 390 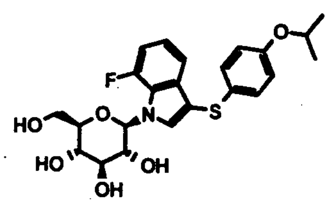 | 391 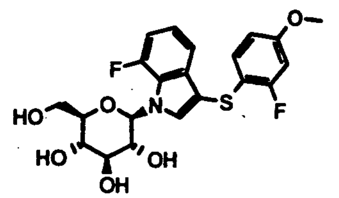 | 392 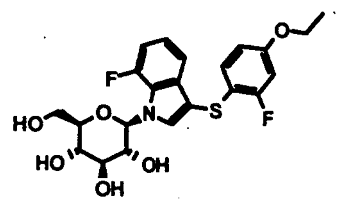 |
| 393 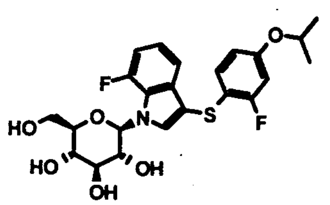 | 394 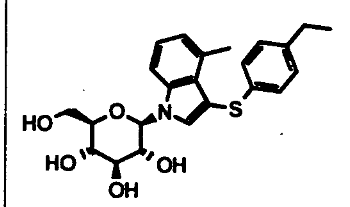 | 395 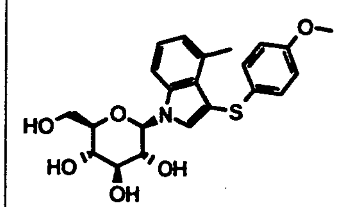 |
| 396 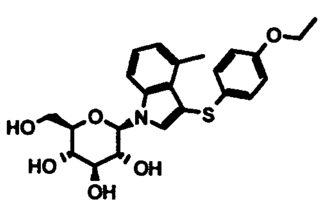 | 397 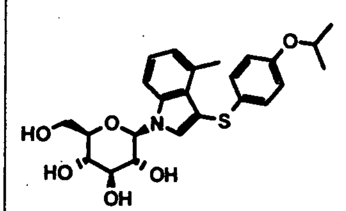 | 398 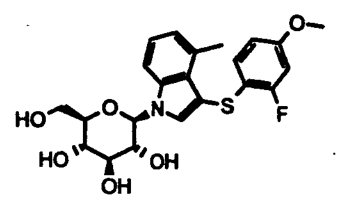 |
| 399 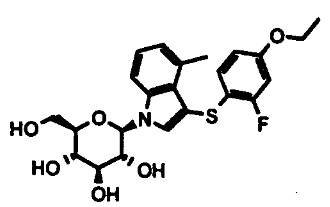 | 400 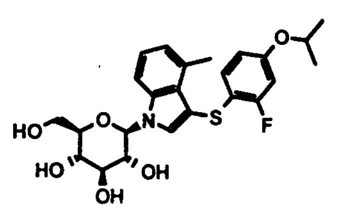 | 401 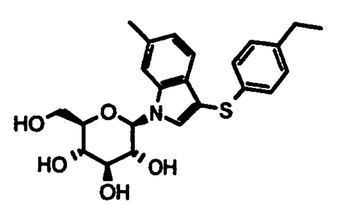 |

Table 7-4

| | | |
|---|---|---|
| 402 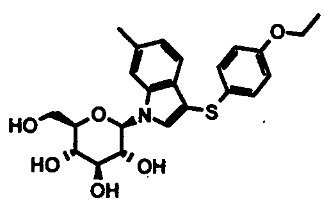 | 403 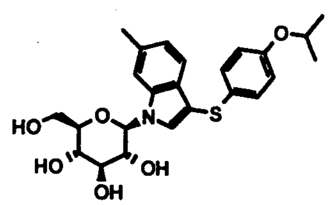 | 404 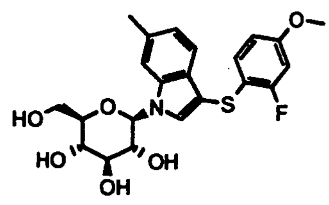 |
| 405 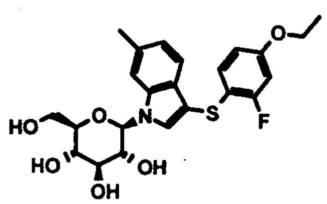 | 406 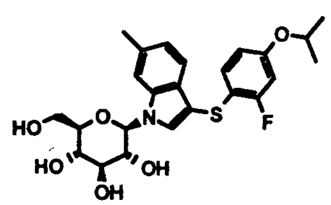 | 407 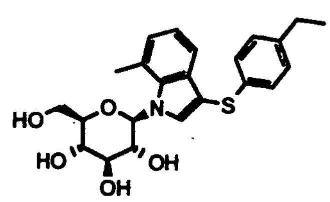 |
| 408 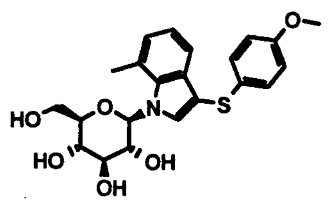 | 409 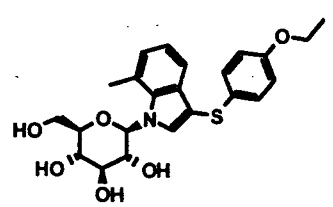 | 410 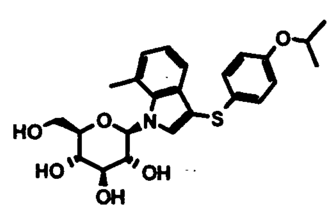 |
| 411 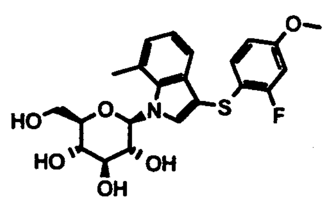 | 412 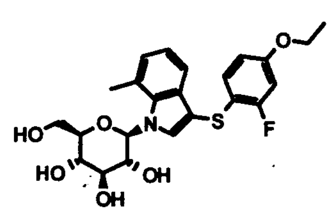 | 413 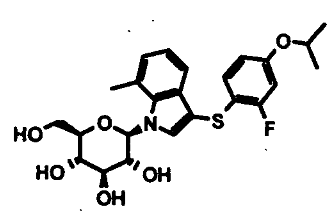 |
| 414 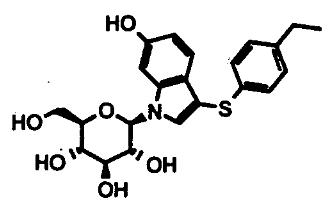 | 415 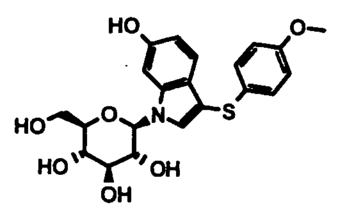 | 416 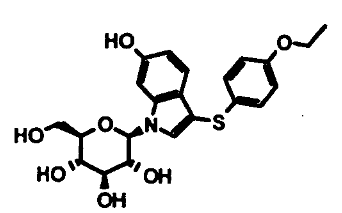 |
| 417 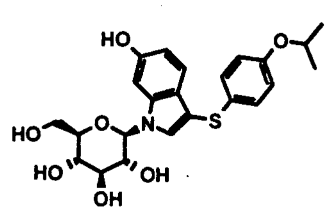 | 418 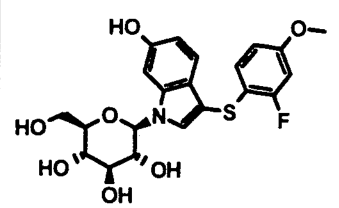 | 419 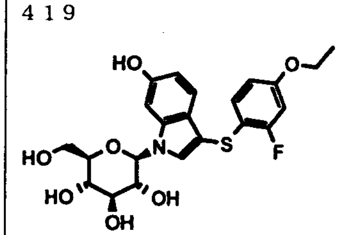 |

Table 7-5

| | | |
|---|---|---|
| 420 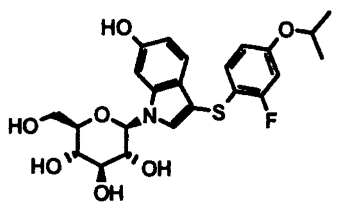 | 421 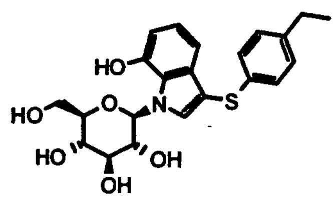 | 422 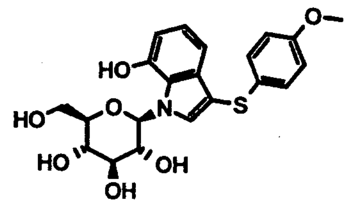 |
| 423 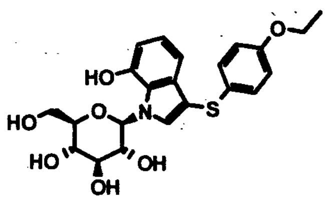 | 424 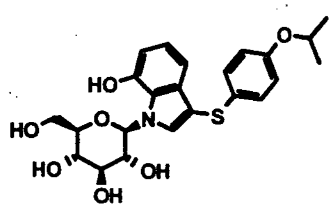 | 425 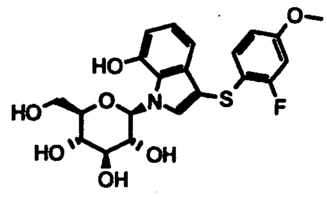 |
| 426 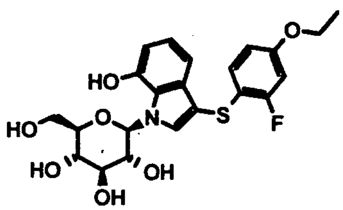 | 427 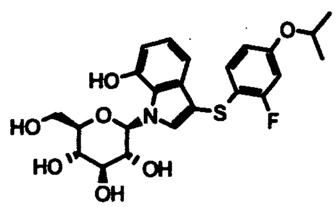 | 428 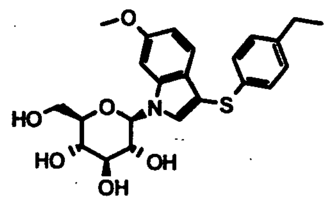 |
| 429 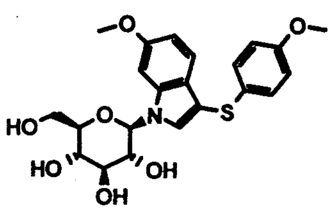 | 430 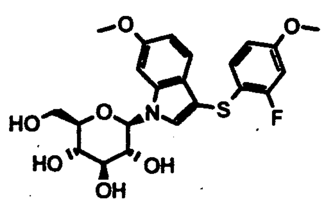 | 431 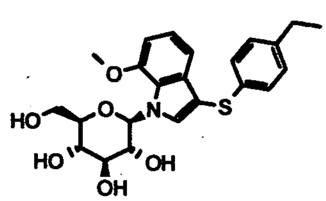 |
| 432 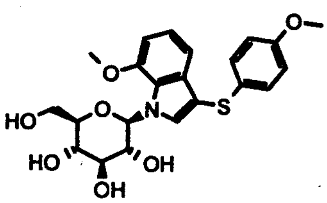 | 433 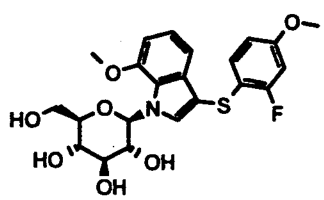 | 434 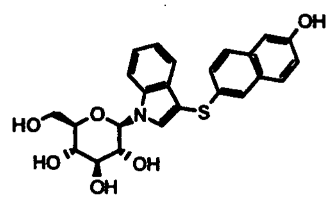 |
| 435 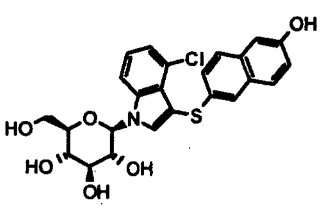 | 436 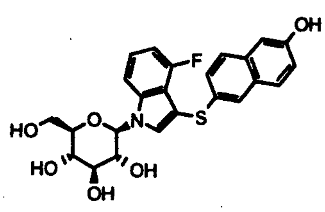 | 437 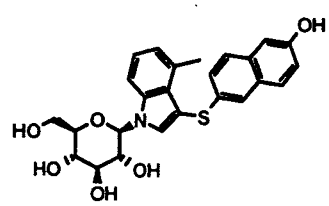 |

Table 7-6

| | | |
|---|---|---|
| 438 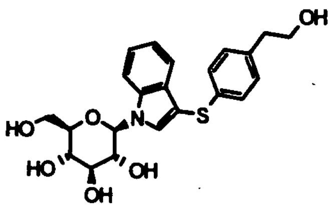 | 439 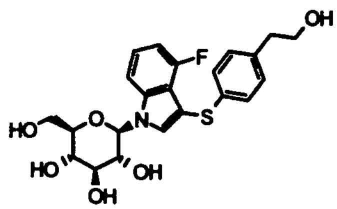 | 440 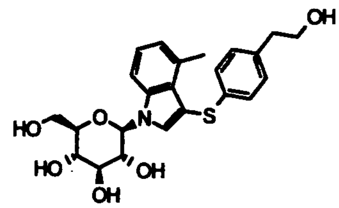 |
| 441 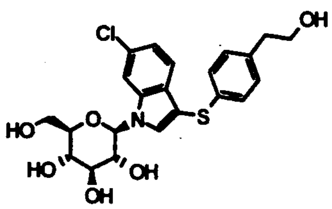 | 442 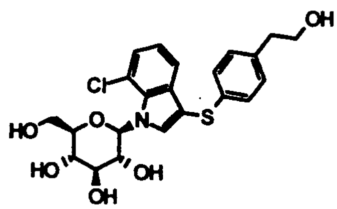 | 443 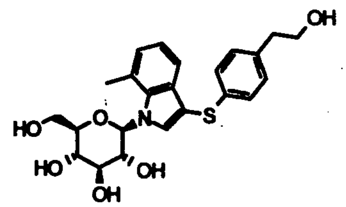 |
| 444 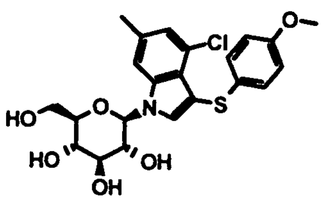 | 445 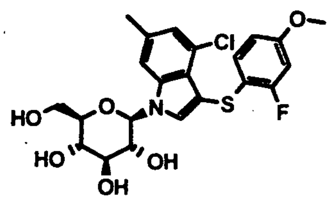 | 446 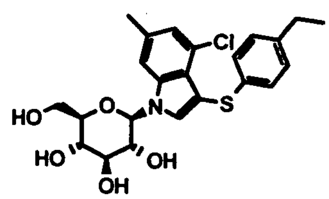 |
| 447 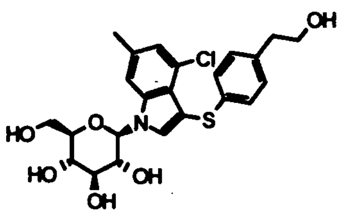 | 448 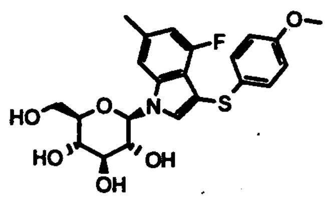 | 449 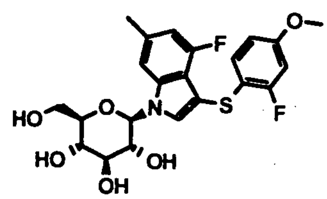 |
| 450 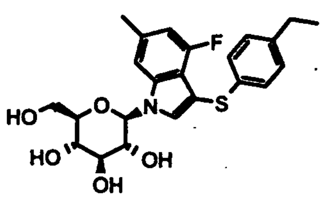 | 451 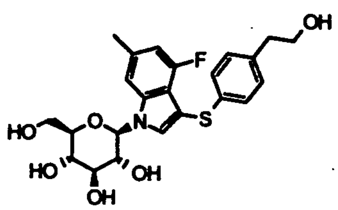 | 452 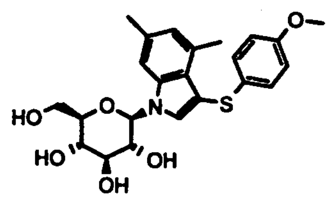 |
| 453 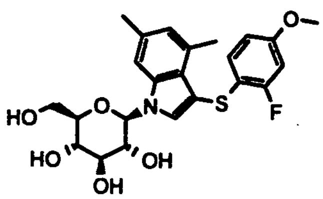 | 454 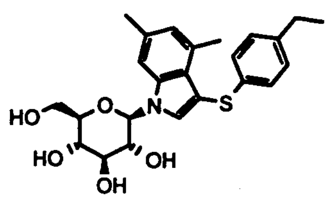 | 455 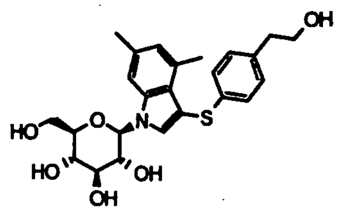 |

Table 7-7

| | | |
|---|---|---|
| 456 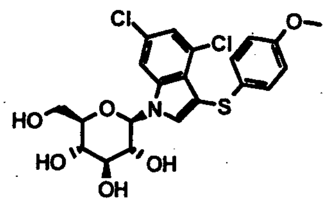 | 457 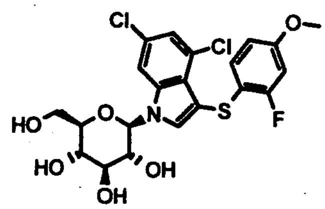 | 458 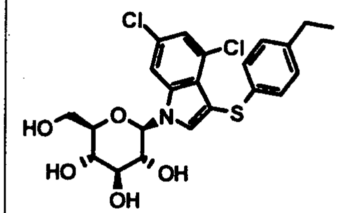 |
| 459 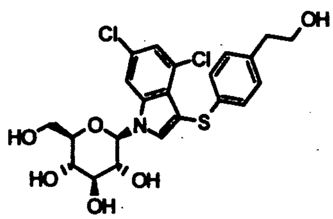 | 460 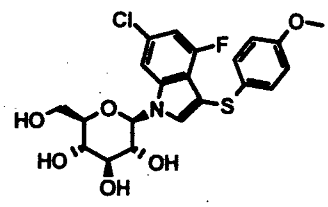 | 461 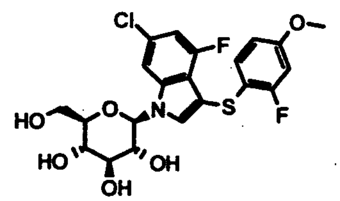 |
| 462 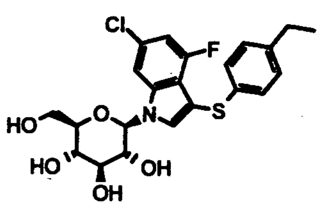 | 463 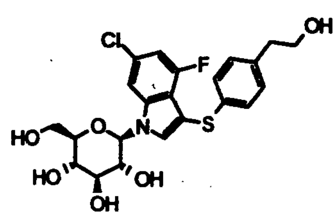 | 464 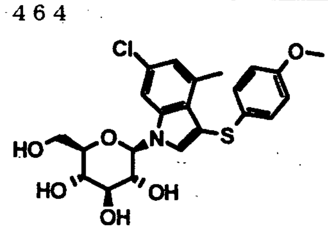 |
| 465 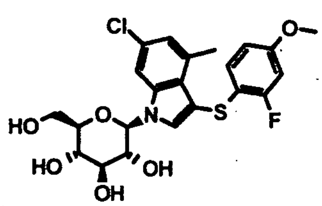 | 466 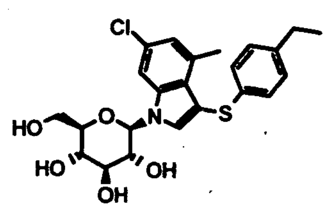 | 467 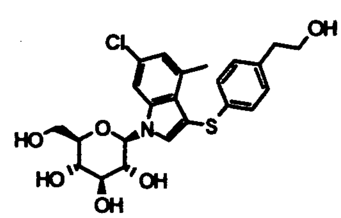 |
| 468 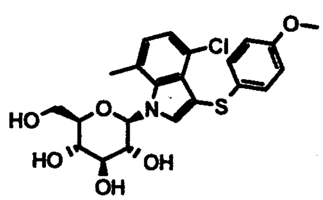 | 469 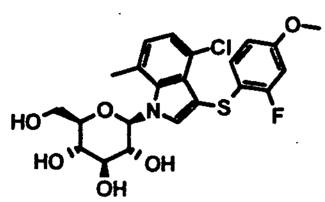 | 470 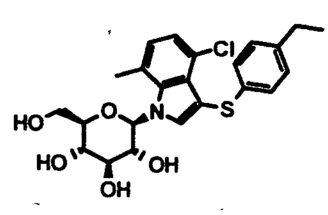 |
| 471 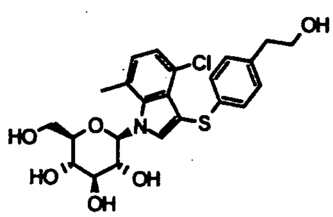 | 472 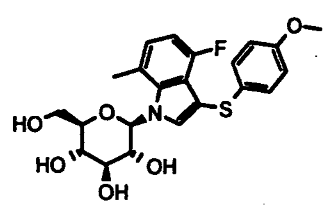 | 473 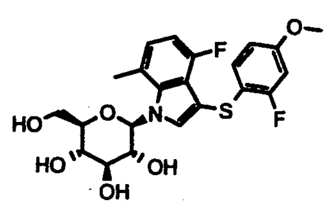 |

Table 7-8

| | | |
|---|---|---|
| 474 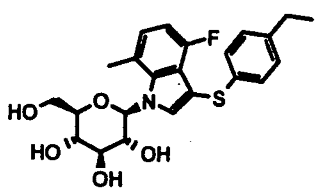 | 475 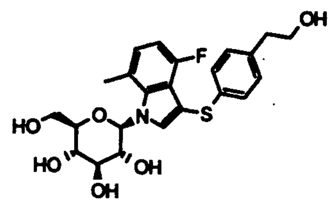 | 476 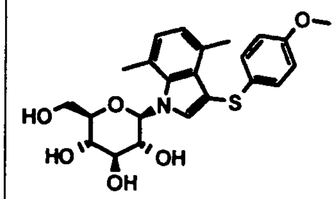 |
| 477 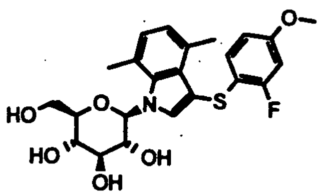 | 478 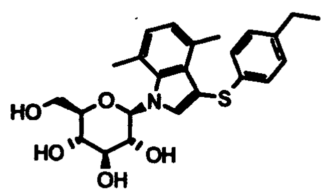 | 479 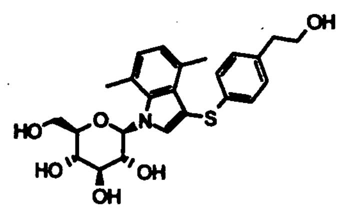 |
| 480 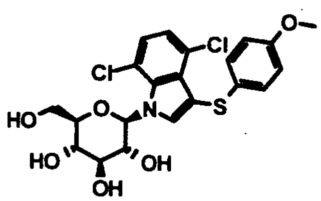 | 481 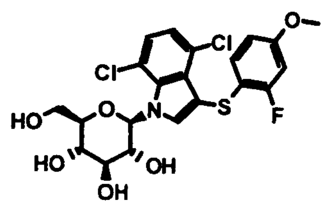 | 482 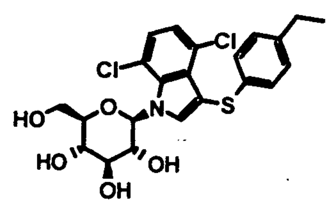 |
| 483 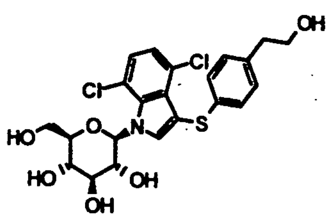 | 484 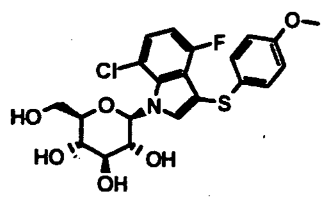 | 485 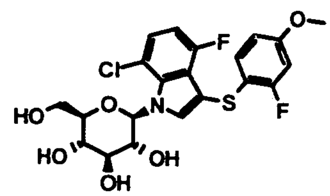 |
| 486 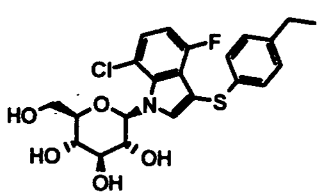 | 487 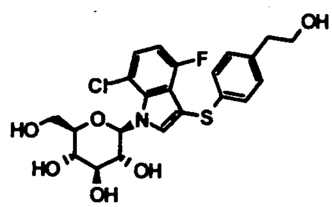 | 488 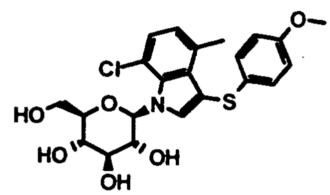 |
| 489 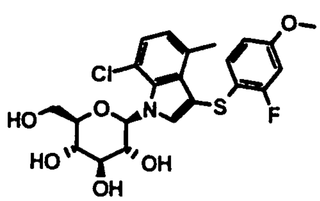 | 490 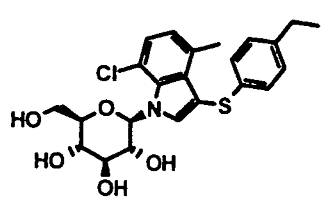 0 | 491 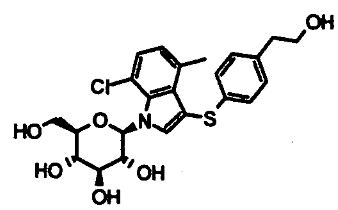 |

Table 7-9

| | | |
|---|---|---|
| 492<br>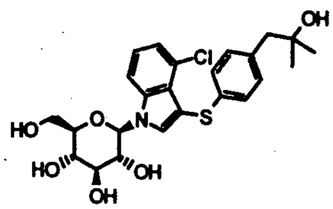 | 493<br>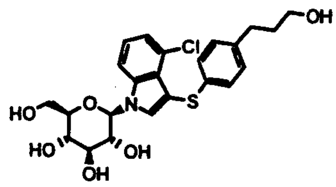 | 494<br>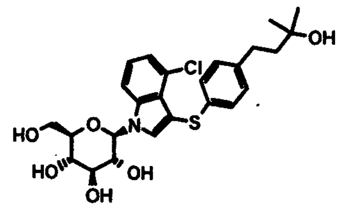 |
| 495<br>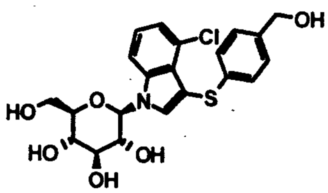 | 496<br>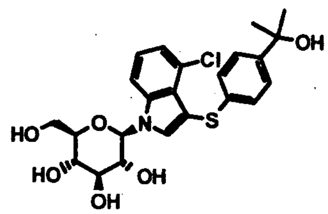 | 497<br>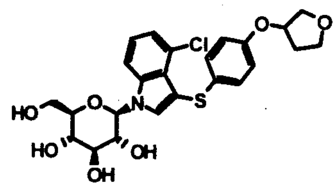 |
| 498<br>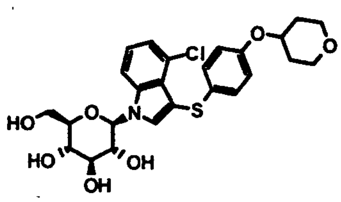 | 499<br>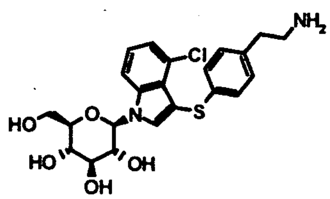 | 500<br>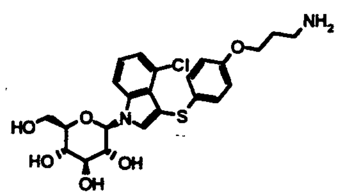 |
| 501<br>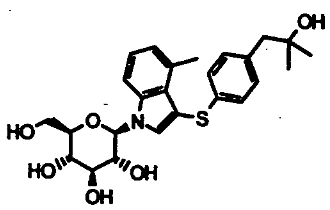 | 502<br>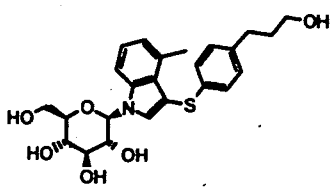 | 503<br>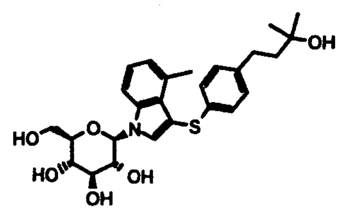 |
| 504<br>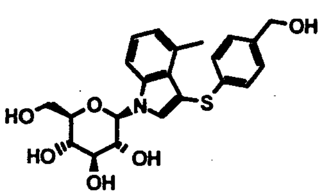 | 505<br>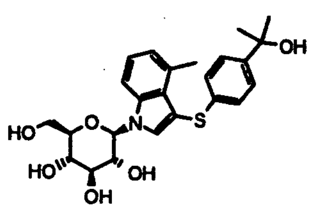 | 506<br>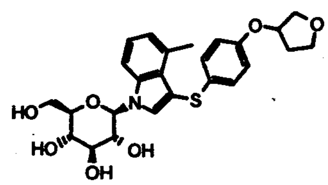 |
| 507<br>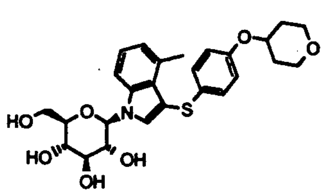 | 508<br>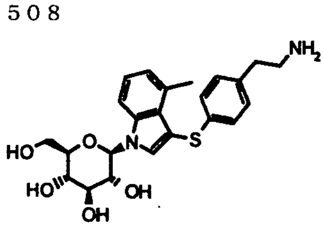 | 509<br>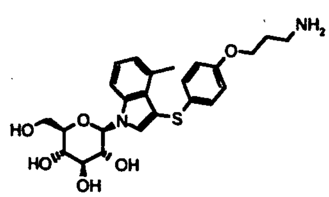 |

Table 7-10

| | | |
|---|---|---|
| 510 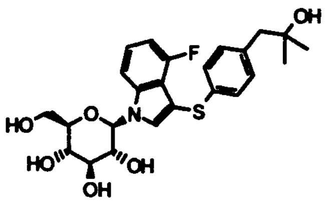 | 511 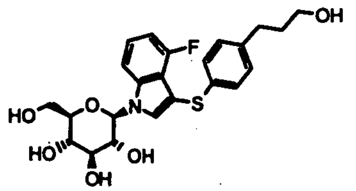 | 512 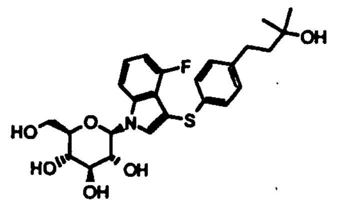 |
| 513 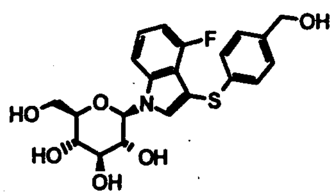 | 514 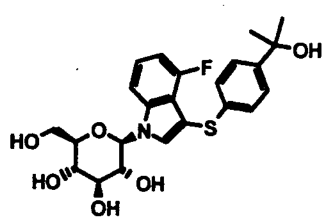 | 515 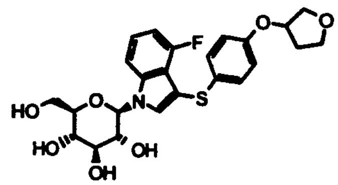 |
| 516 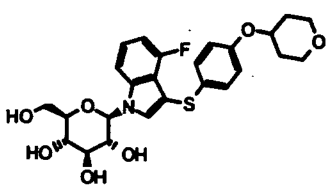 | 517 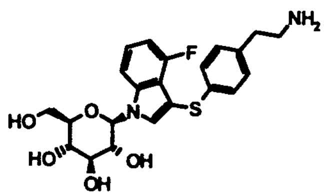 | 518 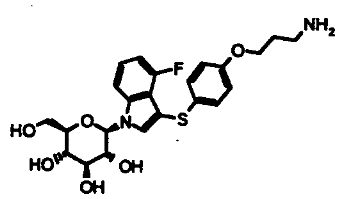 |
| 519 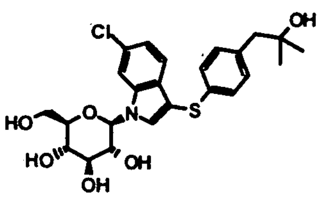 | 520 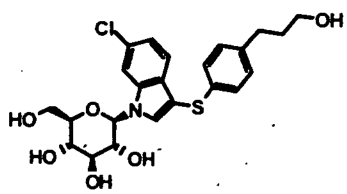 | 521 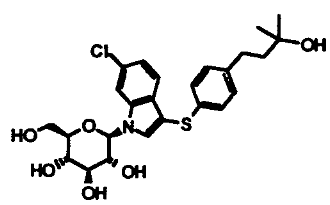 |
| 522 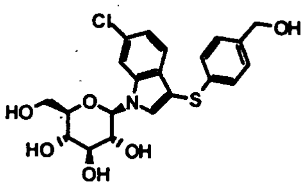 | 523 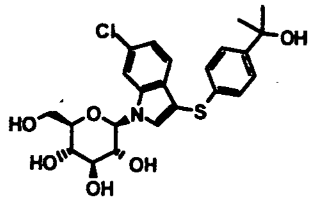 | 524 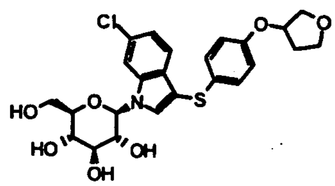 |
| 525 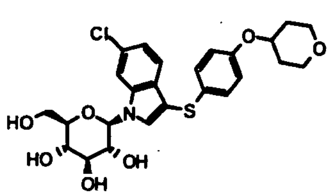 | 526 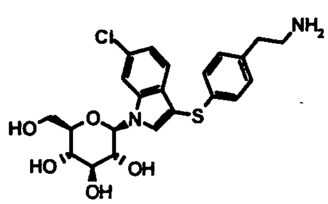 | 527 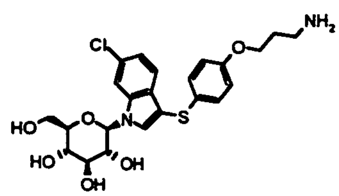 |

Table 7-11

| | | |
|---|---|---|
| 528 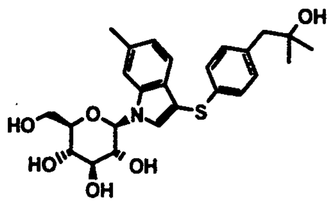 | 529 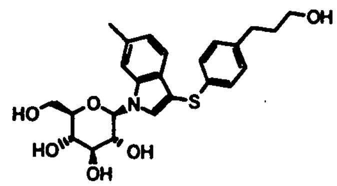 | 530 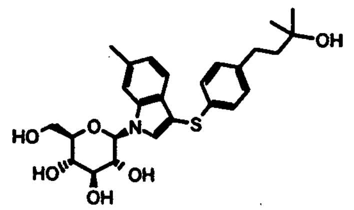 |
| 531 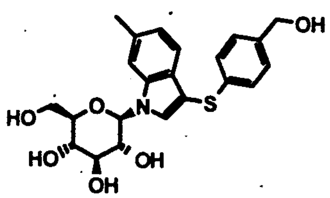 | 532 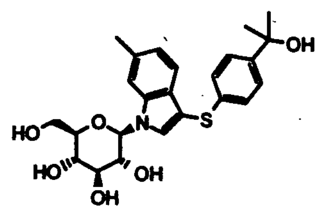 | 533 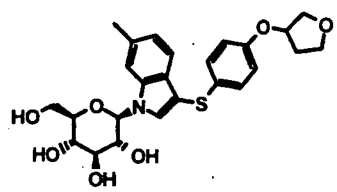 |
| 534 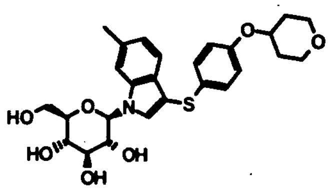 | 535 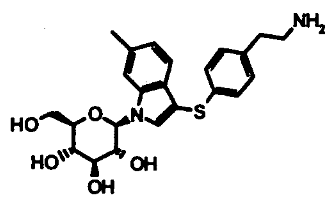 | 536 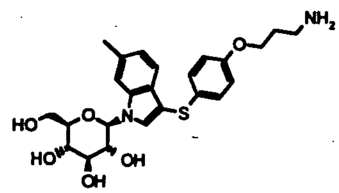 |
| 537 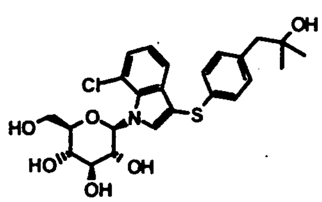 | 538 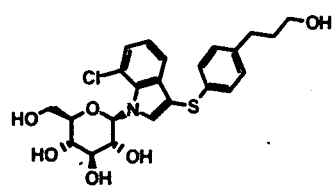 | 539 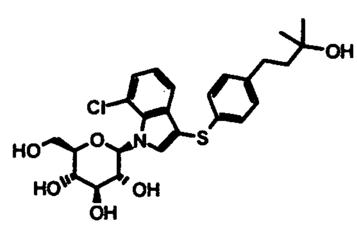 |
| 540 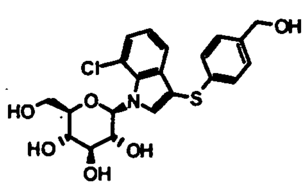 | 541 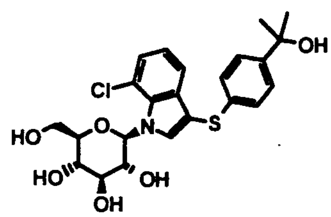 | 542 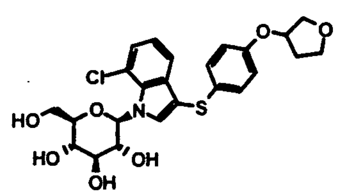 |
| 543 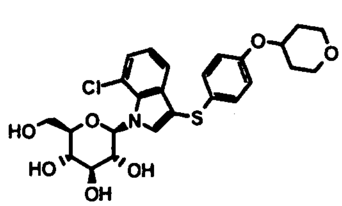 | 544 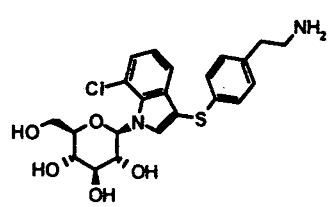 | 545 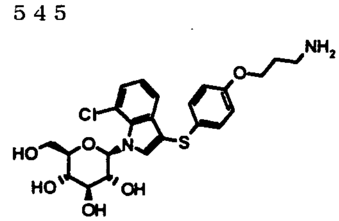 |

Table 7-12

| | | |
|---|---|---|
| 546 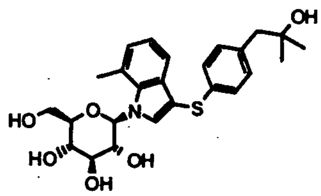 | 547 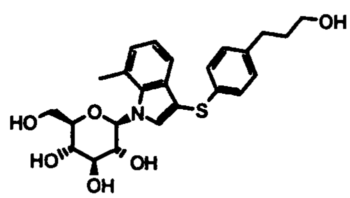 | 548 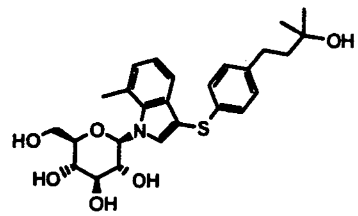 |
| 549 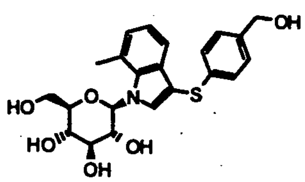 | 550 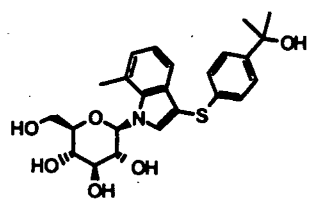 | 551 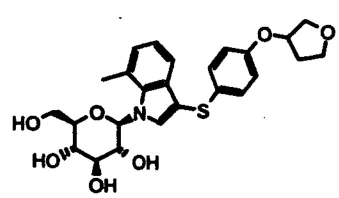 |
| 552 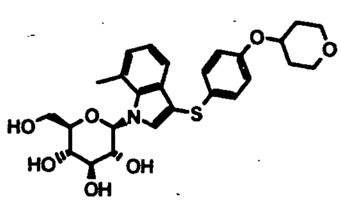 | 553 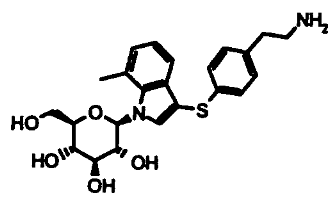 | 554 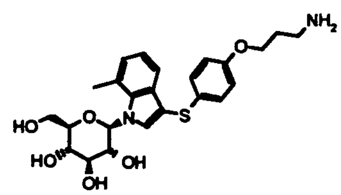 |
| 555 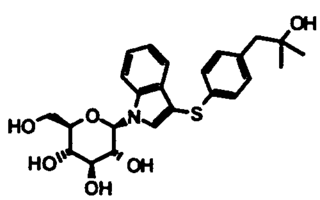 | 556 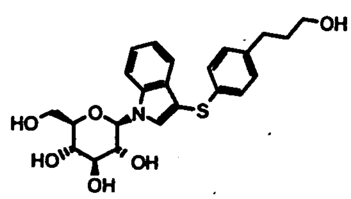 | 557 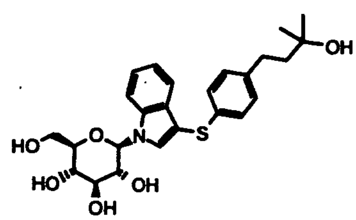 |
| 558 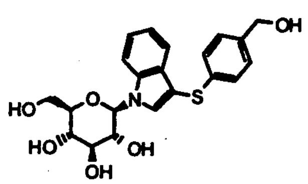 | 559 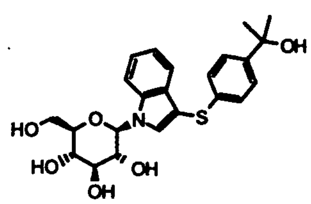 | 560 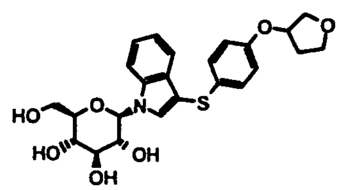 |
| 561 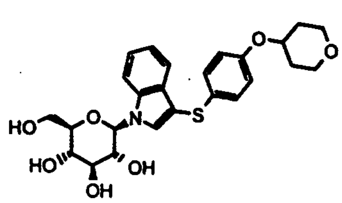 | 562 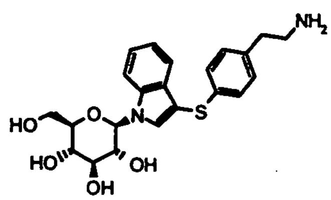 | 563 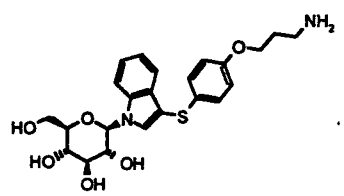 |

Table 7-13

| | | |
|---|---|---|
| 546 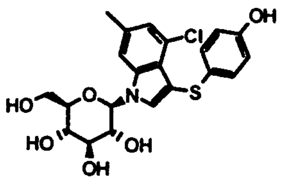 | 565 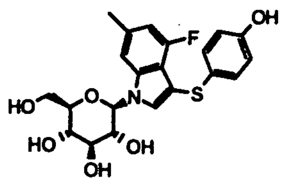 | 566 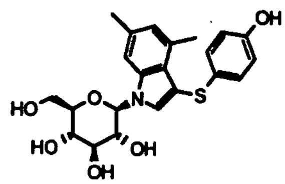 |
| 567 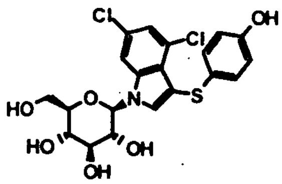 | 568 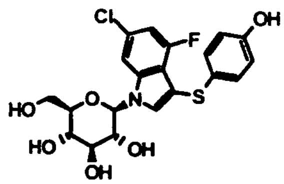 | 569 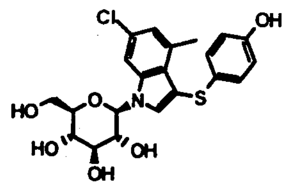 |
| 570 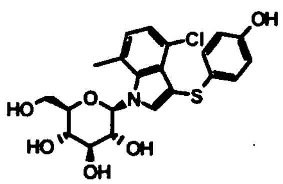 | 571 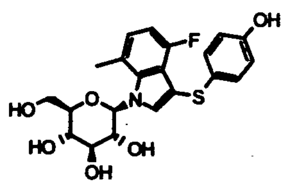 | 572 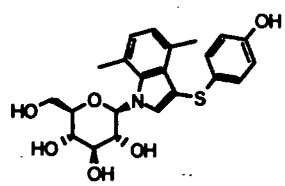 |
| 573 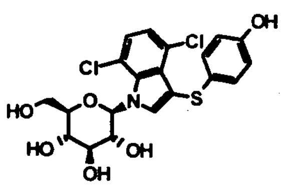 | 574 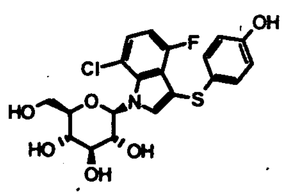 | 575 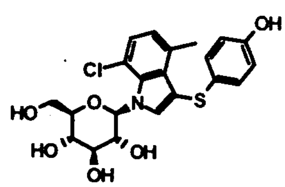 |
| 576 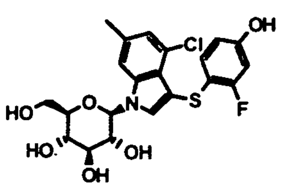 | 577 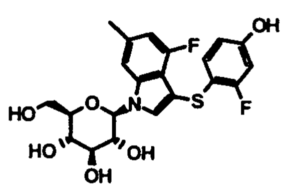 | 578 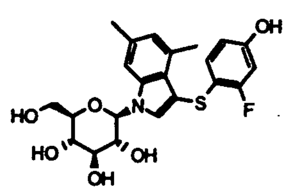 |
| 579 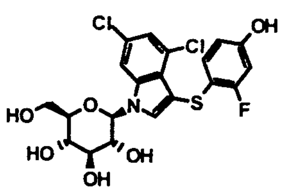 | 580 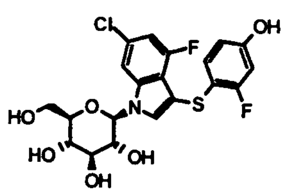 | 581 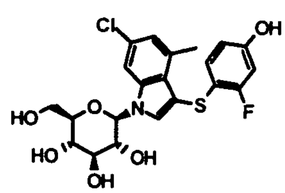 |

Table 7-14

| | | |
|---|---|---|
| 582 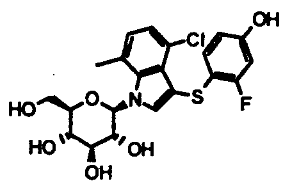 | 583 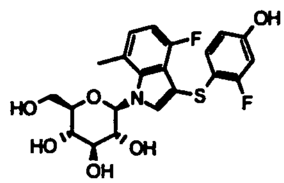 | 584 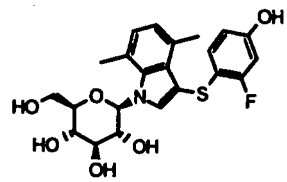 |
| 585 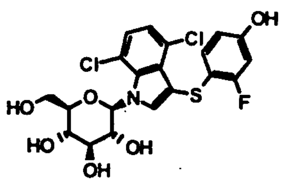 | 586 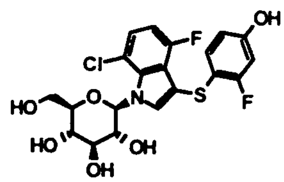 | 587 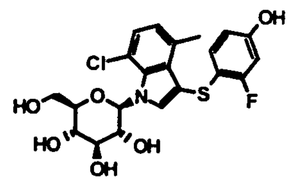 |
| 588 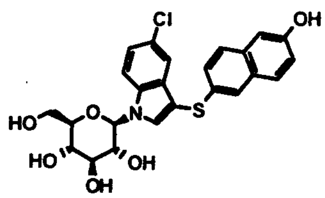 | 589 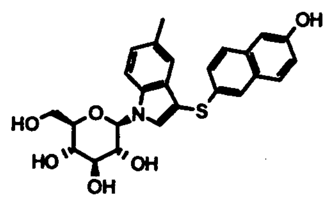 | 590 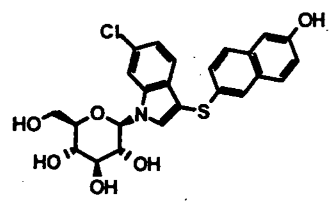 |
| 591 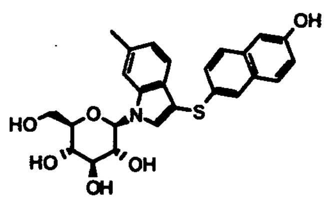 | 592 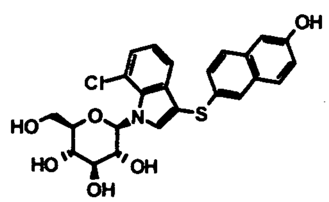 | 593 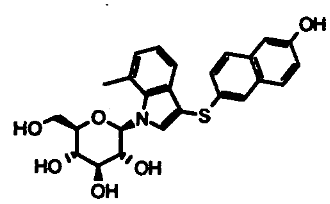 |
| 594 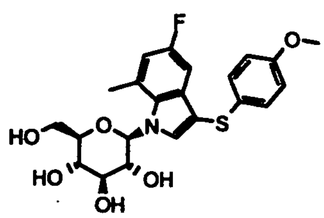 | 595 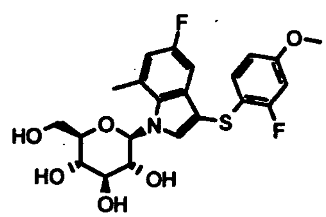 | 596 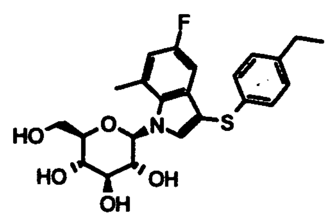 |
| 597 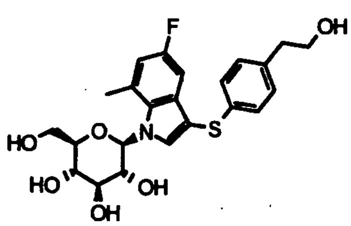 | 598 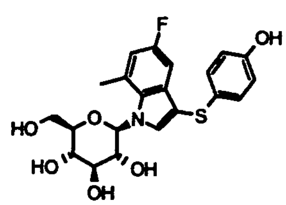 | 599 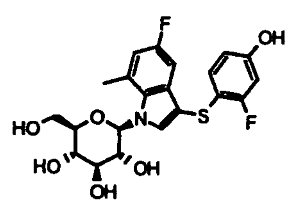 |

Table 7-15

| | | |
|---|---|---|
| 600 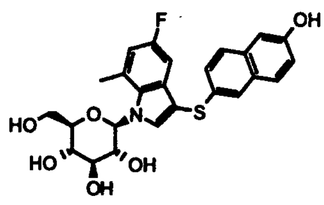 | 601 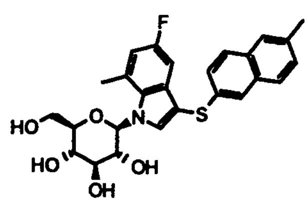 | 602 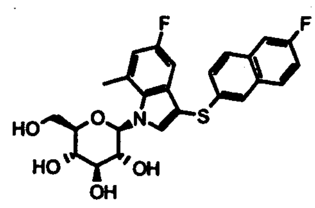 |
| 603 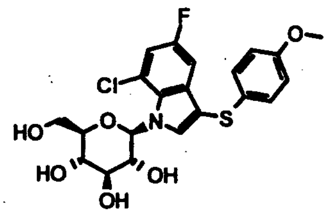 | 604 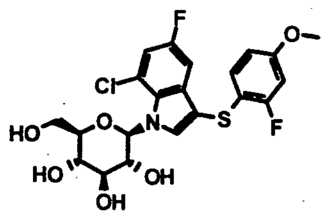 | 605 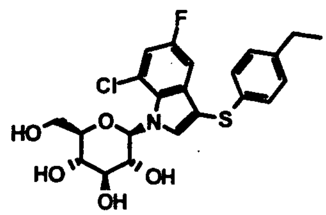 |
| 606 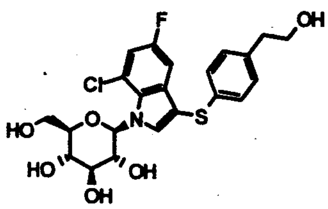 | 607 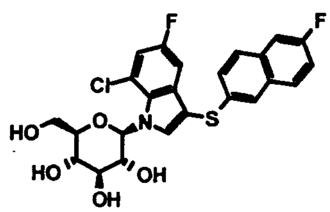 | 608 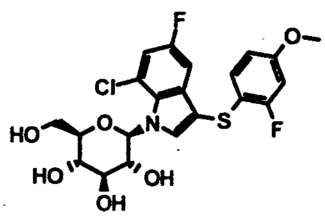 |
| 609 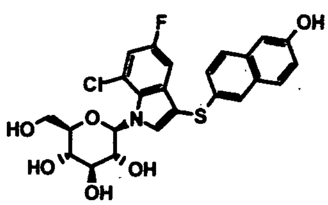 | 610 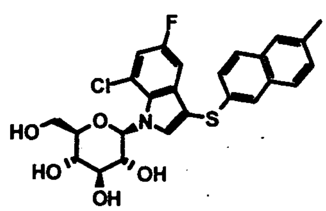 | 611 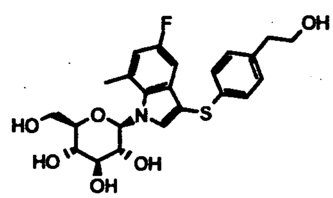 |

Examples 612 to 626

The compounds described in Table 8 can be prepared from the compounds of the Example 63, Example 85 and Example 86 by means of a usual manner to prepare prodrugs.

[Table 8]

| | | |
|---|---|---|
| 612<br>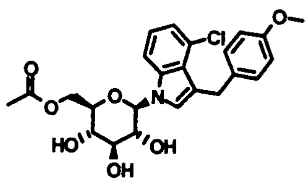 | 613<br>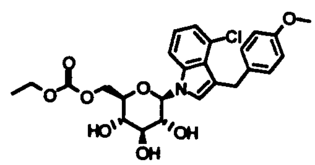 | 614<br>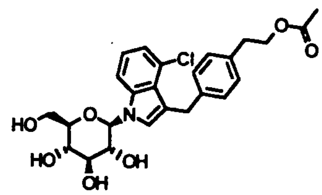 |
| 615<br>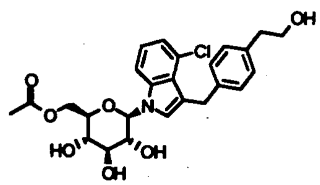 | 616<br>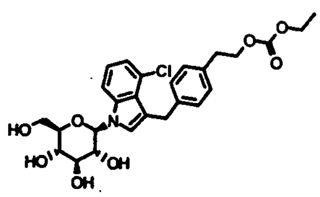 | 617<br>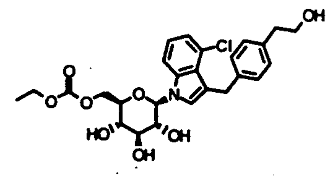 |
| 618<br>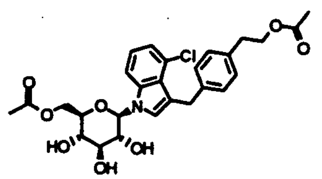 | 619<br>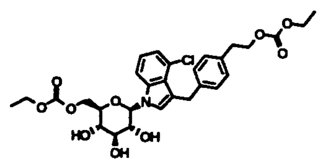 | 620<br>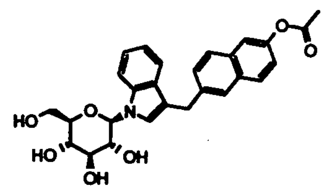 |
| 621<br>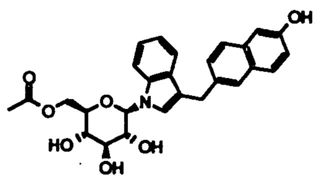 | 622<br>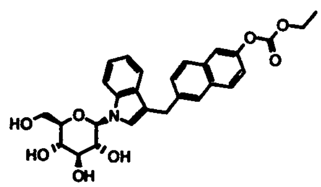 | 623<br>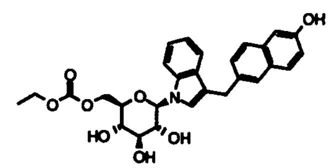 |
| 624<br>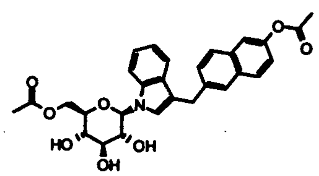 | 625<br>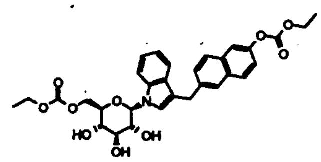 | 626<br>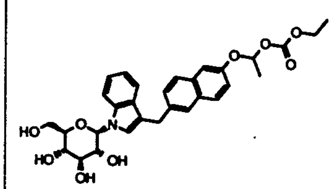 |

Examples 627 to 629

The compounds described in Table 9 were prepared in a similar manner to that described in Example 330 or Example 347.

[Table 9]

| Example No. | Chemical structure | $^1$H-NMR ($CD_3OD$) δ ppm: |
|---|---|---|
| Ex.627 | 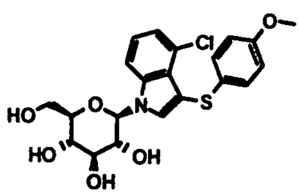 | 3.45-3.55 (1H, m), 3.55-3.6 (2H, m), 3.65-3.8 (4H, m), 3.8-3.95 (2H, m), 5.49 (1H, d, J=9.1Hz), 6.7-6.8 (2H, m), 7-7.1 (3H, m), 7.1-7.2 (1H, m), 7.5-7.6 (1H, m), 7.7 (1 H, s) |

(continued)

| Example No. | Chemical structure | $^1$H-NMR ($CD_3OD$) δ ppm: |
|---|---|---|
| Ex.628 | 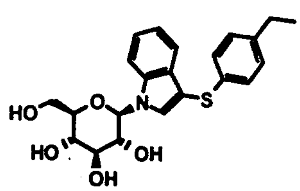 | 1.15 (3H, t, J=7.6Hz), 2.56 (2H, q, J=7.6Hz), 3.45-3.55 (1H, m), 3.55-3.65 (2H, m), 3.73 (1H, dd, J=12.1 Hz, 5.6Hz), 3.85-4 (2H, m), 5.51 (1 H, d, J=9.1Hz), 6.95-7.05 (4H, m), 7.05-7.15 (1H, m), 7.15-7.3 (1H, m), 7.4-7.5 (1H, m), 7.55-7.65 (1 H, m), 7.71 (1 H, s) |
| Ex.629 | 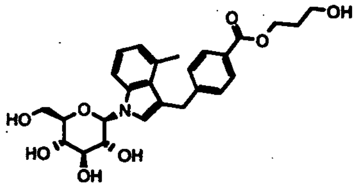 | 1.90-2.00 (2H, m), 2.38 (3H, s), 3.40-3.50 (1H, m), 3.50-3.65 (2H, m), 3.65-3.75 (3H, m), 3.80-3.95 (2H, m), 4.34 (2H, s), 4.39 (2H, t, J=6.3 Hz), 5.42 (1 H, d, J=8.8 Hz), 6.73 (1 H, d, J=7.1 Hz), 6.95-7.05 (1H, m), 7.15 (1H, s), 7.30 (2H, d, J=8.5 Hz), 7.35 (1H, d, J=8.5 Hz), 7.91 (2H, d, J=8.5 Hz) |

Test Example 1

Assay for inhibitory effects on human SGLT activity

1) Cloning and construction of the vector expressing human SGLT1

The cDNA library was prepared for PCR amplification by reverse transcription from total RNA deprived from human small intestine (Ori gene) using oligo-dT as a primer. Using this cDNA library as a template, the DNA fragment coding 1 to 2005 bp of human SGLT1 (ACCESSION: M24847), which was reported by Hediger et al., was amplified by PCR method and inserted into the multi-cloning site of pcDNA3.1(-) (Invitrogen). The DNA sequence inserted was perfectly matched to the previously reported sequence.

2) Cloning and construction of the vector expressing human SGLT2

The cDNA library was prepared for PCR amplification by reverse transcription from total RNA deprived from human kidney (Ori gene) using oligo-dT as a primer. Using this cDNA library as a template, the DNA fragment coding 2 to 2039 bp of human SGLT2 (ACCESSION: M95549, M95299), which was reported by R. G. Wells et al., was amplified by PCR method and inserted into the multi-cloning site of pcDNA3.1(-) (Invitrogen). The DNA sequence inserted was perfectly matched to the previously reported sequence.

3) Preparation of the cells expressing human SGLT1 or SGLT2

The vector expressing human SGLT1 or SGLT2 was transfected into COS-7 cells by lipofection method (Lipofectamine2000: Invitrogen). First, COS-7 cells were plated 5 x $10^4$ cells/ 100μL/well on 96-wells plate and incubated at 37°C for 2 hours. In addition, per 50 μL medium, 0.3 μg of human SGLT1 or SGLT2 expression vector was mixed with 0.5 μL of Lipofectamine2000 and the complex solution was prepared. Fifty μL/well of this complex solution was added to COS-7 cells, previously described, and the plate was mixed gently and was used for uptake assay after 2 days culture.

4) Measurement of the inhibitory activity against the uptake of methyl-α-D-glucopyranoside (α-MG)

A mixture of non-labeled (Sigma) and $^{14}$C-labeled α-MG (Amersham Pharmacia Biotech) was added to the uptake buffer (pH 7.4; containing 140 mM sodium chloride, 2 mM potassium chloride, 1 mM calcium chloride, 1 mM magnesium chloride, 10 mM 2-[4-(2-hydroxyethyl)-1-piperazinyl]ethane sulfonic acid and 5 mM tris(hydroxymethyl) aminomethane) at the final concentration of 1 mM. A test compound was dissolved in dimethyl sulfoxide, and then appropriately diluted with distilled water. The test compound solution was added to the uptake buffer containing 1 mM α-MG, and designated as a measurement buffer. For the control group, the measurement buffer without any test compound was prepared. For measuring the basal uptake, a basal uptakemeasurementbuffer, which contains 140 mM chorine chloride instead of sodium chloride, was prepared. After removing the culture medium of cells expressing human

SGLT1 or human SGLT2, 180 μL of the pre-treatment buffer (the basal uptake buffer without α-MG) was added to each well and incubated at 37°C for 10 minutes. After repeating the same treatment, the pre-treatment buffer was removed, and then 75 μL per well of the measurement buffer or the basal uptake buffer was added and the cells were incubated at 37°C. After 1 hour incubation, the measurement buffer was removed and the cells were washed twice with 180 μL per well of the washing buffer (the basal uptake buffer containing 10 mM non-labeled α-MG). The cells were solubilized by 75 μL per well of 0.2 mol/L sodium hydroxide, and then the cell lysates were transferred into PicoPlates (Packard). One hundred fifty μL of Microscint-40 (Packard) was added to the wells and mixed. Radioactivity was measured by means of micro-scintillation counter TopCount (Packard). One hundred % was set to the difference between the uptake in the control group and the basal uptake, and the uptake of methyl α-D-glucopyranoside at each drug concentration was calculated. The drug concentration, at which 50% uptake of methyl α-D-glucopyranoside was inhibited ($IC_{50}$ value), was calculated using logit plot. The results are shown in Table 10. The blanks in the Table are untested.

[Table 10]

| Example | SGLT1 $IC_{50}$ (nM) | SGLT2 $IC_{50}$ (nM) |
|---|---|---|
| 8 | 10 | 0.6 |
| 10 | | 0.5 |
| 12 | 3200 | 0.9 |
| 15 | | 11 |
| 318 | 122 | |
| 329 | 41 | |
| 346 | | 0.4 |

Comparing the test result of the compound of Example 8 with that of Example 12, it is understandable that 1-(β-D-glycopyranosyl)-3-substituted nitrogen-containing heterocyclic compound, wherein $Q^1$ is a carbon atom that is substituted for by a hydrogen atom in the general formula (I), has an extremely selective SGLT2 inhibitory activity.

Test Example 2

<u>Assay for the effect on urinary glucose excretion</u>

As experimental animals, overnight fasted SD rats (CHARLES RIVER LABORATORIES JAPAN. Inc., Crj:CD (SD(IGS), male, 7 weeks of age, 200-230 g) were used.

Example 10 compound of 2.12 mg was dissolved by adding 636 μL of ethanol and 2544 μL of polyethylene glycol 400. And then the solution was mixed with 3180 μL of saline and sonicated. Example 12 compound of 2.18 mg was dissolved by adding 654 μL of ethanol and 2616 μL of polyethylene glycol 400. And then the solution was mixed with 3270 μL of saline and sonicated. These test compound solutions of 0.33 mg/mL were used for administration.

The body weights of rats were measured and the solution of the test compound was intravenously injected to the tail vein of rats at the dose of 3 mL/kg (1 mg/kg as test compound). Collection of urine was performed for 24 hours after the administration. After urine samples were diluted appropriately with distilled water and mixed, these samples were centrifuged (2,000 x g, 4°C, 10 minutes). The supernatant liquids of 500 μL were stored at -20°C as sample for glucose concentration measurement.

Frozen samples were thawed and mixed well. According to need, a part of samples was diluted with distilled water and the glucose concentration of these samples was measured with a kit for laboratory test: Glucose CII-Test WAKO (Wako Pure Chemical Industries, Ltd.).

The amounts of urinary glucose excretion per 200 g of body weight were calculated from glucose concentration of urinary samples, dilution factor, urinary volume and body weight using the following equation.

Urinary glucose excretion (mg/200g body weight)

=Glucose concentration of urine sample (mg/dL) x Dilution factor

/100 x Urinary volume (mL)/Body weight of rat (g) x 200 .

The results are shown in Table 11.
[Table 11]

| Example | Amount of urinary excretion of glucose in 24 hours (mg/200g body weight) |
|---|---|
| 10 | 11.2 |
| 12 | 111.8 |

Test Example 3

Assay for the effect on urinary glucose excretion

As experimental animals, overnight fasted SD rats (CHARLES RIVER LABORATORIES JAPAN. Inc., Crj:CD (SD)IGS, male, 9 weeks of age, 300-330 g) were used.
Example 346 compound of 2.90 mg was dissolved by adding 29.0 μL of dimethyl sulfoxide and 2320 μL of polyethylene glycol 400. And then, the solution was mixed with 3450 μL of saline. This 0.5mg/mL solution was used for administration. Rats were fasted for overnight. The body weights of rats were measured and urinary glucose excretions were calculated as with the Test Example 2 except for intravenously injection to the tail veins of rats at the dose of 2 mL/kg (1 mg/kg as test compound).
The results are shown in the following Table 12.
[Table 12]

| Example | Amount of urinary excretion of glucose in 24 hours (mg/200g body weight) |
|---|---|
| 346 | 33.12 |

Test Example 4

Assay for inhibitory effects on blood glucose level increase in rats

1) Preparation of diabetic rat model

Rats, aged 8 weeks old, were injected streptozotocin (45 mg/kg) intravenously from tail vain. After 1 week, rats were fasted overnight and then glucose tolerance test (2 g/kg) was performed. The rats, which showed plasma glucose concentration at 1 hour after glucose load was over 300 mg/dL, were selected to use liquid meal tolerance test.

2) Liquid meal tolerance test

After overnight fasted, the diabetic rats were orally administered a test compound (2 mg/kg), which was dissolved in distilled water, in the drug-treating group, or distilled water alone in a control group. Immediately after the compound administration, 3 mL/body of liquid meal (No. 038, Control diet, assorted with dextrin and maltose; Oriental Yeast Co., Ltd., prepared for 323 g/L) was loaded orally. The blood was collected from tail artery immediately before and after administration with the time course, and treated with heparin immediately. The blood was centrifuged, and the plasma was collected and quantified the plasma glucose concentration by glucose oxidase method. Plasma glucose concentrations at pretreatment (0h), 0.5 and 1 hour after the drug administration are shown in the following Table 13. The values in the Table are presented as the mean ± S.E.
[Table 13]

| Example | Plasma glucose concentration (mg/dL) | | |
|---|---|---|---|
| | 0h | 0.5h | 1h |
| Control | 134 ± 6 | 374 ± 20 | 347 ± 23 |
| 320 | 133 ± 10 | 230 ± 14 | 312 ± 15 |

**Industrial Applicability**

The 1-(β-D-glycopyranosyl)-3-substituted nitrogen-containing heterocyclic compound (I) of the present invention or a prodrug thereof, a pharmaceutically acceptable salt thereof, or a hydrate or solvate thereof has SGLT inhibitory activity and can suppress postprandial increase of blood glucose or normalize blood glucose by inhibiting absorption of carbohydrates such as glucose at the small intestine or by inhibiting reabsorption of glucose at the kidney. Therefore, the present invention can provide agents for the prevention or treatment of diabetes, postprandial hyperglycemia, impaired glucose tolerance, diabetic complications, obesity or the like.

**Claims**

1. A 1-(β-D-glycopyranosyl)-3-substituted nitrogen-containing heterocyclic compound represented by the following general formula (I), or a prodrug thereof, or a pharmaceutically acceptable salt thereof, or a hydrate or a solvate thereof, with the exception of 1-(β-D-glucopyranosyl)-3-(2-thiazolyl)indole and 1-(β-D-glucopyranosyl)-6-methoxy-3-(2-thiazolyl) indole:

[Chem.1]

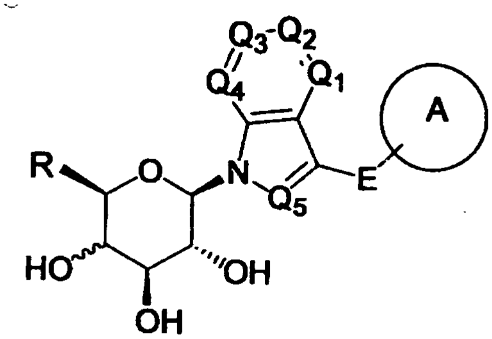

(I)

wherein ring A represents an optionally substituted aryl group or a heteroaryl group; $Q^1$ to $Q^5$ independently represent a carbon atom bound to a hydrogen atom or a substituent, or a nitrogen atom; E represents a single bond, an alkylene group, -O-, -S- or -NH-; and R represents a methyl group, an ethyl group, a fluoromethyl group or a hydroxymethyl group.

2. A 1-(β-D-glycopyranosyl)-3-substituted nitrogen-containing heterocyclic compound, or a prodrug thereof, or a pharmaceutically acceptable salt thereof, or a hydrate or a solvate thereof as claimed in claim 1, wherein the β-D-glycopyranosyl group represents a β-D-glucopyranosyl group or a β-D-galactopyranosyl group.

3. A 1-(β-D-glycopyranosyl)-3-substituted nitrogen-containing heterocyclic compound, or a prodrug thereof, or a pharmaceutically acceptable salt thereof, or a hydrate or a solvate thereof as claimed in claim 1 or 2, wherein $Q^1$ to $Q^5$ independently represent a carbon atom bound to a hydrogen atom or a substituent.

4. A 1-(β-D-glycopyranosyl)-3-substituted nitrogen-containing heterocyclic compound, or a prodrug thereof, or a pharmaceutically acceptable salt thereof, or a hydrate or a solvate thereof as claimed in any one of claims 1 to 3, wherein E represent an alkylene group or -S-.

**5.** An SGLT inhibitor which comprises a 1-(β-D-glycopyranosyl)-3-substituted nitrogen-containing heterocyclic compound, or a prodrug thereof, or a pharmaceutically acceptable salt thereof, or a hydrate or a solvate thereof as claimed in anyone of claims 1 to 4.

**6.** A pharmaceutical composition which comprises a 1-(β-D-glycopyranosyl)-3-substituted nitrogen-containing heterocyclic compound, or a prodrug thereof, or a pharmaceutically acceptable salt thereof, or a hydrate or a solvate thereof as claimed in any one of claims 1 to 4.

**7.** A pharmaceutical composition as claimed in claim 6, which is used as a glucose or galactose absorption inhibitor.

**8.** A pharmaceutical composition as claimed in claim 6, which is used as a glucose reabsorption inhibitor.

**9.** A pharmaceutical composition as claimed in claim 6, which is used as an inhibitor of postprandial hyperglycemia or for the prevention or treatment of a disease selected from the group consisting of diabetes, impaired glucose tolerance, diabetic complications, obesity, hyperinsulinemia, hyperlipidemia, hypercholesterolemia, galactosemia, hypertriglyceridemia, lipid metabolism disorder, atherosclerosis, hypertension, congestive heart failure, edema, hyperuricemia and gout.

**10.** A pharmaceutical composition as claimed in claim 9, which is used for the inhibition of impaired glucose tolerance advancing into diabetes.

**11.** A combination of a pharmaceutical composition as claimed in any one of claims 8 to 10 and at least one drug selected from the group consisting of an insulin sensitivity enhancer, an amylase inhibitor, an α-glucosidase inhibitor, a biguanide, an insulin secretion enhancer, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinositol, a glycogen synthase kinase-3 inhibitor, an 11β-hydroxysteroid-dehydrogenaze inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid dipeptidase inhibitor, insulin-like growth factor-I, platelet-derived growth factor (PDGF), a platelet-derived growth factor (PDGF) analogue, epidermal growth factor (EGF), nerve growth factor, a carnitine derivative, uridine, 5-hydroxy-1-methylhidantoin, EGB-761, bimoclomol, sulodexide, Y-128, an antidiarrhoics, a cathartics, a hydroxymethylglutaryl coenzyme A reductase inhibitor, a fibrate, a $\beta_3$-adrenoceptor agonist, an acyl-coenzyme A: cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyltransferase inhibitor, a squalene synthase inhibitor, a squalene epoxidase inhibitor, a low-density lipoprotein receptor enhancer, a nicotinic acid derivative, abileacidsequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an $\alpha_2$-adrenoceptor agonist, an antiplatelets agent, a uric acid synthesis inhibitor, a uricosuric agent and a urinary alkalinizer.

## INTERNATIONAL SEARCH REPORT

International application No.
PCT/JP2005/017807

A. CLASSIFICATION OF SUBJECT MATTER
***C07H19/04*** (2006.01), ***A61K31/7056*** (2006.01), ***A61K31/706*** (2006.01), ***A61K45/00*** (2006.01), ***A61P3/04*** (2006.01), ***A61P3/06*** (2006.01), ***A61P3/10*** (2006.01), ***A61P9/04*** (2006.01), ***A61P9/10*** (2006.01), ***A61P9/12*** (2006.01), ***A61P19/06***

According to International Patent Classification (IPC) or to both national classification and IPC

B. FIELDS SEARCHED

Minimum documentation searched (classification system followed by classification symbols)
***C07H19/04*** (2006.01), ***A61K31/7056*** (2006.01), ***A61K31/706*** (2006.01), ***A61K45/00*** (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2005/012326 A1 (TANABE SEIYAKU CO., LTD.), 10 February, 2005 (10.02.05), Claims; examples 115, 117 & US 2005/0032711 A1 & US 2005/0032712 A1 & US 2005/0037980 A1 & US 2005/0037981 A1 & WO 2005/011592 A2 & WO 2005/012242 A2 & WO 2005/012243 A2 & WO 2005/012318 A2 | 1-11 |
| X | CECCHI, L. et al., Synthesis of 1-N-glycosides of 3-phenylpyrazolo[4,5-b]pyrazine, Farmaco, Edizione Scientifica, 1983, Vol.38, No.1, pages 24 to 28 | 1,2 |

☒ Further documents are listed in the continuation of Box C.

☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>04 November, 2005 (04.11.05) | Date of mailing of the international search report<br>15 November, 2005 (15.11.05) |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2005/017807

C (Continuation). DOCUMENTS CONSIDERED TO BE RELEVANT

| Category* | Citation of document, with indication, where appropriate, of the relevant passages | Relevant to claim No. |
|---|---|---|
| A | WO 2004/080990 A (Yamanouchi Pharmaceutical Co., Ltd., Kotobuki Pharmaceutical Co., Ltd.), 23 September, 2004 (23.09.04), Full text (Family: none) | 1-11 |
| A | JP 2003-511458 A (Bristol-Myers Squibb Co.), 25 March, 2003 (25.03.03), Claims & WO 2001/027128 A1 & US 2002/0137903 A1 & EP 1224195 A & EP 1506211 A & WO 2003/099836 A1 | 1-11 |

Form PCT/ISA/210 (continuation of second sheet) (April 2005)

**INTERNATIONAL SEARCH REPORT**

International application No.
PCT/JP2005/017807

Continuation of A. CLASSIFICATION OF SUBJECT MATTER
(International Patent Classification (IPC))

(2006.01), ***A61P43/00*** (2006.01)

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 04014932 A
- WO 01018491 A
- JP 2004196788 A
- WO 0116147 A
- WO 0168660 A
- WO 04018491 A
- WO 0414932 A

### Non-patent literature cited in the description


- **YOSHIKATSU KANAI.** Kidney and Dialysis. 1998, vol. 45, 232-237
- **E. TURK.** *Nature,* 1991, vol. 350, 354-356
- **J. DYER.** *American Journal of Physiology,* 2002, vol. 282 (2), G241-G248
- **YOSHIKATSU KANAI.** *J. Clin. Invest.,* 1994, vol. 93, 397-404
- **M. PEDRAS.** *Bioorganic & Medicinal Chemistry,* 2002, vol. 10, 3307-3312
- **EKTOVA,L.V.** *Khikiko-Farmatsevticheskii Zhurnal,* 1988, vol. 22 (7), 860-3
- *J. Org.Chem.,* 2002, vol. 67, 622-6227
- **CICCHILLO,R.M. ; NORRIS,P.** *Carbohydrate Research,* 2000, vol. 328, 431-434